# EUROPEAN PATENT APPLICATION

(11) **EP 2 805 719 A1**
(43) Date of publication of application: **26.11.2014**
(21) Application number: 14169305.1
(22) Date of filing: 30.03.2006
(51) Int. Cl.: A61K 31/445, C07H 7/06, C07H 19/048, A61P 39/00

(54) **Nicotinamide riboside and analogues thereof**

(30) Priority: 30.03.2005 US 667179 P
(62) Divisional of application: 06749076.3
(71) Applicant: GlaxoSmithKline LLC, Wilmington DE 19808 (US)
(72) Inventor: Milburn, Michael, Cary, NC North Carolina 27519-9725 (US); Milne, Jill, Brookline,, MA Massachusetts 02446-2768 (US); Normington, Karl, D, Acton, MA Massachusetts 01720 (US); Nunes, Joseph, J, Andover,, MA Massachusetts 01810 (US); Salzmann, Thomas, Warren, NJ New Jersey 07059 (US); Sinclair, David, West Roxbury, MA Massachusetts 02132 (US); Westphal, Christoph, H, Brookline, MA Massachusetts 02446 (US)
(74) Representative: Valentine, Jill Barbara

(57) **Abstract**

Provided herein are sirtuin-modulating compounds and methods of use thereof. The sirtuin-modulating compounds may be used for increasing the lifespan of a cell, and treating and/or preventing a wide variety of diseases and disorders including, for example, diseases or disorders related to aging or stress, diabetes, obesity, neurodegenerative diseases, cardiovascular disease, blood clotting disorders, inflammation, cancer, and/or flushing. Also provided are compositions comprising a sirtuin-modulating compound in combination with another therapeutic agent.

## Description

### BACKGROUND

The Silent Information Regulator (SIR) family of genes represents a highly conserved group of genes present in the genomes of organisms ranging from archaebacteria to a variety of eukaryotes (Frye, 2000). The encoded SIR proteins are involved in diverse processes from regulation of gene silencing to DNA repair. The proteins encoded by members of the SIR gene family show high sequence conservation in a 250 amino acid core domain. A well-characterized gene in this family is S. cerevisiae SIR2, which is involved in silencing HM loci that contain information specifying yeast mating type, telomere position effects and cell aging (Guarente, 1999; Kaeberlein et al., 1999; Shore, 2000). The yeast Sir2 protein belongs to a family of histone deacetylases (reviewed in Guarente, 2000; Shore, 2000). The Sir2 homolog, CobB, in Salmonella typhimurium, functions as an NAD (nicotinamide adenine dinucleotide)-dependent ADP-ribosyl transferase (Tsang and Escalante-Semerena, 1998).

The Sir2 protein is a class III deacetylase which uses NAD as a cosubstrate (Imai et al., 2000; Moazed, 2001; Smith et al., 2000; Tanner et al., 2000; Tanny and Moazed, 2001). Unlike other deacetylases, many of which are involved in gene silencing, Sir2 is insensitive to class I and II histone deacetylase inhibitors like trichostatin A (TSA) (Imai et al., 2000; Landry et al., 2000a; Smith et al., 2000).

Deacetylation of acetyl-lysine by Sir2 is tightly coupled to NAD hydrolysis, producing nicotinamide and a novel acetyl-ADP ribose compound (Tanner et al., 2000; Landry et al., 2000b; Tanny and Moazed, 2001). The NAD-dependent deacetylase activity of Sir2 is essential for its functions which can connect its biological role with cellular metabolism in yeast (Guarente, 2000; Imai et al., 2000; Lin et al., 2000; Smith et al., 2000). Mammalian Sir2 homologs have NAD-dependent histone deacetylase activity (Imai et al., 2000; Smith et al., 2000). Most information about Sir2 mediated functions comes from the studies in yeast (Gartenberg, 2000; Gottschling, 2000).

Biochemical studies have shown that Sir2 can readily deacetylate the amino-terminal tails of histones H3 and H4, resulting in the formation of 1-*O*-acetyl-ADP-ribose and nicotinamide. Strains with additional copies of *SIR2* display increased rDNA silencing and a 30% longer life span. It has recently been shown that additional copies of the *C*. *elegans SIR2* homolog, *sir-2.1,* and the *D. melanogaster* dSir2 gene greatly extend life span in those organisms. This implies that the *SIR2*-dependent regulatory pathway for aging arose early in evolution and has been well conserved. Today, Sir2 genes are believed to have evolved to enhance an organism's health and stress resistance to increase its chance of surviving adversity.

Caloric restriction has been known for over 70 years to improve the health and extend the lifespan of mammals (Masoro, 2000). Yeast life span, like that of me*tazoans, is also extended by interventions that resemble caloric restriction, such as low glucose. The discovery that both yeast and flies lacking the SIR2 gene do not live longer when calorically restricted provides evidence that SIR2 genes mediate the beneficial health effects of this diet (Anderson et al., 2003; Helfand and Rogina, 2004). Moreover, mutations that reduce the activity of the yeast glucose-responsive cAMP (adenosine 3'5'-monophosphate)-dependent (PKA) pathway extend life span in wild type cells but not in mutant *sir2* strains, demonstrating that *SIR2* is likely to be a key downstream component of the caloric restriction pathway (Lin et al., 2001).

### SUMMARY

The present invention is directed to nicotinamide riboside and analogs thereof, including their use in methods of treating diseases or conditions, such as diabetes/insulin resistance, hyperlipidemia and obesity. It is believed that nicotinamide riboside and its analogs directly or indirectly activate sirtuins, such as the human protein SIRT1. For convenience, the compounds disclosed herein are referred to as "sirtuin modulating compounds"; however, Applicants do not intend this designation to mean that the biological effects of these compounds are dependent upon sirtuin modulation (activation).

In certain embodiments of the invention, the invention is directed to analogs of nicotinamide riboside, particularly compounds that are metabolized, hydrolyzed or otherwise converted to nicotinamide riboside *in vivo.*

In one embodiment, the invention is a method for promoting survival of a eukaryotic cell comprising contacting the cell with at least one compound of Structural Formula (I) or (II): 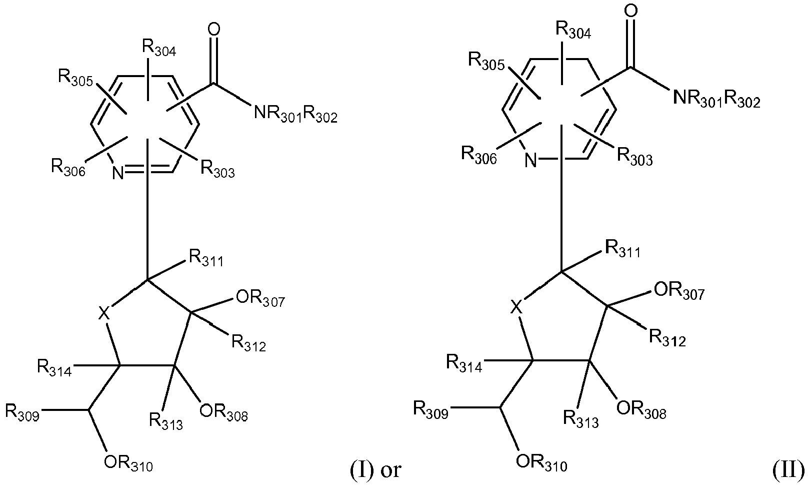 or a pharmaceutically acceptable salt thereof, where:
R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2.

In another embodiment, the invention is method for treating or preventing a disease or disorder associated with cell death, cell dysfunction or aging in a subject, comprising administering to a subject in need thereof a therapeutically effective amount of at least one compound of Structural Formula (I) or (II): 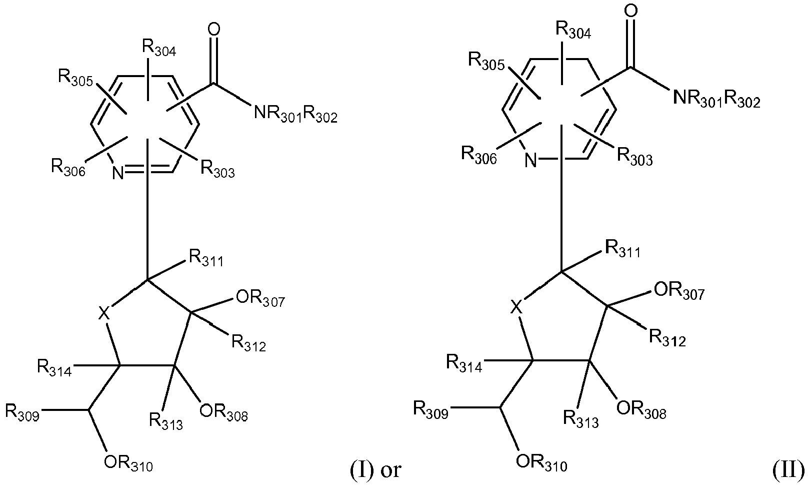 or a pharmaceutically acceptable salt thereof, where:
R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2.

In yet another embodiment, the invention is a method for treating or preventing insulin resistance, a metabolic syndrome, hypercholesterolemia, artherogenic dyslipidemia, diabetes, or complications thereof, or for increasing insulin sensitivity in a subject, comprising administering to a subject in need thereof a therapeutically effective amount of at least one compound of Structural Formula (I) or (II): 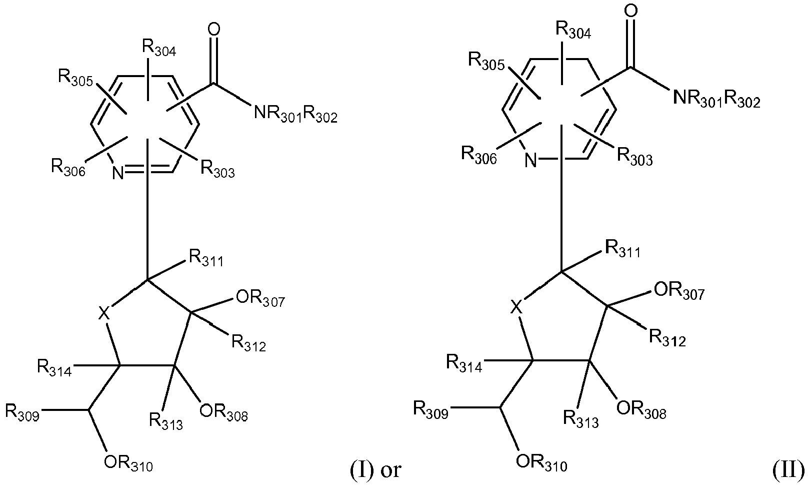 or a pharmaceutically acceptable salt thereof, where:
R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2,
provided that when X is O and R₃₀₁-R₃₀₉ and R₃₁₁-R₃₁₄ are -H, R₃₁₀ is not -H.

In a further embodiment, the invention is method for reducing the weight of a subject, or preventing weight gain in a subject, comprising administering to a subject in need thereof a therapeutically effective amount of at least one compound of Structural Formula (I) or (II): 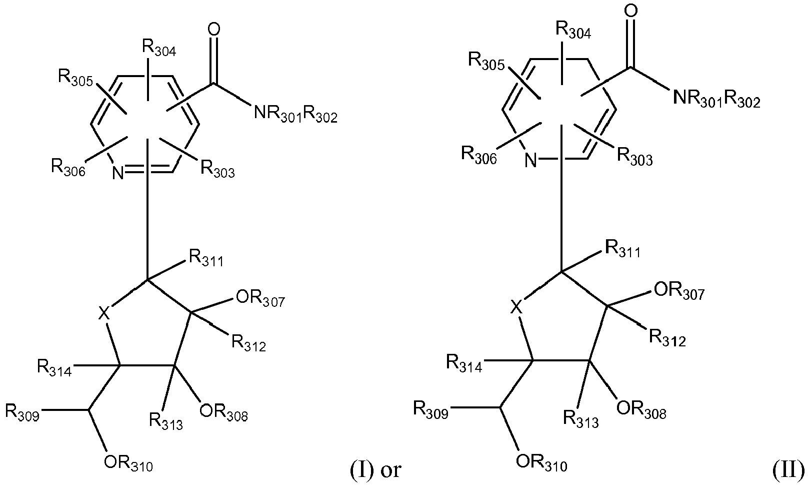 or a pharmaceutically acceptable salt thereof, wherein:
R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR,-S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2.

In one embodiment, the invention is a method for preventing the differentiation of a pre-adipocyte, comprising contacting the pre-adipocyte with at least one compound of Structural Formula (I) or (II): 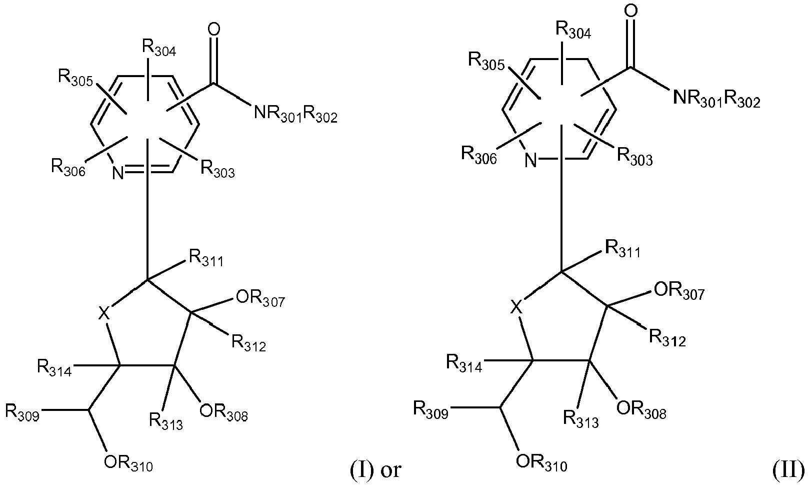 or a pharmaceutically acceptable salt thereof, where:
R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2.

In another embodiment, the invention is a method for prolonging the lifespan of a subject comprising administering to a subject a therapeutically effective amount of at least one compound of Structural Formula (I) or (II): 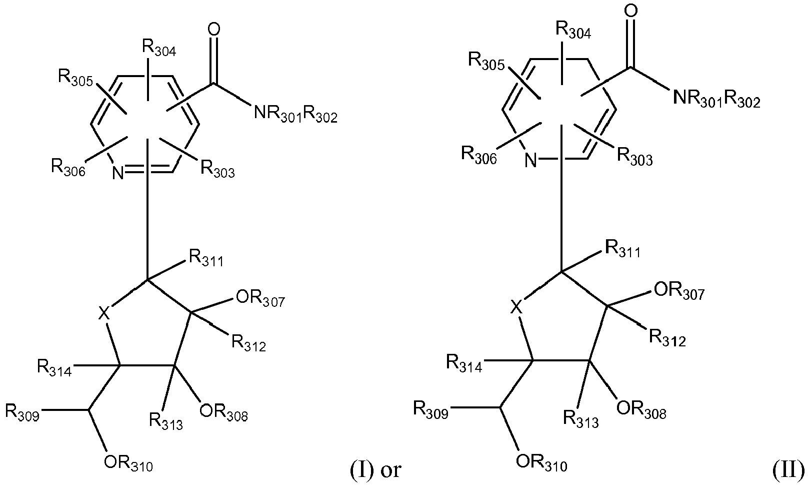 or a pharmaceutically acceptable salt thereof, where:
R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2.

In yet another embodiment, the invention is a method for treating or preventing a neurodegenerative disorder in a subject, comprising administering to a subject in need thereof a therapeutically effective amount of at least one compound of Structural Formula (I) or (II): 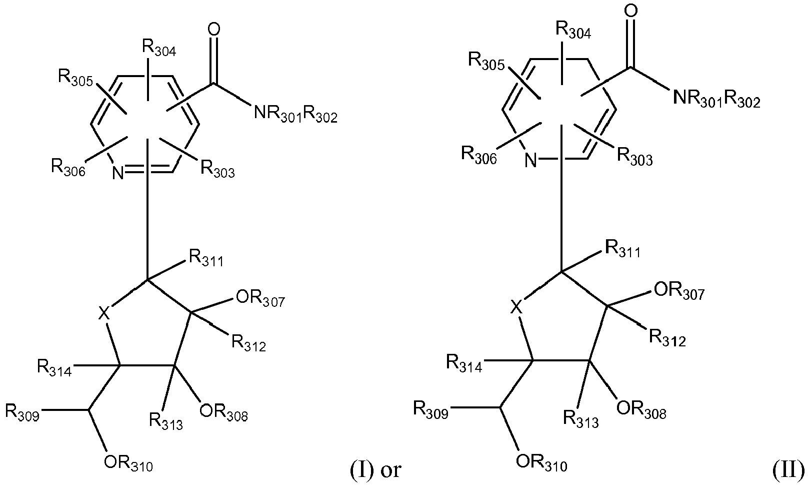 or a pharmaceutically acceptable salt thereof, where:
R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2.

In a further embodiment, the invention is a method of for treating or preventing a blood coagulation disorder in a subject, comprising administering to a subject in need thereof a therapeutically effective amount of at least one compound of Structural Formula (I) or (II): 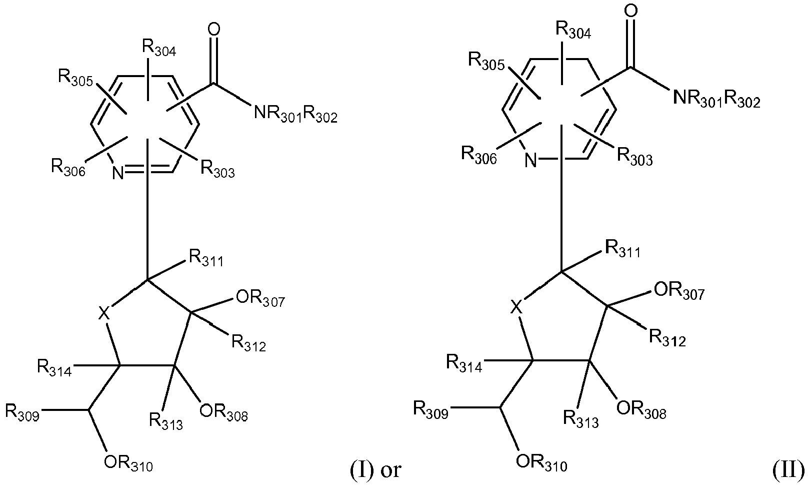 or a pharmaceutically acceptable salt thereof, where:
R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2.

In the above methods, the compound represented by Structural Formula (I) or (II) is typically represented by Structural Formula (III) or (IV): 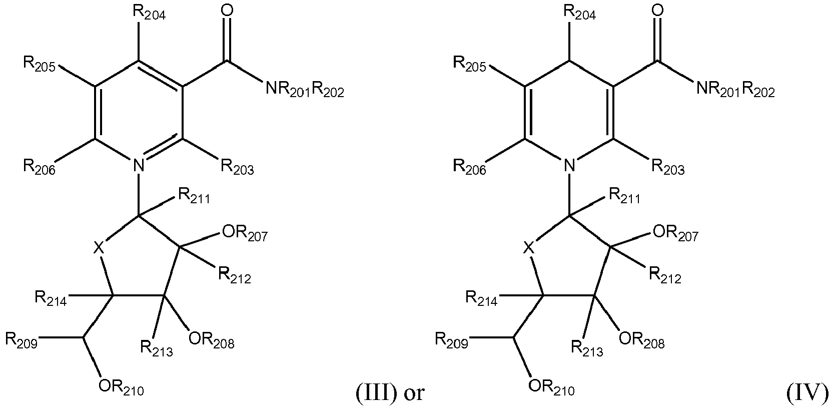 or a pharmaceutically acceptable salt thereof, where:
R₂₀₁ and R₂₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₂₀₁ and R₂₀₂ taken together with the atom to which they are attached form a substituted or unsubstituted non-aromatic heterocyclic group;
R₂₀₃, R₂₀₄, R₂₀₅ and R₂₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H₅ -S(O)ₙR, -S(O)ₙOR,-S(O)ₙNRR', -NRR', -NRC(O)OR', -NO₂ and -NRC(O)R';
R₂₀₇, R₂₀₈ and R₂₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₂₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₂₁₁, R₂₁₂, R₂₁₃ and R₂₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR,-S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2.

Another aspect of the invention is a pharmaceutical composition comprising a pharmaceutically acceptable carrier or diluent and a compound represented by Structural Formula (V) or (VI): 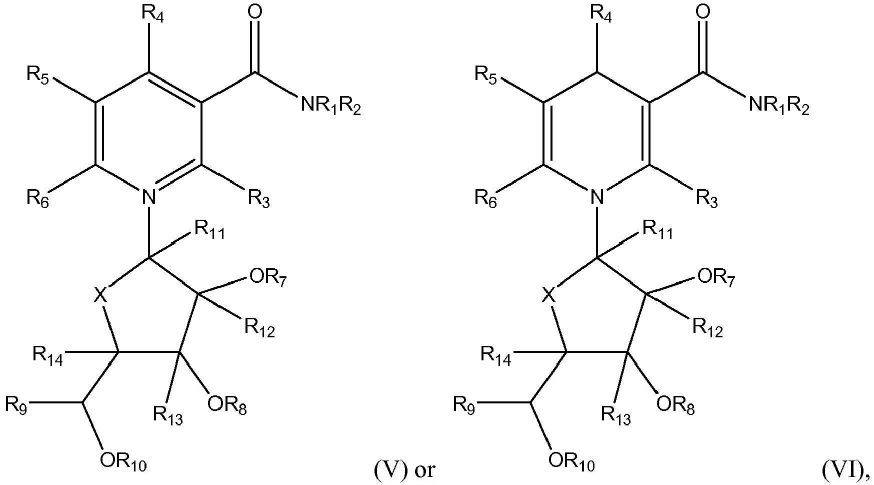 or a pharmaceutically acceptable salt thereof, where:
R₁ and R₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁ and R₂ taken together with the atom to which they are attached form a substituted or unsubstituted non-aromatic heterocyclic group, provided that when one of R₁ and R₂ is -H, the other is not an alkyl group substituted by -C(O)OCH₂CH₃;
R₃, R₄ and R₅ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR,-S(O)ₙNRR', -NRR', -NRC(O)OR', -NO₂ and -NRC(O)R';
R₆ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRC(O)OR', -NO₂ and -NRC(O)R';
R₇, R₈ and R₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₉ selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₁₁, R₁₂, R₁₃ and R₁₄ are independently selected from the group consisting of - H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR,-S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2,
provided that R₁-R₁₄ are not each -H and that R₁-R₉ and R₁₁-R₁₄ are not each -H when R₁₀ is -C(O)C₆H₅.

In one embodiment, the invention is a pharmaceutical composition comprising a pharmaceutically acceptable carrier or diluent and a compound represented by Structural Formula (VII) or (VIII): 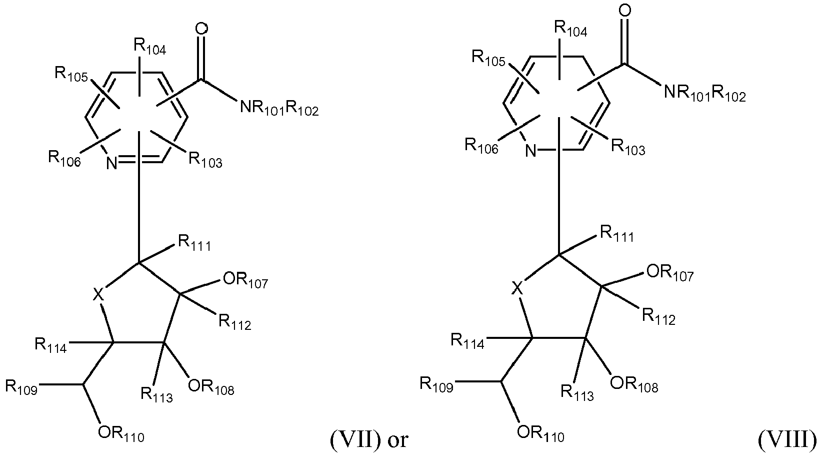 or a pharmaceutically acceptable salt thereof, wherein:
R₁₀₁, and R₁₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁₀₁, and R₁₀₂ taken together with the atom to which they are attached form a substituted or unsubstituted non-aromatic heterocyclic group;
R₁₀₃, R₁₀₄, R₁₀₅ and R₁₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H₅ -S(O)ₙR, -S(O)ₙOR,-S(O)ₙNRR', -NRR', -NRC(O)OR', -NO₂ and -NRC(O)R';
R₁₀₇ and R₁₀₈ are selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, wherein at least one of R₁₀₇ and R₁₀₈ is a substituted or unsubstituted alkyl group, a substituted or unsubstituted aryl group, -C(O)R, -C(O)OR, -C(O)NHR, -C(S)R, -C(S)OR or -C(O)SR;
R₁₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₁₁₀ is selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, provided that R₁₁₀ is not -C(O)C₆H₅;
R₁₁₁, R₁₁₂, R₁₁₃ and R₁₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR,-S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2.

In another embodiment, the invention is a pharmaceutical composition comprising a pharmaceutically acceptable carrier or diluent and a compound represented by Structural Formula (IX) or (X): 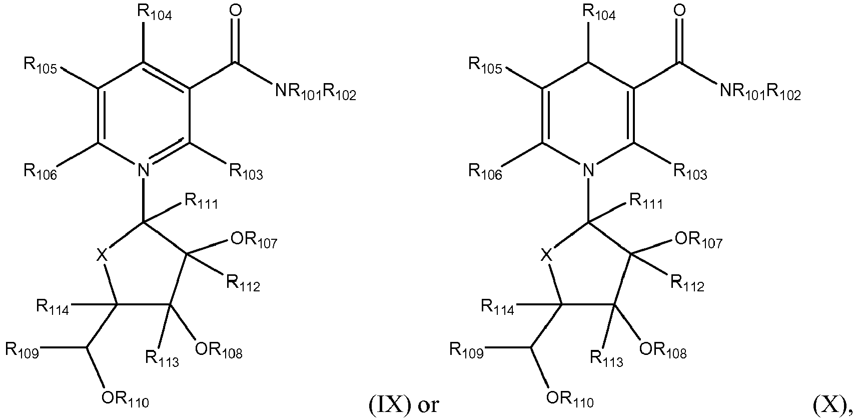 or a pharmaceutically acceptable salt thereof, where:
R₁₀₁, and R₁₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁₀₁, and R₁₀₂ taken together with the atom to which they are attached form a substituted or unsubstituted non-aromatic heterocyclic group;
R₁₀₃, R₁₀₄, R₁₀₅ and R₁₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H₅ -S(O)ₙR, -S(O)ₙOR, -S(O)ₙNRR', -NRR', -NRC(O)OR', -NO₂ and -NRC(O)R';
R₁₀₇ and R₁₀₈ are selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, wherein at least one of R₁₀₇ and R₁₀₈ is a substituted or unsubstituted alkyl group, a substituted or unsubstituted aryl group, -C(O)R, -C(O)OR, -C(O)NHR, -C(S)R, -C(S)OR or -C(O)SR;
R₁₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₁₁₀ is selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, provided that R₁₁₀ is not -C(O)C₆H₅;
R₁₁₁, R₁₁₂, R₁₁₃ and R₁₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2.

A further aspect of the invention is a compound represented by Structural Formula (XI) or (XII): 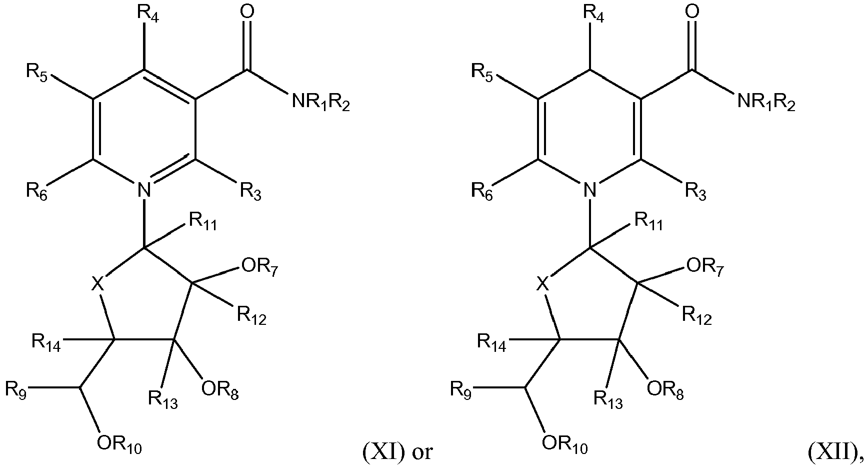 or a pharmaceutically acceptable salt thereof, where:
R₁ and R₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁ and R₂ taken together with the atom to which they are attached form a substituted or unsubstituted non-aromatic heterocyclic group, provided that when one of R₁ and R₂ is -H, the other is not a 2,2,6,6-tetramethyl-1-oxypiperidin-4-yl group and is not an alkyl group substituted by -C(O)OCH₂CH₃;
R₃ and R₄ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
R₅ is selected from the group consisting of -H, a substituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR,-S(O)ₙNRR', - NRR', -NRC(O)OR', -NO₂ and -NRC(O)R';
R₆ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRC(O)OR', -NO₂ and -NRC(O)R';
R₇, R₈ and R₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₉ selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₁₁, R₁₂, R₁₃ and R₁₄ are independently selected from the group consisting of - H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and n is 1 or 2,
provided that R₁-R₆ are not each -H.

Yet another aspect of the invention is a compound represented by Structural Formula (XI) or (XII): 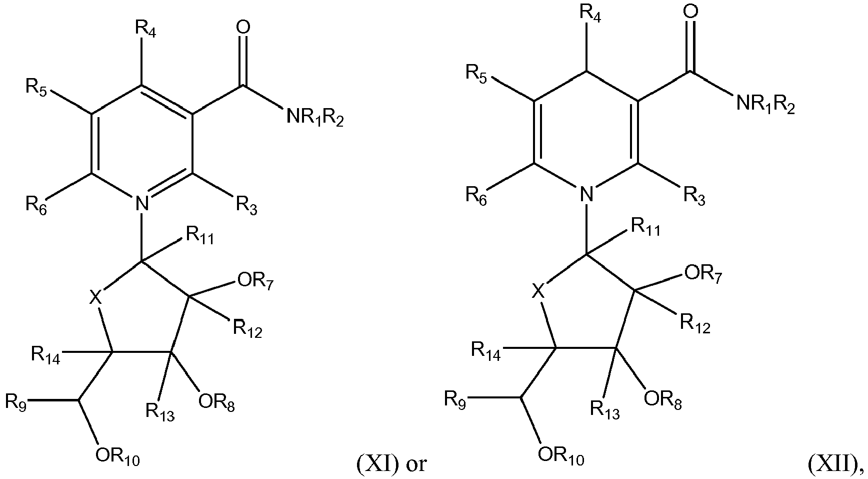 or a pharmaceutically acceptable salt thereof, wherein:
R₁ and R₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁ and R₂ taken together with the atom to which they are attached form a substituted or unsubstituted non-aromatic heterocyclic group;
R₃, R₄, R₅ and R₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR,-S(O)ₙNRR', -NRR', -NRC(O)OR', -NO₂ and -NRC(O)R';
R₇, R₈ and R₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, wherein at least one of R₇ and R₈ is a substituted or unsubstituted alkyl group, a substituted or unsubstituted aryl group, -C(O)R, -C(O)OR, -C(O)NHR, -C(S)R, -C(S)OR or -C(O)SR, provided that none of R₇ and R₈ are -C(O)C₆H₅ and that R₇ and R₈ are not both -C(O)CH₃ or -C(O)C₆H₄F;
R₉ selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₁₁, R₁₂, R₁₃ and R₁₄ are independently selected from the group consisting of - H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR,-S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2.

A further aspect of the invention is a compound represented by Structural Formula (XI) or (XII): 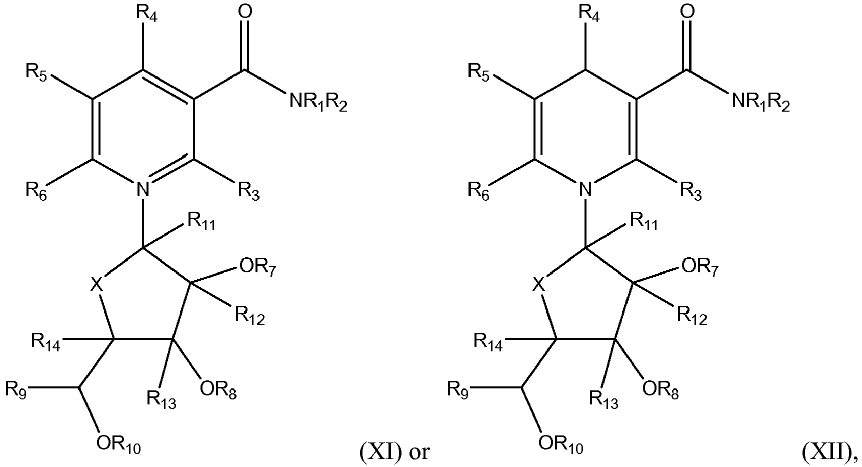 or a pharmaceutically acceptable salt thereof, where:
R₁ and R₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁ and R₂ taken together with the atom to which they are attached form a substituted or unsubstituted non-aromatic heterocyclic group;
R₃, R₄, R₅ and R₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
R₇ and R₈ are selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₉ selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₁₀ is selected from the group consisting of a substituted or unsubstituted alkyl group, a substituted or unsubstituted aryl group, -C(O)R, -C(O)OR, -C(O)NHR, - C(S)R, -C(S)OR and -C(O)SR, provided that Rio is not -C(C₆H₅)₃, -C(O)C₆H₅, - C(O)CH₃, -C(O)C₆H₄F or -C(O)CH(OC(O)CH₃)CH(CH₃)CH₂CH₃;
R₁₁, R₁₂, R₁₃ and R₁₄ are independently selected from the group consisting of - H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2.

In certain aspects, the sirtuin-modulating compounds may be administered alone or in combination with other compounds, including other sirtuin-modulating compounds and/or metabolic inhibitors, or other therapeutic agents.

The invention also includes the use of compounds disclosed herein in medicine. In addition, the invention also the use of the compounds disclosed herein in the manufacture of medicament for treating (including prophylactic treatment) one or more diseases and/or conditions disclosed herein.

### BRIEF DESCRIPTION OF THE FIGURES

FIG. 1A shows the average clinical Experimental autoimmune encephalomyelitis (EAE) score after immunization with proteolipid protein (PLP).
FIG. 1B shows the percentage of eyes from EAE mice that develop optic neuritis.
FIG. 2 shows that there is a significant decrease in Retinal Ganglion Cells (RGCs) progress over time in optic neuritis eyes.

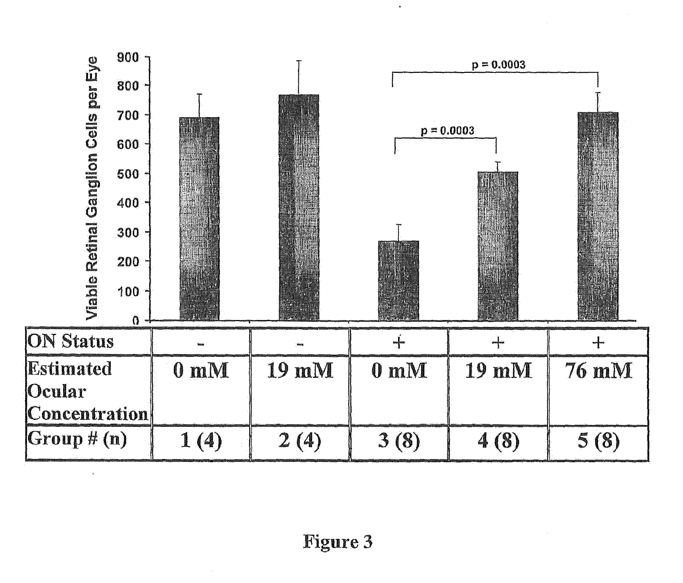

FIG. 3 shows that nicotinamide riboside is effective in an acute optic neuritis model.

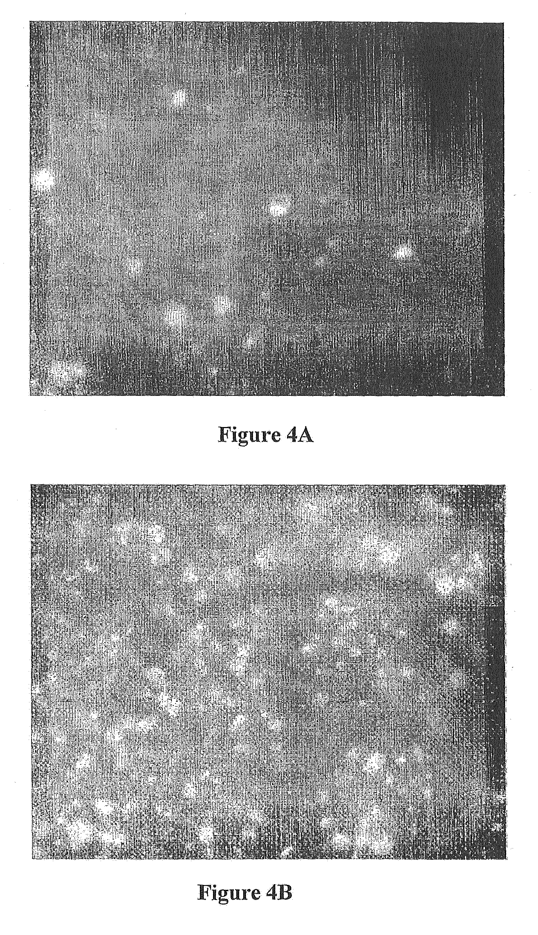

FIGS. 4A and B show studies with fluorogold-labeled RGCs. FIG. 4A shows RGCs of eye with optic neuritis treated with placebo (PBS) (representative of Group 3). FIG. 4B shows RGCs of eye with optic neuritis treated with nicotinamide riboside (representative of Group 5).
FIG. 5 shows a typical chromatogram of triacetoxy nicotinamide riboside in rat plasma.
FIG. 6 shows a typical chromatogram of nicotinamide mononucleotide (NMN) in rat plasma.
FIG. 7 shows a typical chromatogram of nicotinamide riboside in rat plasma.
FIG. 8 shows triacetoxy nicotinamide riboside stability versus time in rat plasma before organic solvent addition.
FIG. 9 shows NMN stability vs. time in rat plasma before organic solvent addition.
FIG. 10 shows nicotinamide riboside stability versus time in rat plasma before organic solvent addition.

### DETAILED DESCRIPTION

The present invention is directed to nicotinamide riboside and its analogs, along with its uses.

NAD and its phosphorylated analog, NADP, are indispensable cofactors for numerous oxidoreductases in all living organisms (Moat and Foster, 1987). NAD and NADP also serve as cofactors for enzymes that do not appear to be involved in oxidation or reduction. For example, sirtuins, a conserved family of protein deacetylases that include Sir2 and Sir2-related enzymes, require NAD for their activity as transcriptional silencers. This NAD-dependent deacetylation activity is believed to cause alterations in gene expression, repression of ribosomal DNA recombination, and the health benefits and lifespan extension provided by calorie restriction. Accordingly, compounds that are capable of modulating sirtuin activity may be useful in a variety of medical conditions in mammals (e.g., mice and humans), such as those that are caused by or associated with changes in gene expression and age of the individual. These medical conditions include disorders related to aging or stress, diabetes, obesity, neurodegenerative diseases, cardiovascular disease, blood clotting disorders, inflammation, cataracts, flushing, cell death, cancer, appetite, and/or weight gain.

NAD can be synthesized de novo from tryptophan via the kynurenine pathway (Krehl et al., 1945; Schutz and Feigelson, 1972) or by salvaging nicotinic acid that is imported extracellularly. Furthermore, nicotinic acid can be deamidated from nicotinamide in yeast (Panozzo et al., 2002; Anderson et al., 2003; Gallo et al., 2004), although this pathway does not appear to be conserved in humans. Instead, it is thought that humans utilize pre-B-cell colony enhancing factor (PBEF) to synthesize nicotinamide mononucleotide (NMN), which is a precursor to NAD.

An alternate NAD biosynthetic pathway appears to be conserved in humans and yeast. In this pathway, nicotinamide riboside is phosphorylated to generate NMN, which in turn is used to generate NAD.

### 1. Definitions

As used herein, the following terms and phrases shall have the meanings set forth below. Unless defined otherwise, all technical and scientific terms used herein have the same meaning as commonly understood to one of ordinary skill in the art.

The singular forms "a," "an," and "the" include plural reference unless the context clearly dictates otherwise.

The term "agent" is used herein to denote a chemical compound, a mixture of chemical compounds, a biological macromolecule (such as a nucleic acid, an antibody, a protein or portion thereof, e.g., a peptide), or an extract made from biological materials such as bacteria, plants, fungi, or animal (particularly mammalian) cells or tissues. The activity of such agents may render it suitable as a "therapeutic agent" which is a biologically, physiologically, or pharmacologically active substance (or substances) that acts locally or systemically in a subject.

The term "bioavailable" when referring to a compound is art-recognized and refers to a form of a compound that allows for it, or a portion of the amount of compound administered, to be absorbed by, incorporated to, or otherwise physiologically available to a subject or patient to whom it is administered.

"Biologically active portion of a sirtuin" refers to a portion of a sirtuin protein having a biological activity, such as the ability to deacetylate. Biologically active portions of sirtuins may comprise the core domain of sirtuins. Biologically active portions of SIRT1 having GenBank Accession No. NP_036370 that encompass the NAD⁺ binding domain and the substrate binding domain, for example, may include without limitation, amino acids 62-293 of GenBank Accession No. NP_036370, which are encoded by nucleotides 237 to 932 of GenBank Accession No. NM_012238. Therefore, this region is sometimes referred to as the core domain. Other biologically active portions of SIRT1, also sometimes referred to as core domains, include about amino acids 261 to 447 of GenBank Accession No. NP_036370, which are encoded by nucleotides 834 to 1394 of GenBank Accession No. NMN_012238; about amino acids 242 to 493 of GenBank Accession No. NP_036370, which are encoded by nucleotides 777 to 1532 of GenBank Accession No. NMN_012238; or about amino acids 254 to 495 of GenBank Accession No. NP_036370, which are encoded by nucleotides 813 to 1538 of GenBank Accession No .NM_012238.

The term "companion animals" refers to cats and dogs. As used herein, the term "dog(s)" denotes any member of the species Canis familiaris, of which there are a large number of different breeds. The term "cat(s)" refers to a feline animal including domestic cats and other members of the family Felidae, genus Felis.

The terms "comprise" and "comprising" are used in the inclusive, open sense, meaning that additional elements may be included.

The term "conserved residue" refers to an amino acid that is a member of a group of amino acids having certain common properties. The term "conservative amino acid substitution" refers to the substitution (conceptually or otherwise) of an amino acid from one such group with a different amino acid from the same group. A functional way to define common properties between individual amino acids is to analyze the normalized frequencies of amino acid changes between corresponding proteins of homologous organisms (Schulz, G. E. and R. H. Schirmer., Principles of Protein Structure, Springer-Verlag). According to such analyses, groups of amino acids may be defined where amino acids within a group exchange preferentially with each other, and therefore resemble each other most in their impact on the overall protein structure (Schulz, G. E. and R. H. Schirmer, Principles of Protein Structure, Springer-Verlag). One example of a set of amino acid groups defined in this manner include: (i) a charged group, consisting of Glu and Asp, Lys, Arg and His, (ii) a positively-charged group, consisting of Lys, Arg and His, (iii) a negatively-charged group, consisting of Glu and Asp, (iv) an aromatic group, consisting of Phe, Tyr and Trp, (v) a nitrogen ring group, consisting of His and Trp, (vi) a large aliphatic nonpolar group, consisting of Val, Leu and Ile, (vii) a slightly-polar group, consisting of Met and Cys, (viii) a small-residue group, consisting of Ser, Thr, Asp, Asn, Gly, Ala, Glu, Gln and Pro, (ix) an aliphatic group consisting of Val, Leu, Ile, Met and Cys, and (x) a small hydroxyl group consisting of Ser and Thr.

"Diabetes" refers to high blood sugar or ketoacidosis, as well as chronic, general metabolic abnormalities arising from a prolonged high blood sugar status or a decrease in glucose tolerance. "Diabetes" encompasses both the type I and type II (Non Insulin Dependent Diabetes Mellitus or NIDDM) forms of the disease. The risk factors for diabetes include the following factors: waistline of more than 40 inches for men or 35 inches for women, blood pressure of 130/85 mmHg or higher, triglycerides above 150 mg/dl, fasting blood glucose greater than 100 mg/dl or high-density lipoprotein of less than 40 mg/dl in men or 50 mg/dl in women.

A "direct activator" of a sirtuin is a molecule that activates a sirtuin by binding to it. A "direct inhibitor" of a sirtuin is a molecule inhibits a sirtuin by binding to it.

The term "ED₅₀" is art-recognized. In certain embodiments, ED₅₀ means the dose of a drug which produces 50% of its maximum response or effect, or alternatively, the dose which produces a pre-determined response in 50% of test subjects or preparations. The term "LD₅₀" is art-recognized. In certain embodiments, LD₅₀ means the dose of a drug which is lethal in 50% of test subjects. The term "therapeutic index" is an art-recognized term which refers to the therapeutic index of a drug, defined as LD₅₀/ED₅₀.

The term "hyperinsulinemia" refers to a state in an individual in which the level of insulin in the blood is higher than normal.

The term "including" is used to mean "including but not limited to". "Including" and "including but not limited to" are used interchangeably.

The term "insulin resistance" refers to a state in which a normal amount of insulin produces a subnormal biologic response relative to the biological response in a subject that does not have insulin resistance.

An "insulin resistance disorder," as discussed herein, refers to any disease or condition that is caused by or contributed to by insulin resistance. Examples include: diabetes, obesity, metabolic syndrome, insulin-resistance syndromes, syndrome X, insulin resistance, high blood pressure, hypertension, high blood cholesterol, dyslipidemia, hyperlipidemia, dyslipidemia, atherosclerotic disease including stroke, coronary artery disease or myocardial infarction, hyperglycemia, hyperinsulinemia and/or hyperproinsulinemia, impaired glucose tolerance, delayed insulin release, diabetic complications, including coronary heart disease, angina pectoris, congestive heart failure, stroke, cognitive functions in dementia, retinopathy, peripheral neuropathy, nephropathy, glomerulonephritis, glomerulosclerosis, nephrotic syndrome, hypertensive nephrosclerosis some types of cancer (such as endometrial, breast, prostate, and colon), complications of pregnancy, poor female reproductive health (such as menstrual irregularities, infertility, irregular ovulation, polycystic ovarian syndrome (PCOS)), lipodystrophy, cholesterol related disorders, such as gallstones, cholescystitis and cholelithiasis, gout, obstructive sleep apnea and respiratory problems, osteoarthritis, and prevention and treatment of bone loss, e.g. osteoporosis.

The term "livestock animals" refers to domesticated quadrupeds, which includes those being raised for meat and various byproducts, e.g., a bovine animal including cattle and other members of the genus Bos, a porcine animal including domestic swine and other members of the genus Sus, an ovine animal including sheep and other members of the genus Ovis, domestic goats and other members of the genus Capra; domesticated quadrupeds being raised for specialized tasks such as use as a beast of burden, e.g., an equine animal including domestic horses and other members of the family Equidae, genus Equus.

The term "mammal" is known in the art, and exemplary mammals include humans, primates, livestock animals (including bovines, porcines, etc.), companion animals (e.g., canines, felines, etc.) and rodents (e.g., mice and rats).

The term "naturally occurring form" when referring to a compound means a compound that is in a form, e.g., a composition, in which it can be found naturally. For example, since resveratrol can be found in red wine, it is present in red wine in a form that is naturally occurring. A compound is not in a form that is naturally occurring if, e.g., the compound has been purified and separated from at least some of the other molecules that are found with the compound in nature. A "naturally occurring compound" refers to a compound that can be found in nature, i.e., a compound that has not been designed by man. A naturally occurring compound may have been made by man or by nature.

A "naturally occurring compound" refers to a compound that can be found in nature, i.e., a compound that has not been designed by man. A naturally occurring compound may have been made by man or by nature. For example, resveratrol is a naturally-occurring compound. A "non-naturally occurring compound" is a compound that is not known to exist in nature or that does not occur in nature.

"Obese" individuals or individuals suffering from obesity are generally individuals having a body mass index (BMI) of at least 25 or greater. Obesity may or may not be associated with insulin resistance.

The terms "parenteral administration" and "administered parenterally" are art-recognized and refer to modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intra-articulare, subcapsular, subarachnoid, intraspinal, and intrasternal injection and infusion.

A "patient", "subject", "individual" or "host" refers to either a human or a non-human animal.

The term "percent identical" refers to sequence identity between two amino acid sequences or between two nucleotide sequences. Identity can each be determined by comparing a position in each sequence which may be aligned for purposes of comparison. When an equivalent position in the compared sequences is occupied by the same base or amino acid, then the molecules are identical at that position; when the equivalent site occupied by the same or a similar amino acid residue (e.g., similar in steric and/or electronic nature), then the molecules can be referred to as homologous (similar) at that position. Expression as a percentage of homology, similarity, or identity refers to a function of the number of identical or similar amino acids at positions shared by the compared sequences. Expression as a percentage of homology, similarity, or identity refers to a function of the number of identical or similar amino acids at positions shared by the compared sequences. Various alignment algorithms and/or programs may be used, including FASTA, BLAST, or ENTREZ. FASTA and BLAST are available as a part of the GCG sequence analysis package (University of Wisconsin, Madison, Wis.), and can be used with, e.g., default settings. ENTREZ is available through the National Center for Biotechnology Information, National Library of Medicine, National Institutes of Health, Bethesda, MD. In one embodiment, the percent identity of two sequences can be determined by the GCG program with a gap weight of 1, e.g., each amino acid gap is weighted as if it were a single amino acid or nucleotide mismatch between the two sequences.

Other techniques for alignment are described in Methods in Enzymology, vol. 266: Computer Methods for Macromolecular Sequence Analysis (1996), ed. Doolittle, Academic Press, Inc., a division of Harcourt Brace & Co., San Diego, California, USA. Preferably, an alignment program that permits gaps in the sequence is utilized to align the sequences. The Smith-Waterman is one type of algorithm that permits gaps in sequence alignments. See Meth. Mol. Biol. 70: 173-187 (1997). Also, the GAP program using the Needleman and Wunsch alignment method can be utilized to align sequences. An alternative search strategy uses MPSRCH software, which runs on a MASPAR computer. MPSRCH uses a Smith-Waterman algorithm to score sequences on a massively parallel computer. This approach improves ability to pick up distantly related matches, and is especially tolerant of small gaps and nucleotide sequence errors. Nucleic acid-encoded amino acid sequences can be used to search both protein and DNA databases.

The term "pharmaceutically acceptable carrier" is art-recognized and refers to a pharmaceutically-acceptable material, composition or vehicle, such as a liquid or solid filler, diluent, excipient, solvent or encapsulating material, involved in carrying or transporting any subject composition or component thereof. Each carrier must be "acceptable" in the sense of being compatible with the subject composition and its components and not injurious to the patient. Some examples of materials which may serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The terms "polynucleotide", and "nucleic acid" are used interchangeably. They refer to a polymeric form of nucleotides of any length, either deoxyribonucleotides or ribonucleotides, or analogs thereof. Polynucleotides may have any three-dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified, such as by conjugation with a labeling component. The term "recombinant" polynucleotide means a polynucleotide of genomic, cDNA, semisynthetic, or synthetic origin which either does not occur in nature or is linked to another polynucleotide in a nonnatural arrangement.

The term "prophylactic" or "therapeutic" treatment is art-recognized and refers to administration of a drug to a host. If it is administered prior to clinical manifestation of the unwanted condition (e.g., disease or other unwanted state of the host animal) then the treatment is prophylactic, i.e., it protects the host against developing the unwanted condition, whereas if administered after manifestation of the unwanted condition, the treatment is therapeutic (i.e., it is intended to diminish, ameliorate or maintain the existing unwanted condition or side effects therefrom).

The term "protecting group" is art-recognized and refers to temporary substituents that protect a potentially reactive functional group from undesired chemical transformations. Examples of such protecting groups include esters of carboxylic acids, silyl ethers of alcohols, and acetals and ketals of aldehydes and ketones, respectively. The field of protecting group chemistry has been reviewed by Greene and Wuts in Protective Groups in Organic Synthesis (2nd ed., Wiley: New York, 1991).

"Replicative lifespan" of a cell refers to the number of daughter cells produced by an individual "mother cell." "Chronological aging" or "chronological lifespan," on the other hand, refers to the length of time a population of non-dividing cells remains viable when deprived of nutrients. "Increasing the lifespan of a cell" or "extending the lifespan of a cell," as applied to cells or organisms, refers to increasing the number of daughter cells produced by one cell; increasing the ability of cells or organisms to cope with stresses and combat damage, e.g., to DNA, proteins; and/or increasing the ability of cells or organisms to survive and exist in a living state for longer under a particular condition, e.g., stress (for example, heatshock, osmotic stress, high energy radiation, chemically-induced stress, DNA damage, inadequate salt level, inadequate nitrogen level, or inadequate nutrient level). Lifespan can be increased by at least about 20%, 30%, 40%, 50%, 60% or between 20% and 70%, 30% and 60%, 40% and 60% or more using methods described herein.

"Sirtuin-activating compound" refers to a compound that increases the level of a sirtuin protein and/or increases at least one activity of a sirtuin protein. In an exemplary embodiment, a sirtuin-activating compound may increase at least one biological activity of a sirtuin protein by at least about 10%, 25%, 50%, 75%, 100%, or more. Exemplary biological activities of sirtuin proteins include deacetylation, e.g., of histones and p53; extending lifespan; increasing genomic stability; silencing transcription; and controlling the segregation of oxidized proteins between mother and daughter cells.

"Sirtuin-inhibiting compound" refers to a compound that decreases the level of a sirtuin protein and/or decreases at least one activity of a sirtuin protein. In an exemplary embodiment, a sirtuin-inhibiting compound may decrease at least one biological activity of a sirtuin protein by at least about 10%, 25%, 50%, 75%, 100%, or more. Exemplary biological activities of sirtuin proteins include deacetylation, e.g., of histones and p53; extending lifespan; increasing genomic stability; silencing transcription; and controlling the segregation of oxidized proteins between mother and daughter cells.

"Sirtuin-modulating compound" refers to a compound of Formulas (I)-(XII) as described herein. In exemplary embodiments, a sirtuin-modulating compound may either up regulate (e.g., activate or stimulate), down regulate (e.g., inhibit or suppress) or otherwise change a functional property or biological activity of a sirtuin protein. Sirtuin-modulating compounds may act to modulate a sirtuin protein either directly or indirectly. In certain embodiments, a sirtuin-modulating compound may be a sirtuin-activating compound or a sirtuin-inhibiting compound.

"Sirtuin protein" refers to a member of the sirtuin deacetylase protein family, or preferably to the sir2 family, which include yeast Sir2 (GenBank Accession No. P53685), *C. elegans* Sir-2.1 (GenBank Accession No. NP_501912), and human SIRT1 (GenBank Accession No. NM_012238 and NP_036370 (or AF083106)) and SIRT2 (GenBank Accession No. NMN_012237, NMN_030593, NP_036369, NP_085096, and AF083107) proteins. Other family members include the four additional yeast Sir2-like genes termed *"HST* genes" (homologues of Sir two) HST1, HST2, HST3 and HST4, and the five other human homologues hSIRT3, hSIRT4, hSIRT5, hSIRT6 and hSIRT7 (Brachmann et al. (1995) Genes Dev. 9:2888 and Frye et al. (1999) BBRC 260:273). Preferred sirtuins are those that share more similarities with SIRT1, i.e., hSIRT1, and/or Sir2 than with SIRT2, such as those members having at least part of the N-terminal sequence present in SIRT1 and absent in SIRT2 such as SIRT3 has.

"SIRT1 protein" refers to a member of the sir2 family of sirtuin deacetylases. In one embodiment, a SIRT1 protein includes yeast Sir2 (GenBank Accession No. P53685), *C. elegans* Sir-2.1 (GenBank Accession No. NP_501912), human SIRT1 (GenBank Accession No. NM_012238 and NP_036370 (or AF083106)), human SIRT2 (GenBank Accession No. NMN_012237, NMN_030593, NP_036369, NP_085096, and AF083107) proteins, and equivalents and fragments thereof. In another embodiment, a SIRT1 protein includes a polypeptide comprising a sequence consisting of, or consisting essentially of, the amino acid sequence set forth in GenBank Accession Nos. NP_036370,NP_501912, NP_085096, NP_036369, and P53685. SIRT1 proteins include polypeptides comprising all or a portion of the amino acid sequence set forth in GenBank Accession Nos. NP_036370, NP_501912, NP_085096, NP_036369, and P53685; the amino acid sequence set forth in GenBank Accession Nos. NP_036370, NP_501912, NP_085096, NP_036369, and P53685 with 1 to about 2, 3, 5, 7, 10, 15, 20, 30, 50, 75 or more conservative amino acid substitutions; an amino acid sequence that is at least 60%, 70%, 80%, 90%, 95%, 96%, 97%, 98%, or 99% identical to GenBank Accession Nos. NP_036370, NP_501912, NP_085096, NP_036369, and P53685 and functional fragments thereof. Polypeptides of the invention also include homologs (e.g., orthologs and paralogs), variants, or fragments, of GenBank Accession Nos. NP_036370, NP_501912, NP_085096, NP_036369, and P53685.

The term "substantially homologous" when used in connection with amino acid sequences, refers to sequences which are substantially identical to or similar in sequence with each other, giving rise to a homology of conformation and thus to retention, to a useful degree, of one or more biological (including immunological) activities. The term is not intended to imply a common evolution of the sequences.

The term "synthetic" is art-recognized and refers to production by *in vitro* chemical or enzymatic synthesis.

The terms "systemic administration," "administered systemically," "peripheral administration" and "administered peripherally" are art-recognized and refer to the administration of a subject composition, therapeutic or other material other than directly into the central nervous system, such that it enters the patient's system and, thus, is subject to metabolism and other like processes.

The term "therapeutic agent" is art-recognized and refers to any chemical moiety that is a biologically, physiologically, or pharmacologically active substance that acts locally or systemically in a subject. The term also means any substance intended for use in the diagnosis, cure, mitigation, treatment or prevention of disease or in the enhancement of desirable physical or mental development and/or conditions in an animal or human.

The term "therapeutic effect" is art-recognized and refers to a local or systemic effect in animals, particularly mammals, and more particularly humans caused by a pharmacologically active substance. The phrase "therapeutically-effective amount" means that amount of such a substance that produces some desired local or systemic effect at a reasonable benefit/risk ratio applicable to any treatment. The therapeutically effective amount of such substance will vary depending upon the subject and disease condition being treated, the weight and age of the subject, the severity of the disease condition, the manner of administration and the like, which can readily be determined by one of ordinary skill in the art. For example, certain compositions described herein may be administered in a sufficient amount to produce a desired effect at a reasonable benefit/risk ratio applicable to such treatment.

"Transcriptional regulatory sequence" is a generic term used throughout the specification to refer to DNA sequences, such as initiation signals, enhancers, and promoters, which induce or control transcription of protein coding sequences with which they are operable linked. In preferred embodiments, transcription of one of the recombinant genes is under the control of a promoter sequence (or other transcriptional regulatory sequence) which controls the expression of the recombinant gene in a cell-type which expression is intended. It will also be understood that the recombinant gene can be under the control of transcriptional regulatory sequences which are the same or which are different from those sequences which control transcription of the naturally-occurring forms of genes as described herein.

"Treating" a condition or disease refers to curing as well as ameliorating at least one symptom of the condition or disease.

A "vector" is a self-replicating nucleic acid molecule that transfers an inserted nucleic acid molecule into and/or between host cells. The term includes vectors that function primarily for insertion of a nucleic acid molecule into a cell, replication of vectors that function primarily for the replication of nucleic acid, and expression vectors that function for transcription and/or translation of the DNA or RNA. Also included are vectors that provide more than one of the above functions. As used herein, "expression vectors" are defined as polynucleotides which, when introduced into an appropriate host cell, can be transcribed and translated into a polypeptide(s). An "expression system" usually connotes a suitable host cell comprised of an expression vector that can function to yield a desired expression product.

### 2. Sirtuin Modulators

In one aspect, the invention provides novel sirtuin-modulating compounds for treating and/or preventing a wide variety of diseases and disorders including, for example, diseases or disorders related to aging or stress, diabetes, obesity, neurodegenerative diseases, cardiovascular disease, blood clotting disorders, inflammation, cancer, and/or flushing, etc. Other compounds disclosed herein may be suitable for use in a pharmaceutical composition and/or one or more methods disclosed herein.

In one embodiment, compounds for use in the methods described herein are represented by Structural Formula (I) or (II): 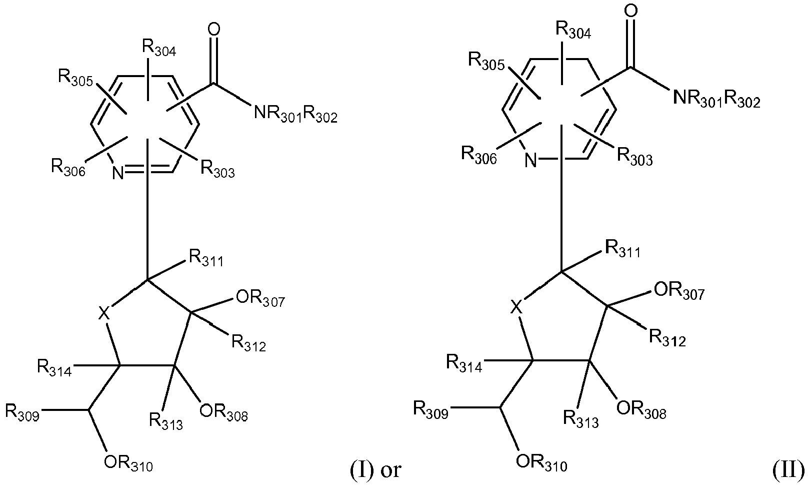 or a pharmaceutically acceptable salt thereof, where:
R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, S(O)ₙNRR', -NRR', -NRC(O)OR' and -NRC(O)R';
R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, CO₂R, -OCOR, -OCO₂R, -C(O)NRR', -OC(O)NRR', -C(O)R, -COR, -OSO₃H, S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2.

A group of suitable compounds encompassed by Structural Formulas (I) and (II) is represented by Structural Formulas (III) and (IV): 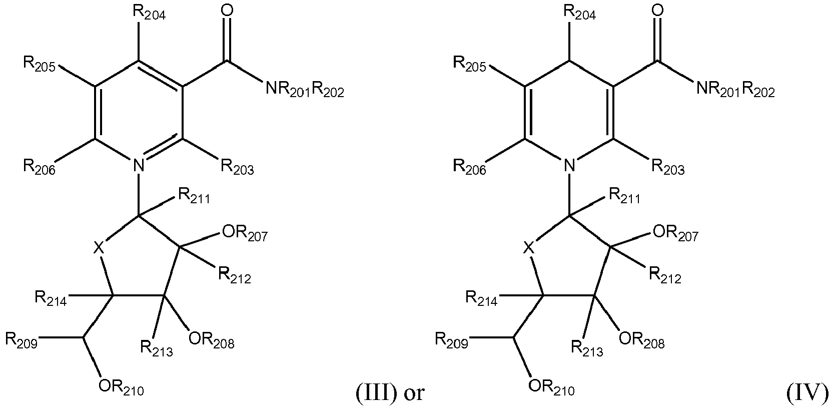 or a pharmaceutically acceptable salt thereof, where:
R₂₀₁ and R₂₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₂₀₁ and R₂₀₂ taken together with the atom to which they are attached form a substituted or unsubstituted non-aromatic heterocyclic group;
R₂₀₃, R₂₀₄, R₂₀₅ and R₂₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
R₂₀₇, R₂₀₈ and R₂₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₂₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₂₁₁, R₂₁₂, R₂₁₃ and R₂₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S, preferably O; and
n is 1 or 2.

In a particular group of compounds represented by Structural Formula (III) or (IV), at least one of R₂₀₇, R₂₀₈ and R₂₁₀ is a substituted or unsubstituted alkyl group, a substituted or unsubstituted aryl group, -C(O)R, -C(O)OR, -C(O)NHR, -C(S)R, - C(S)OR or -C(O)SR. Typically, at least one of R₂₀₇, R₂₀₈ and R₂₁₀ is -C(O)R or - C(O)OR. More typically, at least one of R₂₀₇, R₂₀₈ and R₂₁₀ is -C(O)R. In such compounds, R is preferably a substituted or unsubstituted alkyl, particularly an unsubstituted alkyl group such as methyl or ethyl.

In another particular group of compounds represented by Structural Formula (III) or (IV), R₂₀₄ is a halogen (e.g., fluorine, bromine, chlorine) or hydrogen (including a deuterium and/or tritium isotope). Suitable compounds include those where at least one of R₂₀₇, R₂₀₈ and R₂₁₀ is a substituted or unsubstituted alkyl group, a substituted or unsubstituted aryl group, -C(O)R, -C(O)OR, -C(O)NHR, -C(S)R, - C(S)OR or -C(O)SR and R₂₀₄ is a halogen or hydrogen.

Typically, for compounds represented by Structural Formulas (III) and (IV), R₂₀₃-R₂₀₆ are -H. In addition, R₂₀₉ and R₂₁₁-R₂₁₄ are typically -H. Particular compounds represented by Structural Formulas (III) and (IV) are selected such that R₂₀₃-R₂₀₆, R₂₀₉ and R₂₁₁-R₂₁₄ are all -H. For these compounds, R₂₀₄, R₂₀₇, R₂₀₈ and R₂₁₀ have the values described above.

R₂₀₁ and R₂₀₂ are typically -H or a substituted or unsubstituted alkyl group, more typically -H. In compounds having these values of R₂₀₁ and R₂₀₂, R₂₀₃-R₂₀₆, R₂₀₉ and R₂₁₁-R₂₁₄ typically have the values described above.

In certain methods of the invention, at least one of R₂₀₁-R₂₁₄ is not -H when X is O.

In certain methods of the invention, R₂₀₆ is not -H or -NH₂ when R₂₀₁-R₂₀₅ and R₂₀₇-R₂₁₄ are each -H.

In certain methods of the invention, the method does not include the reduction of a prodrug to an active agent by a NAD(P)H quinone reductase.

In certain methods of the invention, the subject being treated is not in need of an increase in nitric oxide bioactivity.

In certain methods of the invention, the method does not include the reduction of a prodrug to an active agent by a NAD(P)H quinone reductase and the subject being treated is not in need of an increase in nitric oxide bioactivity.

One embodiment of pharmaceutical compositions of the invention includes a pharmaceutically acceptable carrier or diluent and a compound represented by Structural Formula (V) or (VI): 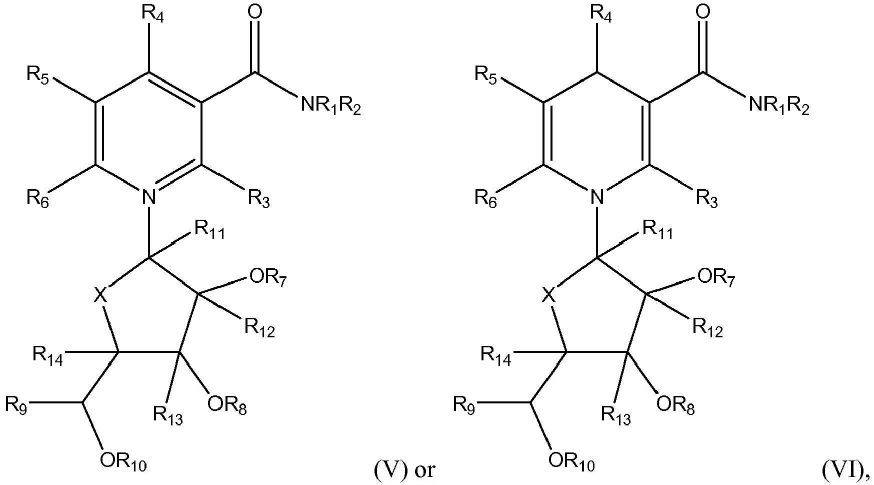 or a pharmaceutically acceptable salt thereof, wherein:
R₁ and R₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁ and R₂ taken together with the atom to which they are attached form a substituted or unsubstituted non-aromatic heterocyclic group, provided that when one of R₁ and R₂ is -H, the other is not an alkyl group substituted by -C(O)OCH₂CH₃;
R₃, R₄ and R₅ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
R₆ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRC(O)OR', -NO₂ and -NRC(O)R';
R₇, R₈ and R₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₉ selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₁₁, R₁₂, R₁₃ and R₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S, preferably O; and
n is 1 or 2,
provided that R₁-R₁₄ are not each -H and that R₁-R₉ and R₁₁-R₁₄ are not each -H when R₁₀ is -C(O)C₆H₅.

In certain embodiments, R₁ is -H.

In certain embodiments, R₇, R₈ and R₁₀ are independently -H, -C(O)R or -C(O)OR, typically -H or -C(O)R such as -H or -C(O)CH₃. In particular embodiments, R₁ is -H and R₇, R₈ and R₁₀ are independently -H, -C(O)R or -C(O)OR.

In certain embodiments, R₉ is -H. In particular embodiments, R₉ is -H when R₁ is -H and/or R₇, R₈ and R₁₀ are independently -H, -C(O)R or -C(O)OR.

In certain embodiments, R₂ is -H. In particular embodiments, R₂ is -H when R₉ is -H, R₁ is -H and/or R₇, R₈ and R₁₀ are independently -H, -C(O)R or -C(O)OR. Typically, R₂ is -H when R₉ is -H, R₁ is -H and R₇, R₈ and R₁₀ are independently -H, -C(O)R or -C(O)OR.

In certain embodiments, R₄ is -H or a halogen, such as deuterium or fluorine.

In one embodiment, the invention is pharmaceutical composition comprising a pharmaceutically acceptable carrier or diluent and a compound represented by Structural Formula (VII) or (VIII): 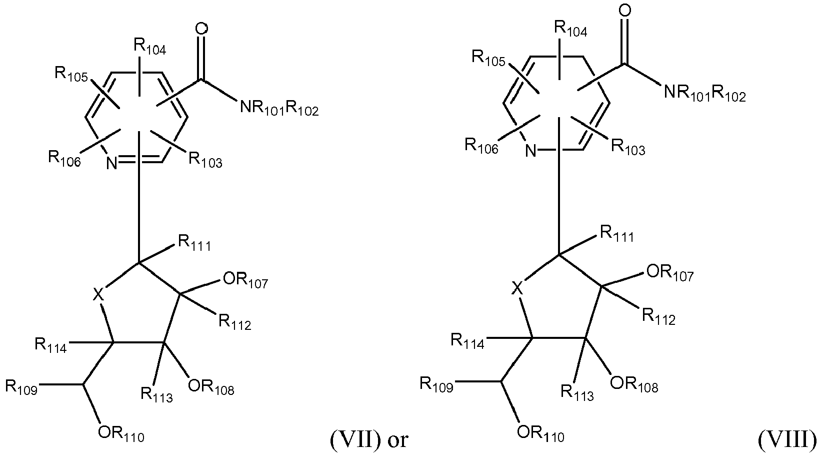 or a pharmaceutically acceptable salt thereof, wherein:
R₁₀₁ and R₁₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁₀₁ and R₁₀₂ taken together with the atom to which they are attached form a substituted or unsubstituted non-aromatic heterocyclic group;
R₁₀₃, R₁₀₄, R₁₀₅ and R₁₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
R₁₀₇ and R₁₀₈ are selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, wherein at least one of R₁₀₇ and R₁₀₈ is a substituted or unsubstituted alkyl group, a substituted or unsubstituted aryl group, -C(O)R, -C(O)OR, -C(O)NHR, -C(S)R, -C(S)OR or -C(O)SR;
R₁₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₁₁₀ is selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, provided that R₁₁₀ is not -C(O)C₆H₅;
R₁₁₁, R₁₁₂, R₁₁₃ and R₁₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2.

In another embodiment, the invention is a pharmaceutical composition comprising a pharmaceutically acceptable carrier or diluent and a compound represented by Structural Formula (IX) or (X): 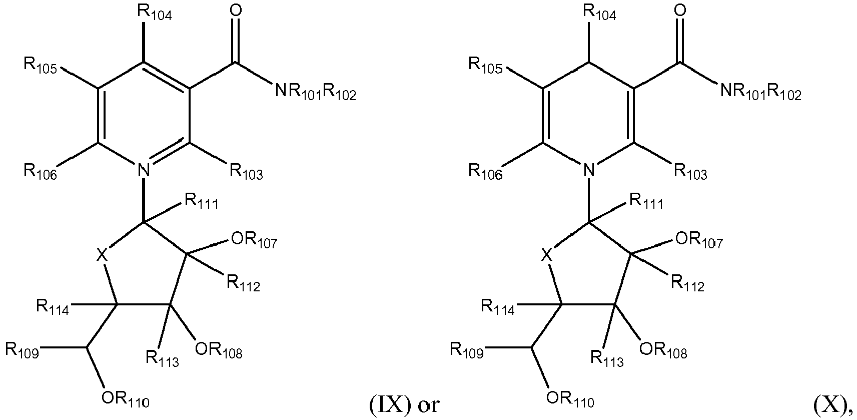 or a pharmaceutically acceptable salt thereof, where:
R₁₀₁ and R₁₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁₀₁ and R₁₀₂ taken together with the atom to which they are attached form a substituted or unsubstituted non-aromatic heterocyclic group;
R₁₀₃₅ R₁₀₄, R₁₀₅ and R₁₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H₅ -S(O)ₙR, -S(O)ₙOR, -S(O)ₙNRR', -NRR', -NRC(O)OR', -NO₂ and -NRC(O)R';
R₁₀₇ and R₁₀₈ are selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, wherein at least one of R₁₀₇ and R₁₀₈ is a substituted or unsubstituted alkyl group, a substituted or unsubstituted aryl group, -C(O)R, -C(O)OR, -C(O)NHR, -C(S)R, -C(S)OR or -C(O)SR;
R₁₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
R₁₁₀ is selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, provided that R₁₁₀ is not -C(O)C₆H₅;
R₁₁₁, R₁₁₂, R₁₁₃ and R₁₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2.

For compounds represented by Structural Formulas (VII)-(X), typically at least one of R₁₀₇ and R₁₀₈ is -C(O)R, such as -C(O)CH₃. In particular embodiments, R₁₀₇, R₁₀₈ and R₁₁₀ are independently -H or -C(O)R (e.g., -C(O)CH₃).

In certain embodiments, such as when R₁₀₇, R₁₀₈ and R₁₁₀ have the values described above, R₁₀₁ and R₁₀₂ are each -H.

In certain embodiments, R₁₀₉ is -H.

In certain embodiments, R₁₀₃-R₁₀₆ are each -H.

In certain embodiments, R₁₁₁-R₁₁₄ are each -H.

In particular embodiments, R₁₀₇, R₁₀₈ and R₁₁₀ have the values described above and R₁₀₁-R₁₀₆, R₁₀₉ and R₁₁₁-R₁₁₄ are each -H.

In certain embodiments, R₁₀₄ is -H or a halogen, typically deuterium or fluorine. The remaining values are as described above.

Compounds included in pharmaceutical compositions of the invention can also be used in the methods described above.

For compounds represented by Structural Formula (XI) or (XII): 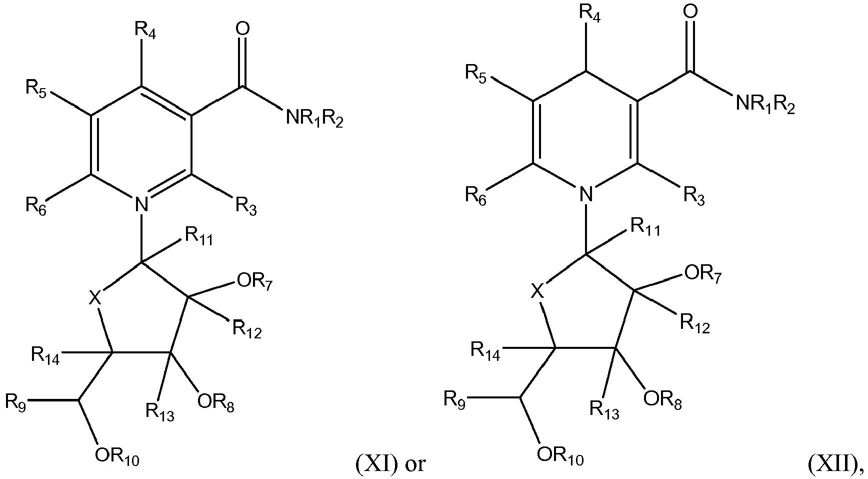 R₄ in certain embodiments is -H (e.g., deuterium, tritium) or a halogen (e.g., fluorine, bromine, chlorine).

In embodiments of the invention where R₁-R₆ can each be -H, they typically are each -H. In embodiments of the invention where one of R₁-R₆ is not -H, typically the remaining values are each -H and the non-H value is a substituted or unsubstituted alkyl group or a halogen (R₁ and R₂ are typically a substituted or unsubstituted alkyl group).

In certain embodiments, R₁₁-R₁₄ are each -H. When R₁₁-R₁₄ are each -H, R₁-R₆ typically have the values described above.

In certain embodiments, R₉ is -H. When R₉ is -H, typically R₁₁-R₁₄ are each -H and R₁-R₆ have the values described above.

Novel compounds of the invention can also be used in the methods described above.

The compounds and salts thereof described herein also include their corresponding hydrates (e.g., hemihydrate, monohydrate, dihydrate, trihydrate, tetrahydrate) and solvates. Suitable solvents for preparation of solvates and hydrates can generally be selected by a skilled artisan.

The compounds and salts thereof can be present in amorphous or crystalline (including co-crystalline and polymorph) forms.

Sirtuin-modulating compounds also include the related secondary metabolites, such as phosphate, sulfate, acyl (e.g., acetyl, fatty acid acyl) and sugar (e.g., glucurondate, glucose) derivatives (e.g., of hydroxyl groups), particularly the sulfate, acyl and sugar derivatives. In other words, substituent groups -OH also include -OSO₃⁻ M⁺, where M⁺ is a suitable cation (preferably H⁺, NH₄⁺ or an alkali metal ion such as Na⁺ or K⁺) and sugars such as 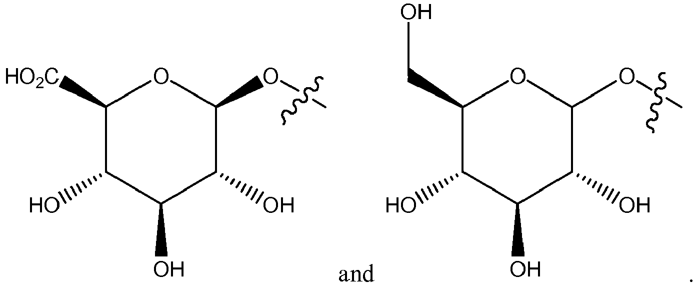 These groups are generally cleavable to -OH by hydrolysis or by metabolic (e.g., enzymatic) cleavage.

In certain embodiments, compounds having Structural Formula A are excluded from the compounds, pharmaceutical compositions and/or methods of the invention: 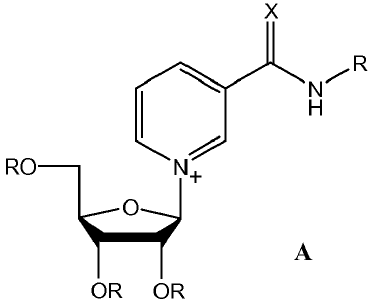 wherein
R represents independently for each occurrence H, acetyl, benzoyl, acyl, phosphate, sulfate, (alkyoxy)methyl, triarylmethyl, (trialkyl)silyl, (dialkyl)(aryl)silyl, (alkyl)(diaryl)silyl, or (triaryl)silyl; and
X represents O or S.

Sirtuin-modulating compounds of the invention advantageously modulate the level and/or activity of a sirtuin protein, particularly the deacetylase activity of the sirtuin protein.

Separately or in addition to the above properties, certain sirtuin-modulating compounds of the invention do not substantially have one or more of the following activities: inhibition of PI3-kinase, inhibition of aldoreductase, inhibition of tyrosine kinase, transactivation of EGFR tyrosine kinase, coronary dilation, or spasmolytic activity, at concentrations of the compound that are effective for modulating the deacetylation activity of the SIRT1 protein.

An alkyl group is a straight chained, branched or cyclic non-aromatic hydrocarbon which is completely saturated. Typically, a straight chained or branched alkyl group has from 1 to about 20 carbon atoms, preferably from 1 to about 10, and a cyclic alkyl group has from 3 to about 10 carbon atoms, preferably from 3 to about 8. Examples of straight chained and branched alkyl groups include methyl, ethyl, n-propyl, iso-propyl, n-butyl, sec-butyl, tert-butyl, pentyl, hexyl, pentyl and octyl. A C1-C4 straight chained or branched alkyl group is also referred to as a "lower alkyl" group.

An alkenyl group is a straight chained, branched or cyclic non-aromatic hydrocarbon which contains one or more double bonds. Typically, the double bonds are not located at the terminus of the alkenyl group, such that the double bond is not adjacent to another functional group.

An alkynyl group is a straight chained, branched or cyclic non-aromatic hydrocarbon which contains one or more triple bonds. Typically, the triple bonds are not located at the terminus of the alkynyl group, such that the triple bond is not adjacent to another functional group.

A cyclic ring (e.g., a 5- to 7-membered ring) includes carbocyclic and heterocyclic rings. Such rings can be saturated or unsaturated, including aromatic. Heterocyclic rings typically contain 1 to 4 heteroatoms, although oxygen and sulfur atoms cannot be adjacent to each other.

Aromatic (aryl) groups include carbocyclic aromatic groups such as phenyl, naphthyl, and anthracyl, and heteroaryl groups such as imidazolyl, thienyl, furanyl, pyridyl, pyrimidyl, pyranyl, pyrazolyl, pyrroyl, pyrazinyl, thiazolyl, oxazolyl, and tetrazolyl.

Aromatic groups also include fused polycyclic aromatic ring systems in which a carbocyclic aromatic ring or heteroaryl ring is fused to one or more other heteroaryl rings. Examples include benzothienyl, benzofuranyl, indolyl, quinolinyl, benzothiazole, benzooxazole, benzimidazole, quinolinyl, isoquinolinyl and isoindolyl.

Non-aromatic heterocyclic rings are non-aromatic carbocyclic rings which include one or more heteroatoms such as nitrogen, oxygen or sulfur in the ring. The ring can be five, six, seven or eight-membered. Examples include tetrahydrofuranyl, tetrahyrothiophenyl, morpholino, thiomorpholino, pyrrolidinyl, piperazinyl, piperidinyl, and thiazolidinyl, along with the cyclic form of sugars.

A ring fused to a second ring shares at least one common bond.

Suitable substituents on an alkyl, alkenyl, alkynyl, aryl, non-aromatic heterocyclic or aryl group (carbocyclic and heteroaryl) are those which do not substantially interfere with the ability of the disclosed compounds to have one or more of the properties disclosed herein. A substituent substantially interferes with the properties of a compound when the magnitude of the property is reduced by more than about 50% in a compound with the substituent compared with a compound without the substituent. Examples of suitable substituents include -OH, halogen (-Br, -Cl, -I and -F), -OR^{a}, -O-COR^{a}, -COR^{a}, -C(O)R^{a}, -CN, -NO², -COOH, -COOR^{a}, -OCO₂R^{a}, -C(O)NR^{a}R^{b}, -OC(O)NR^{a}R^{b}, -SO₃H, -NH₂, -NHR^{a}, -N(R^{a}R^{b}), -COOR^{a}, -CHO, -CONH₂, -CONHR^{a}, -CON(R^{a}R^{b}), -NHCOR^{a}, -NRCOR^{a}, -NHCONH₂, -NHCONR^{a}H, -NHCON(R^{a}R^{b}), -NR^{c}CONH₂, -NR^{c}CONR^{a}H, -NR^{c}CON(R^{a}R^{b}), -C(=NH)-NH₂, -C(=NH)-NHR^{a}, -C(=NH)-N(R^{a}R^{b}), -C(=NR^{c})-NH₂, -C(=NR^{c})-NHR^{a}, -C(=NR^{c})-N(R^{a}R^{b}), -NH-C(=NH)-NH₂, -NH-C(=NH)-NHR^{a}, -NH-C(=NH)-N(R^{a}R^{b}), -NH-C(=NR^{c})-NH₂, -NH-C(=NR^{c})-NHR^{a}, -NH-C(=NR^{c})-N(R^{a}R^{b}), -NR^{d}H-C(=NH)-NH₂, -NR^{d}-C(=NH)-NHR^{a}, -NR^{d}-C(=NH)-N(R^{a}R^{b}), -NR^{d}-C(=NR^{c})-NH₂, -NR^{d}-C(=NR^{c})-NHR^{a}, -NR^{d}-C(=NR^{c})-N(R^{a}R^{b}), -NHNH₂, -NHNHR^{a}, -NHR^{a}R^{b}, -SO₂NH₂, -SO₂NHRₐ, -SO₂NR^{a}R^{b}, -CH=CHR^{a}, -CH=CR^{a}R^{b}, -CR^{c}=CR^{a}R^{b}, CR^{c}=CHR^{a}, -CR^{c}=CR^{a}R^{b}, -CCR^{a}, -SH, -SOₖR^{a} (k is 0, 1 or 2), -S(O)ₖOR^{a} (k is 0, 1 or 2) and -NH-C(=NH)-NH₂. R^{a}-R^{d} are each independently an aliphatic, substituted aliphatic, benzyl, substituted benzyl, aromatic or substituted aromatic group, preferably an alkyl, benzylic or aryl group. In addition, -NR^{a}R^{b}, taken together, can also form a substituted or unsubstituted non-aromatic heterocyclic group. A non-aromatic heterocyclic group, benzylic group or aryl group can also have an aliphatic or substituted aliphatic group as a substituent. A substituted aliphatic group can also have a non-aromatic heterocyclic ring, a substituted a non-aromatic heterocyclic ring, benzyl, substituted benzyl, aryl or substituted aryl group as a substituent. A substituted aliphatic, non-aromatic heterocyclic group, substituted aryl, or substituted benzyl group can have more than one substituent.

A sugar is an aldehyde or ketone derivative of a straight-chain polyhydroxy alcohol, which contains at least three carbon atoms. A sugar can exist as a linear molecule or, preferably, as a cyclic molecule (e.g., in the pyranose or furanose form). Preferably, a sugar is a monosaccharide such as glucose or glucuronic acid. In embodiments of the invention where, for example, prolonged residence of a compound derivatized with a sugar is desired, the sugar is preferably a non-naturally occurring sugar. For example, one or more hydroxyl groups are substituted with another group, such as a halogen (e.g., chlorine). The stereochemical configuration at one or more carbon atoms can also be altered, as compared to a naturally occurring sugar. One example of a suitable non-naturally occurring sugar is sucralose.

A fatty acid is a carboxylic acid having a long-chained hydrocarbon moiety. Typically, a fatty acid has an even number of carbon atoms ranging from 12 to 24, often from 14 to 20. Fatty acids can be saturated or unsaturated and substituted or unsubstituted, but are typically unsubstituted. Fatty acids can be naturally or non-naturally occurring. In embodiments of the invention where, for example, prolonged residence time of a compound having a fatty acid moiety is desired, the fatty acid is preferably non-naturally occurring. The acyl group of a fatty acid consists of the hydrocarbon moiety and the carbonyl moiety of the carboxylic acid functionality, but excludes the -OH moiety associated with the carboxylic acid functionality.

Also included in the present invention are salts, particularly pharmaceutically acceptable salts, of the sirtuin-modulating compounds described herein. The compounds of the present invention that possess a sufficiently acidic, a sufficiently basic, or both functional groups, can react with any of a number of inorganic bases, and inorganic and organic acids, to form a salt. Alternatively, compounds that are inherently charged, such as those with a quaternary nitrogen, can form a salt with an appropriate counterion (e.g., a halide such as bromide, chloride, or fluoride, particularly bromide).

Acids commonly employed to form acid addition salts are inorganic acids such as hydrochloric acid, hydrobromic acid, hydroiodic acid, sulfuric acid, phosphoric acid, and the like, and organic acids such as p-toluenesulfonic acid, methanesulfonic acid, oxalic acid, p-bromophenyl-sulfonic acid, carbonic acid, succinic acid, citric acid, benzoic acid, acetic acid, and the like. Examples of such salts include the sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, gamma-hydroxybutyrate, glycolate, tartrate, methanesulfonate, propanesulfonate, naphthalene-1-sulfonate, naphthalene-2-sulfonate, mandelate, and the like.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, and the like.

In an exemplary embodiment, a sirtuin-modulating compound may traverse the cytoplasmic membrane of a cell. For example, a compound may have a cell-permeability of at least about 20%, 50%, 75%, 80%, 90% or 95%.

Sirtuin-modulating compounds described herein may also have one or more of the following characteristics: the compound may be essentially non-toxic to a cell or subject; the sirtuin-modulating compound may be an organic molecule or a small molecule of 2000 amu or less, 1000 amu or less; a compound may have a half-life under normal atmospheric conditions of at least about 30 days, 60 days, 120 days, 6 months or 1 year; the compound may have a half-life in solution of at least about 30 days, 60 days, 120 days, 6 months or 1 year; a sirtuin-modulating compound may be more stable in solution than resveratrol by at least a factor of about 50%, 2 fold, 5 fold, 10 fold, 30 fold, 50 fold or 100 fold; a sirtuin-modulating compound may promote deacetylation of the DNA repair factor Ku70; a sirtuin-modulating compound may promote deacetylation of RelA/p65; a compound may increase general turnover rates and enhance the sensitivity of cells to TNF-induced apoptosis.

In certain embodiments, a sirtuin-modulating compound does not have any substantial ability to inhibit a histone deacetylase (HDACs) class I, a HDAC class II, or HDACs I and II, at concentrations (e.g., in vivo) effective for modulating the deacetylase activity of the sirtuin. For instance, in preferred embodiments the sirtuin-modulating compound is a sirtuin-activating compound and is chosen to have an EC₅₀ for activating sirtuin deacetylase activity that is at least 5 fold less than the EC₅₀ for inhibition of an HDAC I and/or HDAC II, and even more preferably at least 10 fold, 100 fold or even 1000 fold less. Methods for assaying HDAC I and/or HDAC II activity are well known in the art and kits to perform such assays may be purchased commercially. See e.g., BioVision, Inc. (Mountain View, CA; world wide web at biovision.com) and Thomas Scientific (Swedesboro, NJ; world wide web at tomassci.com).

In certain embodiments, a sirtuin-modulating compound does not have any substantial ability to modulate sirtuin homo logs. In one embodiment, an activator of a human sirtuin protein may not have any substantial ability to activate a sirtuin protein from lower eukaryotes, particularly yeast or human pathogens, at concentrations (e.g., in vivo) effective for activating the deacetylase activity of human sirtuin. For example, a sirtuin-activating compound may be chosen to have an EC₅₀ for activating a human sirtuin, such as SIRT1, deacetylase activity that is at least 5 fold less than the EC₅₀ for activating a yeast sirtuin, such as Sir2 (such as Candida, S. cerevisiae, etc.), and even more preferably at least 10 fold, 100 fold or even 1000 fold less. In another embodiment, an inhibitor of a sirtuin protein from lower eukaryotes, particularly yeast or human pathogens, does not have any substantial ability to inhibit a sirtuin protein from humans at concentrations (e.g., in vivo) effective for inhibiting the deacetylase activity of a sirtuin protein from a lower eukaryote. For example, a sirtuin-inhibiting compound may be chosen to have an IC₅₀ for inhibiting a human sirtuin, such as SIRT1, deacetylase activity that is at least 5 fold less than the IC₅₀ for inhibiting a yeast sirtuin, such as Sir2 (such as Candida, S. cerevisiae, etc.), and even more preferably at least 10 fold, 100 fold or even 1000 fold less.

In certain embodiments, a sirtuin-modulating compound may have the ability to modulate one or more sirtuin protein homo logs, such as, for example, one or more of human SIRT1, SIRT2, SIRT3, SIRT4, SIRT5, SIRT6, or SIRT7. In other embodiments, a SIRT1 modulator does not have any substantial ability to modulate other sirtuin protein homo logs, such as, for example, one or more of human SIRT2, SIRT3, SIRT4, SIRT5, SIRT6, or SIRT7, at concentrations (e.g., in vivo) effective for modulating the deacetylase activity of human SIRT1. For example, a sirtuin-modulating compound may be chosen to have an ED₅₀ for modulating human SIRT1 deacetylase activity that is at least 5 fold less than the ED₅₀ for modulating one or more of human SIRT2, SIRT3, SIRT4, SIRT5, SIRT6, or SIRT7, and even more preferably at least 10 fold, 100 fold or even 1000 fold less.

In certain embodiments, a sirtuin-modulating compound may have a binding affinity for a sirtuin protein of about 10⁻⁹M, 10⁻¹⁰M, 10⁻¹¹M, 10⁻¹²M or less. A sirtuin-modulating compound may reduce the Kₘ of a sirtuin protein for its substrate or NAD+ by a factor of at least about 2, 3, 4, 5, 10, 20, 30, 50 or 100. A sirtuin-modulating compound may increase the Vₘₐₓ of a sirtuin protein by a factor of at least about 2, 3, 4, 5, 10, 20, 30, 50 or 100. A sirtuin-modulating compound may have an ED₅₀ for modulating the deacetylase activity of a SIRT1 protein of less than about 1 nM, less than about 10 nM, less than about 100 nM, less than about 1 µM, less than about 10 µM, less than about 100 µM, or from about 1-10 nM, from about 10-100 nM, from about 0.1-1 µM, from about 1-10 µM or from about 10-100 µM. A sirtuin-modulating compound may modulate the deacetylase activity of a SIRT1 protein by a factor of at least about 5, 10, 20, 30, 50, or 100, as measured in a cellular assay or in a cell based assay. A sirtuin-activating compound may cause at least about 10%, 30%, 50%, 80%, 2 fold, 5 fold, 10 fold, 50 fold or 100 fold greater induction of the deacetylase activity of a sirtuin protein relative to the same concentration of resveratrol. A sirtuin-modulating compound may have an ED₅₀ for modulating SIRT5 that is at least about 10 fold, 20 fold, 30 fold, 50 fold greater than that for modulating SIRT1.

### 3. Exemplary Uses

In certain aspects, the invention provides methods for modulating the level and/or activity of a sirtuin protein and methods of use thereof.

In certain embodiments, the invention provides methods for using sirtuin-modulating compounds wherein the sirtuin-modulating compounds activate a sirtuin protein, e.g., increase the level and/or activity of a sirtuin protein. Sirtuin-modulating compounds that increase the level and/or activity of a sirtuin protein may be useful for a variety of therapeutic applications including, for example, increasing the lifespan of a cell, and treating and/or preventing a wide variety of diseases and disorders including, for example, diseases or disorders related to aging or stress, diabetes, obesity, neurodegenerative diseases, cardiovascular disease, blood clotting disorders, inflammation, cancer, and/or flushing, etc. The methods comprise administering to a subject in need thereof a pharmaceutically effective amount of a sirtuin-modulating compound, e.g., a sirtuin-activating compound.

In other embodiments, the invention provides methods for using sirtuin-modulating compounds wherein the sirtuin-modulating compounds decrease sirtuin activity, e.g., decrease the level and/or activity of a sirtuin protein. Sirtuin-modulating compounds that decrease the level and/or activity of a sirtuin protein may be useful for a variety of therapeutic application including, for example, increasing cellular sensitivity to stress (including increasing radiosensitivity and/or chemosensitivity), increasing the amount and/or rate of apoptosis, treatment of cancer (optionally in combination another chemotherapeutic agent), stimulation of appetite, and/or stimulation of weight gain, etc. The methods comprise administering to a subject in need thereof a pharmaceutically effective amount of a sirtuin-modulating compound, e.g., a sirtuin-inhibiting compound.

In certain embodiments, the sirtuin-modulating compounds described herein may be taken alone or in combination with other compounds. In one embodiment, a mixture of two or more sirtuin-modulating compounds may be administered to a subject in need thereof. In another embodiment, a sirtuin-modulating compound that increases the level and/or activity of a sirtuin protein may be administered with one or more of the following compounds: resveratrol, butein, fisetin, piceatannol, or quercetin. In an exemplary embodiment, a sirtuin-modulating compound that increases the level and/or activity of a sirtuin protein may be administered in combination with nicotinic acid. In another embodiment, a sirtuin-activating compound that decreases the level and/or activity of a sirtuin protein may be administered with one or more of the following compounds: nicotinamide (NAM), suranim; NF023 (a G-protein antagonist); NF279 (a purinergic receptor antagonist); Trolox (6-hydroxy-2,5,7,8,tetramethylchroman-2-carboxylic acid); (-)-epigallocatechin (hydroxy on sites 3,5,7,3',4',5'); (-)-epigallocatechin gallate (Hydroxy sites 5,7,3',4',5' and gallate ester on 3); cyanidin choloride (3,5,7,3',4'-pentahydroxyflavylium chloride); delphinidin chloride (3,5,7,3',4',5'-hexahydroxyflavylium chloride); myricetin (cannabiscetin; 3,5,7,3',4',5'-hexahydroxyflavone); 3,7,3',4',5'-pentahydroxyflavone; gossypetin (3,5,7,8,3',4'-hexahydroxyflavone), sirtinol; and splitomicin (see e.g., Howitz et al. (2003) Nature 425:191; Grozinger et al. (2001) J. Biol. Chem. 276:38837; Dedalov et al. (2001) PNAS 98:15113; and Hirao et al. (2003) J. Biol. Chem 278:52773). In yet another embodiment, one or more sirtuin-modulating compounds may be administered with one or more therapeutic agents for the treatment or prevention of various diseases, including, for example, cancer, diabetes, neurodegenerative diseases, cardiovascular disease, blood clotting, inflammation, flushing, obesity, ageing, stress, etc. In various embodiments, combination therapies comprising a sirtuin-modulating compound may refer to (1) pharmaceutical compositions that comprise one or more sirtuin-modulating compounds in combination with one or more therapeutic agents; and (2) co-administration of one or more sirtuin-modulating compounds with one or more therapeutic agents wherein the sirtuin-modulating compound and therapeutic agent have not been formulated in the same compositions. When using separate formulations, the sirtuin-modulating compound may be administered at the same, intermittent, staggered, prior to, subsequent to, or combinations thereof, with the administration of another therapeutic agent.

In certain embodiments, methods for reducing, preventing or treating diseases or disorders using a sirtuin-modulating compound may also comprise increasing the protein level of a sirtuin, such as human SIRT1 or homologs thereof. Increasing protein levels can be achieved by introducing into a cell one or more copies of a nucleic acid that encodes a sirtuin. For example, the level of a sirtuin can be increased in a mammalian cell by introducing into the mammalian cell a nucleic acid encoding the sirtuin, e.g., increasing the level of SIRT1 by introducing a nucleic acid encoding the amino acid sequence set forth in GenBank Accession No. NP_036370. The nucleic acid may be under the control of a promoter that regulates the expression of the SIRT1 nucleic acid. Alternatively, the nucleic acid may be introduced into the cell at a location in the genome that is downstream of a promoter. Methods for increasing the level of a protein using these methods are well known in the art.

A nucleic acid that is introduced into a cell to increase the protein level of a sirtuin may encode a protein that is at least about 80%, 85%, 90%, 95%, 98%, or 99% identical to the sequence of a sirtuin, e.g., GenBank Accession No. NP_036370. For example, the nucleic acid encoding the protein may be at least about 80%, 85%, 90%, 95%, 98%, or 99% identical to GenBank Accession No. NM_012238. The nucleic acid may also be a nucleic acid that hybridizes, preferably under stringent hybridization conditions, to a nucleic acid encoding a wild-type sirtuin, e.g., GenBank Accession No. NM_012238. Stringent hybridization conditions may include hybridization and a wash in 0.2 x SSC at 65 °C. When using a nucleic acid that encodes a protein that is different from a wild-type sirtuin protein, such as a protein that is a fragment of a wild-type sirtuin, the protein is preferably biologically active, e.g., is capable of deacetylation. It is only necessary to express in a cell a portion of the sirtuin that is biologically active. For example, a protein that differs from wild-type SIRT1 having GenBank Accession No. NP_036370, preferably contains the core structure thereof. The core structure sometimes refers to amino acids 62-293 of GenBank Accession No. NP_036370, which are encoded by nucleotides 237 to 932 of GenBank Accession No. NMN_012238, which encompasses the NAD binding as well as the substrate binding domains. The core domain of SIRT1 may also refer to about amino acids 261 to 447 of GenBank Accession No. NP_036370, which are encoded by nucleotides 834 to 1394 of GenBank Accession No. NM_012238; to about amino acids 242 to 493 of GenBank Accession No. NP_036370, which are encoded by nucleotides 777 to 1532 of GenBank Accession No. NM 012238; or to about amino acids 254 to 495 of GenBank Accession No. NP_036370, which are encoded by nucleotides 813 to 1538 of GenBank Accession No. NM_012238. Whether a protein retains a biological function, e.g., deacetylation capabilities, can be determined according to methods known in the art.

In certain embodiments, methods for reducing, preventing or treating diseases or disorders using a sirtuin-modulating compound may also comprise decreasing the protein level of a sirtuin, such as human SIRT1 or homo logs thereof. Decreasing a sirtuin protein level can be achieved according to methods known in the art. For example, an siRNA, an antisense nucleic acid, or a ribozyme targeted to the sirtuin can be expressed in the cell. A dominant negative sirtuin mutant, e.g., a mutant that is not capable of deacetylating, may also be used. For example, mutant H363Y of SIRT1, described, e.g., in Luo et al. (2001) Cell 107:137 can be used. Alternatively, agents that inhibit transcription can be used.

Methods for modulating sirtuin protein levels also include methods for modulating the transcription of genes encoding sirtuins, methods for stabilizing/destabilizing the corresponding mRNAs, and other methods known in the art.

### Aging/Stress

In one embodiment, the invention provides a method extending the lifespan of a cell, extending the proliferative capacity of a cell, slowing aging of a cell, promoting the survival of a cell, delaying cellular senescence in a cell, mimicking the effects of calorie restriction, increasing the resistance of a cell to stress, or preventing apoptosis of a cell, by contacting the cell with a sirtuin-modulating compound of the invention that increases the level and/or activity of a sirtuin protein. In an exemplary embodiment, the methods comprise contacting the cell with a sirtuin-activating compound.

The methods described herein may be used to increase the amount of time that cells, particularly primary cells (i.e., cells obtained from an organism, e.g., a human), may be kept alive in a cell culture. Embryonic stem (ES) cells and pluripotent cells, and cells differentiated therefrom, may also be treated with a sirtuin-modulating compound that increases the level and/or activity of a sirtuin protein to keep the cells, or progeny thereof, in culture for longer periods of time. Such cells can also be used for transplantation into a subject, e.g., after *ex vivo* modification.

In one embodiment, cells that are intended to be preserved for long periods of time may be treated with a sirtuin-modulating compound that increases the level and/or activity of a sirtuin protein. The cells may be in suspension (e.g., blood cells, serum, biological growth media, etc.) or in tissues or organs. For example, blood collected from an individual for purposes of transfusion may be treated with a sirtuin-modulating compound that increases the level and/or activity of a sirtuin protein to preserve the blood cells for longer periods of time. Additionally, blood to be used for forensic purposes may also be preserved using a sirtuin-modulating compound that increases the level and/or activity of a sirtuin protein. Other cells that may be treated to extend their lifespan or protect against apoptosis include cells for consumption, e.g., cells from non-human mammals (such as meat) or plant cells (such as vegetables).

Sirtuin-modulating compounds that increase the level and/or activity of a sirtuin protein may also be applied during developmental and growth phases in mammals, plants, insects or microorganisms, in order to, e.g., alter, retard or accelerate the developmental and/or growth process.

In another embodiment, sirtuin-modulating compounds that increase the level and/or activity of a sirtuin protein may be used to treat cells useful for transplantation or cell therapy, including, for example, solid tissue grafts, organ transplants, cell suspensions, stem cells, bone marrow cells, etc. The cells or tissue may be an autograft, an allograft, a syngraft or a xenograft. The cells or tissue may be treated with the sirtuin-modulating compound prior to administration/implantation, concurrently with administration/implantation, and/or post administration/implantation into a subject. The cells or tissue may be treated prior to removal of the cells from the donor individual, *ex vivo* after removal of the cells or tissue from the donor individual, or post implantation into the recipient. For example, the donor or recipient individual may be treated systemically with a sirtuin-modulating compound or may have a subset of cells/tissue treated locally with a sirtuin-modulating compound that increases the level and/or activity of a sirtuin protein. In certain embodiments, the cells or tissue (or donor/recipient individuals) may additionally be treated with another therapeutic agent useful for prolonging graft survival, such as, for example, an immunosuppressive agent, a cytokine, an angiogenic factor, etc.

In yet other embodiments, cells may be treated with a sirtuin-modulating compound that increases the level and/or activity of a sirtuin protein *in vivo,* e.g., to increase their lifespan or prevent apoptosis. For example, skin can be protected from aging (e.g., developing wrinkles, loss of elasticity, etc.) by treating skin or epithelial cells with a sirtuin-modulating compound that increases the level and/or activity of a sirtuin protein. In an exemplary embodiment, skin is contacted with a pharmaceutical or cosmetic composition comprising a sirtuin-modulating compound that increases the level and/or activity of a sirtuin protein. Exemplary skin afflictions or skin conditions that may be treated in accordance with the methods described herein include disorders or diseases associated with or caused by inflammation, sun damage or natural aging. For example, the compositions find utility in the prevention or treatment of contact dermatitis (including irritant contact dermatitis and allergic contact dermatitis), atopic dermatitis (also known as allergic eczema), actinic keratosis, keratinization disorders (including eczema), epidermolysis bullosa diseases (including penfigus), exfoliative dermatitis, seborrheic dermatitis, erythemas (including erythema multiforme and erythema nodosum), damage caused by the sun or other light sources, discoid lupus erythematosus, dermatomyositis, psoriasis, skin cancer and the effects of natural aging. In another embodiment, sirtuin-modulating compounds that increase the level and/or activity of a sirtuin protein may be used for the treatment of wounds and/or burns to promote healing, including, for example, first-, second- or third-degree burns and/or thermal, chemical or electrical burns. The formulations may be administered topically, to the skin or mucosal tissue, as an ointment, lotion, cream, microemulsion, gel, solution or the like, as further described herein, within the context of a dosing regimen effective to bring about the desired result.

Topical formulations comprising one or more sirtuin-modulating compounds that increase the level and/or activity of a sirtuin protein may also be used as preventive, e.g., chemopreventive, compositions. When used in a chemopreventive method, susceptible skin is treated prior to any visible condition in a particular individual.

Sirtuin-modulating compounds may be delivered locally or systemically to a subject. In one embodiment, a sirtuin-modulating compound is delivered locally to a tissue or organ of a subject by injection, topical formulation, etc.

In another embodiment, a sirtuin-modulating compound that increases the level and/or activity of a sirtuin protein may be used for treating or preventing a disease or condition induced or exacerbated by cellular senescence in a subject; methods for decreasing the rate of senescence of a subject, e.g., after onset of senescence; methods for extending the lifespan of a subject; methods for treating or preventing a disease or condition relating to lifespan; methods for treating or preventing a disease or condition relating to the proliferative capacity of cells; and methods for treating or preventing a disease or condition resulting from cell damage or death. In certain embodiments, the method does not act by decreasing the rate of occurrence of diseases that shorten the lifespan of a subject. In certain embodiments, a method does not act by reducing the lethality caused by a disease, such as cancer.

In yet another embodiment, a sirtuin-modulating compound that increases the level and/or activity of a sirtuin protein may be administered to a subject in order to generally increase the lifespan of its cells and to protect its cells against stress and/or against apoptosis. It is believed that treating a subject with a compound described herein is similar to subjecting the subject to hormesis, i.e., mild stress that is beneficial to organisms and may extend their lifespan.

Sirtuin-modulating compounds that increase the level and/or activity of a sirtuin protein can also be administered to subjects for treatment of diseases, e.g., chronic diseases, associated with cell death, in order to protect the cells from cell death. Exemplary diseases include those associated with neural cell death, neuronal dysfunction, or muscular cell death or dysfunction, such as Parkinson's disease, Alzheimer's disease, multiple sclerosis, amyotropic lateral sclerosis, and muscular dystrophy; AIDS; fulminant hepatitis; diseases linked to degeneration of the brain, such as Creutzfeld-Jakob disease, retinitis pigmentosa and cerebellar degeneration; myelodysplasis such as aplastic anemia; ischemic diseases such as myocardial infarction and stroke; hepatic diseases such as alcoholic hepatitis, hepatitis B and hepatitis C; joint-diseases such as osteoarthritis; atherosclerosis; alopecia; damage to the skin due to UV light; lichen planus; atrophy of the skin; cataract; and graft rejections. Cell death can also be caused by surgery, drug therapy, chemical exposure or radiation exposure.

Sirtuin-modulating compounds that increase the level and/or activity of a sirtuin protein can also be administered to a subject suffering from an acute disease, e.g., damage to an organ or tissue, e.g., a subject suffering from stroke or myocardial infarction or a subject suffering from a spinal cord injury. Sirtuin-modulating compounds that increase the level and/or activity of a sirtuin protein may also be used to repair an alcoholic's liver.

### Cardiovascular Disease

In another embodiment, the invention provides a method for treating and/or preventing a cardiovascular disease by administering to a subject in need thereof a sirtuin-modulating compound that increases the level and/or activity of a sirtuin protein.

Cardiovascular diseases that can be treated or prevented using the sirtuin-modulating compounds that increase the level and/or activity of a sirtuin protein include cardiomyopathy or myocarditis; such as idiopathic cardiomyopathy, metabolic cardiomyopathy, alcoholic cardiomyopathy, drug-induced cardiomyopathy, ischemic cardiomyopathy, and hypertensive cardiomyopathy. Also treatable or preventable using compounds and methods described herein are atheromatous disorders of the major blood vessels (macrovascular disease) such as the aorta, the coronary arteries, the carotid arteries, the cerebrovascular arteries, the renal arteries, the iliac arteries, the femoral arteries, and the popliteal arteries. Other vascular diseases that can be treated or prevented include those related to platelet aggregation, the retinal arterioles, the glomerular arterioles, the vasa nervorum, cardiac arterioles, and associated capillary beds of the eye, the kidney, the heart, and the central and peripheral nervous systems. The sirtuin-modulating compounds that increase the level and/or activity of a sirtuin protein may also be used for increasing HDL levels in plasma of an individual.

Yet other disorders that may be treated with sirtuin-modulating compounds that increase the level and/or activity of a sirtuin protein include restenosis, e.g., following coronary intervention, and disorders relating to an abnormal level of high density and low density cholesterol.

In one embodiment, a sirtuin-modulating compound that increases the level and/or activity of a sirtuin protein may be administered as part of a combination therapeutic with another cardiovascular agent including, for example, an anti-arrhythmic agent, an antihypertensive agent, a calcium channel blocker, a cardioplegic solution, a cardiotonic agent, a fibrinolytic agent, a sclerosing solution, a vasoconstrictor agent, a vasodilator agent, a nitric oxide donor, a potassium channel blocker, a sodium channel blocker, statins, or a naturiuretic agent.

In one embodiment, a sirtuin-modulating compound that increases the level and/or activity of a sirtuin protein may be administered as part of a combination therapeutic with an anti-arrhythmia agent. Anti-arrhythmia agents are often organized into four main groups according to their mechanism of action: type I, sodium channel blockade; type II, beta-adrenergic blockade; type III, repolarization prolongation; and type IV, calcium channel blockade. Type I anti-arrhythmic agents include lidocaine, moricizine, mexiletine, tocainide, procainamide, encainide, flecanide, tocainide, phenytoin, propafenone, quinidine, disopyramide, and flecainide. Type II anti-arrhythmic agents include propranolol and esmolol. Type III includes agents that act by prolonging the duration of the action potential, such as amiodarone, artilide, bretylium, clofilium, isobutilide, sotalol, azimilide, dofetilide, dronedarone, ersentilide, ibutilide, tedisamil, and trecetilide. Type IV anti-arrhythmic agents include verapamil, diltaizem, digitalis, adenosine, nickel chloride, and magnesium ions.

In another embodiment, a sirtuin-modulating compound that increases the level and/or activity of a sirtuin protein may be administered as part of a combination therapeutic with another cardiovascular agent. Examples of cardiovascular agents include vasodilators, for example, hydralazine; angiotensin converting enzyme inhibitors, for example, captopril; anti-anginal agents, for example, isosorbide nitrate, glyceryl trinitrate and pentaerythritol tetranitrate; anti-arrhythmic agents, for example, quinidine, procainaltide and lignocaine; cardioglycosides, for example, digoxin and digitoxin; calcium antagonists, for example, verapamil and nifedipine; diuretics, such as thiazides and related compounds, for example, bendrofluazide, chlorothiazide, chlorothalidone, hydrochlorothiazide and other diuretics, for example, fursemide and triamterene, and sedatives, for example, nitrazepam, flurazepam and diazepam.

Other exemplary cardiovascular agents include, for example, a cyclooxygenase inhibitor such as aspirin or indomethacin, a platelet aggregation inhibitor such as clopidogrel, ticlopidene or aspirin, fibrinogen antagonists or a diuretic such as chlorothiazide, hydrochlorothiazide, flumethiazide, hydroflumethiazide, bendroflumethiazide, methylchlorthiazide, trichloromethiazide, polythiazide or benzthiazide as well as ethacrynic acid tricrynafen, chlorthalidone, furosemide, musolimine, bumetanide, triamterene, amiloride and spironolactone and salts of such compounds, angiotensin converting enzyme inhibitors such as captopril, zofenopril, fosinopril, enalapril, ceranopril, cilazopril, delapril, pentopril, quinapril, ramipril, lisinopril, and salts of such compounds, angiotensin II antagonists such as losartan, irbesartan or valsartan, thrombolytic agents such as tissue plasminogen activator (tPA), recombinant tPA, streptokinase, urokinase, prourokinase, and anisoylated plasminogen streptokinase activator complex (APSAC, Eminase, Beecham Laboratories), or animal salivary gland plasminogen activators, calcium channel blocking agents such as verapamil, nifedipine or diltiazem, thromboxane receptor antagonists such as ifetroban, prostacyclin mimetics, or phosphodiesterase inhibitors. Such combination products if formulated as a fixed dose employ the compounds of this invention within the dose range described above and the other pharmaceutically active agent within its approved dose range.

Yet other exemplary cardiovascular agents include, for example, vasodilators, e.g., bencyclane, cinnarizine, citicoline, cyclandelate, cyclonicate, ebumamonine, phenoxezyl, flunarizine, ibudilast, ifenprodil, lomerizine, naphlole, nikamate, nosergoline, nimodipine, papaverine, pentifylline, nofedoline, vincamin, vinpocetine, vichizyl, pentoxifylline, prostacyclin derivatives (such as prostaglandin E1 and prostaglandin 12), an endothelin receptor blocking drug (such as bosentan), diltiazem, nicorandil, and nitroglycerin. Examples of the cerebral protecting drug include radical scavengers (such as edaravone, vitamin E, and vitamin C), glutamate antagonists, AMPA antagonists, kainate antagonists, NMDA antagonists, GABA agonists, growth factors, opioid antagonists, phosphatidylcholine precursors, serotonin agonists, Na⁺/Ca²⁺ channel inhibitory drugs, and K⁺ channel opening drugs. Examples of the brain metabolic stimulants include amantadine, tiapride, and gamma-aminobutyric acid. Examples of the anticoagulant include heparins (such as heparin sodium, heparin potassium, dalteparin sodium, dalteparin calcium, heparin calcium, parnaparin sodium, reviparin sodium, and danaparoid sodium), warfarin, enoxaparin, argatroban, batroxobin, and sodium citrate. Examples of the antiplatelet drug include ticlopidine hydrochloride, dipyridamole, cilostazol, ethyl icosapentate, sarpogrelate hydrochloride, dilazep hydrochloride, trapidil, a nonsteroidal antiinflammatory agent (such as aspirin), beraprostsodium, iloprost, and indobufene. Examples of the thrombolytic drug include urokinase, tissue-type plasminogen activators (such as alteplase, tisokinase, nateplase, pamiteplase, monteplase, and rateplase), and nasaruplase. Examples of the antihypertensive drug include angiotensin converting enzyme inhibitors (such as captopril, alacepril, lisinopril, imidapril, quinapril, temocapril, delapril, benazepril, cilazapril, trandolapril, enalapril, ceronapril, fosinopril, imadapril, mobertpril, perindopril, ramipril, spirapril, and randolapril), angiotensin II antagonists (such as losartan, candesartan, valsartan, eprosartan, and irbesartan), calcium channel blocking drugs (such as aranidipine, efonidipine, nicardipine, bamidipine, benidipine, manidipine, cilnidipine, nisoldipine, nitrendipine, nifedipine, nilvadipine, felodipine, amlodipine, diltiazem, bepridil, clentiazem, phendilin, galopamil, mibefradil, prenylamine, semotiadil, terodiline, verapamil, cilnidipine, elgodipine, isradipine, lacidipine, lercanidipine, nimodipine, cinnarizine, flunarizine, lidoflazine, lomerizine, bencyclane, etafenone, and perhexiline), β-adrenaline receptor blocking drugs (propranolol, pindolol, indenolol, carteolol, bunitrolol, atenolol, acebutolol, metoprolol, timolol, nipradilol, penbutolol, nadolol, tilisolol, carvedilol, bisoprolol, betaxolol, celiprolol, bopindolol, bevantolol, labetalol, alprenolol, amosulalol, arotinolol, befunolol, bucumolol, bufetolol, buferalol, buprandolol, butylidine, butofilolol, carazolol, cetamolol, cloranolol, dilevalol, epanolol, levobunolol, mepindolol, metipranolol, moprolol, nadoxolol, nevibolol, oxprenolol, practol, pronetalol, sotalol, sufinalol, talindolol, tertalol, toliprolol, xybenolol, and esmolol), α-receptor blocking drugs (such as amosulalol, prazosin, terazosin, doxazosin, bunazosin, urapidil, phentolamine, arotinolol, dapiprazole, fenspiride, indoramin, labetalol, naftopidil, nicergoline, tamsulosin, tolazoline, trimazosin, and yohimbine), sympathetic nerve inhibitors (such as clonidine, guanfacine, guanabenz, methyldopa, and reserpine), hydralazine, todralazine, budralazine, and cadralazine. Examples of the antianginal drug include nitrate drugs (such as amyl nitrite, nitroglycerin, and isosorbide), β-adrenaline receptor blocking drugs (such as propranolol, pindolol, indenolol, carteolol, bunitrolol, atenolol, acebutolol, metoprolol, timolol, nipradilol, penbutolol, nadolol, tilisolol, carvedilol, bisoprolol, betaxolol, celiprolol, bopindolol, bevantolol, labetalol, alprenolol, amosulalol, arotinolol, befunolol, bucumolol, bufetolol, buferalol, buprandolol, butylidine, butofilolol, carazolol, cetamolol, cloranolol, dilevalol, epanolol, levobunolol, mepindolol, metipranolol, moprolol, nadoxolol, nevibolol, oxprenolol, practol, pronetalol, sotalol, sufinalol, talindolol, tertalol, toliprolol, andxybenolol), calcium channel blocking drugs (such as aranidipine, efonidipine, nicardipine, bamidipine, benidipine, manidipine, cilnidipine, nisoldipine, nitrendipine, nifedipine, nilvadipine, felodipine, amlodipine, diltiazem, bepridil, clentiazem, phendiline, galopamil, mibefradil, prenylamine, semotiadil, terodiline, verapamil, cilnidipine, elgodipine, isradipine, lacidipine, lercanidipine, nimodipine, cinnarizine, flunarizine, lidoflazine, lomerizine, bencyclane, etafenone, and perhexiline) trimetazidine, dipyridamole, etafenone, dilazep, trapidil, nicorandil, enoxaparin, and aspirin. Examples of the diuretic include thiazide diuretics (such as hydrochlorothiazide, methyclothiazide, trichlormethiazide, benzylhydrochlorothiazide, and penflutizide), loop diuretics (such as furosemide, etacrynic acid, bumetanide, piretanide, azosemide, and torasemide), K⁺ sparing diuretics (spironolactone, triamterene, andpotassiumcanrenoate), osmotic diuretics (such as isosorbide, D-mannitol, and glycerin), nonthiazide diuretics (such as meticrane, tripamide, chlorthalidone, and mefruside), and acetazolamide. Examples of the cardiotonic include digitalis formulations (such as digitoxin, digoxin, methyldigoxin, deslanoside, vesnarinone, lanatoside C, and proscillaridin), xanthine formulations (such as aminophylline, choline theophylline, diprophylline, and proxyphylline), catecholamine formulations (such as dopamine, dobutamine, and docarpamine), PDE III inhibitors (such as amrinone, olprinone, and milrinone), denopamine, ubidecarenone, pimobendan, levosimendan, aminoethylsulfonic acid, vesnarinone, carperitide, and colforsin daropate. Examples of the antiarrhythmic drug include ajmaline, pirmenol, procainamide, cibenzoline, disopyramide, quinidine, aprindine, mexiletine, lidocaine, phenyloin, pilsicainide, propafenone, flecainide, atenolol, acebutolol, sotalol, propranolol, metoprolol, pindolol, amiodarone, nifekalant, diltiazem, bepridil, and verapamil. Examples of the antihyperlipidemic drug include atorvastatin, simvastatin, pravastatin sodium, fluvastatin sodium, clinofibrate, clofibrate, simfibrate, fenofibrate, bezafibrate, colestimide, and colestyramine. Examples of the immunosuppressant include azathioprine, mizoribine, cyclosporine, tacrolimus, gusperimus, and methotrexate.

### Cell Death/Cancer

Sirtuin-modulating compounds that increase the level and/or activity of a sirtuin protein may be administered to subjects who have recently received or are likely to receive a dose of radiation or toxin. In one embodiment, the dose of radiation or toxin is received as part of a work-related or medical procedure, e.g., working in a nuclear power plant, flying an airplane, an X-ray, CAT scan, or the administration of a radioactive dye for medical imaging; in such an embodiment, the compound is administered as a prophylactic measure. In another embodiment, the radiation or toxin exposure is received unintentionally, e.g., as a result of an industrial accident, habitation in a location of natural radiation, terrorist act, or act of war involving radioactive or toxic material. In such a case, the compound is preferably administered as soon as possible after the exposure to inhibit apoptosis and the subsequent development of acute radiation syndrome.

Sirtuin-modulating compounds may also be used for treating and/or preventing cancer. In certain embodiments, sirtuin-modulating compounds that increase the level and/or activity of a sirtuin protein may be used for treating and/or preventing cancer. Calorie restriction has been linked to a reduction in the incidence of age-related disorders including cancer (see e.g., Bordone and Guarente, Nat. Rev. Mol. Cell Biol. (2005 epub); Guarente and Picard, Cell 120: 473-82 (2005); Berrigan, et al., Carcinogenesis 23: 817-822 (2002); and Heilbronn and Ravussin, Am. J. Clin. Nutr. 78: 361-369 (2003)). Additionally, the Sir2 protein from yeast has been shown to be required for lifespan extension by glucose restriction (see e.g., Lin et al., Science 289: 2126-2128 (2000); Anderson et al., Nature 423: 181-185 (2003)), a yeast model for calorie restriction. Accordingly, an increase in the level and/or activity of a sirtuin protein may be useful for treating and/or preventing the incidence of age-related disorders, such as, for example, cancer. In other embodiments, sirtuin-modulating compounds that decrease the level and/or activity of a sirtuin protein may be used for treating or preventing cancer. For example, inhibitory compounds may be used to stimulate acetylation of substrates such as p53 and thereby increase apoptosis, as well as to reduce the lifespan of cells and organisms, render them more sensitive to stress, and/or increase the radiosensitivity and/or chemosensitivity of a cell or organism. Thus, inhibitory compounds may be used, e.g., for treating cancer. Exemplary cancers that may be treated using a sirtuin-modulating compound are those of the brain and kidney; hormone-dependent cancers including breast, prostate, testicular, and ovarian cancers; lymphomas, and leukemias. In cancers associated with solid tumors, a modulating compound may be administered directly into the tumor. Cancer of blood cells, e.g., leukemia, can be treated by administering a modulating compound into the blood stream or into the bone marrow. Benign cell growth can also be treated, e.g., warts. Other diseases that can be treated include autoimmune diseases, e.g., systemic lupus erythematosus, scleroderma, and arthritis, in which autoimmune cells should be removed. Viral infections such as herpes, HIV, adenovirus, and HTLV-1 associated malignant and benign disorders can also be treated by administration of sirtuin-modulating compound. Alternatively, cells can be obtained from a subject, treated *ex vivo* to remove certain undesirable cells, e.g., cancer cells, and administered back to the same or a different subject.

Chemotherapeutic agents that may be coadministered with modulating compounds described herein as having anti-cancer activity (e.g., compounds that induce apoptosis, compounds that reduce lifespan or compounds that render cells sensitive to stress) include: aminoglutethimide, amsacrine, anastrozole, asparaginase, bcg, bicalutamide, bleomycin, buserelin, busulfan, campothecin, capecitabine, carboplatin, carmustine, chlorambucil, cisplatin, cladribine, clodronate, colchicine, cyclophosphamide, cyproterone, cytarabine, dacarbazine, dactinomycin, daunorubicin, dienestrol, diethylstilbestrol, docetaxel, doxorubicin, epirubicin, estradiol, estramustine, etoposide, exemestane, filgrastim, fludarabine, fludrocortisone, fluorouracil, fluoxymesterone, flutamide, gemcitabine, genistein, goserelin, hydroxyurea, idarubicin, ifosfamide, imatinib, interferon, irinotecan, ironotecan, letrozole, leucovorin, leuprolide, levamisole, lomustine, mechlorethamine, medroxyprogesterone, megestrol, melphalan, mercaptopurine, mesna, methotrexate, mitomycin, mitotane, mitoxantrone, nilutamide, nocodazole, octreotide, oxaliplatin, paclitaxel, pamidronate, pentostatin, plicamycin, porfimer, procarbazine, raltitrexed, rituximab, streptozocin, suramin, tamoxifen, temozolomide, teniposide, testosterone, thioguanine, thiotepa, titanocene dichloride, topotecan, trastuzumab, tretinoin, vinblastine, vincristine, vindesine, and vinorelbine.

These chemotherapeutic agents may be categorized by their mechanism of action into, for example, following groups: anti-metabolites/anti-cancer agents, such as pyrimidine analogs (5-fluorouracil, floxuridine, capecitabine, gemcitabine and cytarabine) and purine analogs, folate antagonists and related inhibitors (mercaptopurine, thioguanine, pentostatin and 2-chlorodeoxyadenosine (cladribine)); antiproliferative/antimitotic agents including natural products such as vinca alkaloids (vinblastine, vincristine, and vinorelbine), microtubule disruptors such as taxane (paclitaxel, docetaxel), vincristin, vinblastin, nocodazole, epothilones and navelbine, epidipodophyllotoxins (teniposide), DNA damaging agents (actinomycin, amsacrine, anthracyclines, bleomycin, busulfan, camptothecin, carboplatin, chlorambucil, cisplatin, cyclophosphamide, cytoxan, dactinomycin, daunorubicin, docetaxel, doxorubicin, epirubicin, hexamethylmelamineoxaliplatin, iphosphamide, melphalan, merchlorethamine, mitomycin, mitoxantrone, nitrosourea, paclitaxel, plicamycin, procarbazine, teniposide, triethylenethiophosphoramide and etoposide (VP16)); antibiotics such as dactinomycin (actinomycin D), daunorubicin, doxorubicin (adriamycin), idarubicin, anthracyclines, mitoxantrone, bleomycins, plicamycin (mithramycin) and mitomycin; enzymes (L-asparaginase which systemically metabolizes L-asparagine and deprives cells which do not have the capacity to synthesize their own asparagine); antiplatelet agents; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nitrosoureas (carmustine (BCNU) and analogs, streptozocin), trazenes - dacarbazinine (DTIC); antiproliferative/antimitotic antimetabolites such as folic acid analogs (methotrexate); platinum coordination complexes (cisplatin, carboplatin), procarbazine, hydroxyurea, mitotane, aminoglutethimide; hormones, hormone analogs (estrogen, tamoxifen, goserelin, bicalutamide, nilutamide) and aromatase inhibitors (letrozole, anastrozole); anticoagulants (heparin, synthetic heparin salts and other inhibitors of thrombin); fibrinolytic agents (such as tissue plasminogen activator, streptokinase and urokinase), aspirin, COX-2 inhibitors, dipyridamole, ticlopidine, clopidogrel, abciximab; antimigratory agents; antisecretory agents (breveldin); immunosuppressives (cyclosporine, tacrolimus (FK-506), sirolimus (rapamycin), azathioprine, mycophenolate mofetil); anti-angiogenic compounds (TNP-470, genistein) and growth factor inhibitors (vascular endothelial growth factor (VEGF) inhibitors, fibroblast growth factor (FGF) inhibitors, epidermal growth factor (EGF) inhibitors); angiotensin receptor blocker; nitric oxide donors; anti-sense oligonucleotides; antibodies (trastuzumab); cell cycle inhibitors and differentiation inducers (tretinoin); mTOR inhibitors, topoisomerase inhibitors (doxorubicin (adriamycin), amsacrine, camptothecin, daunorubicin, dactinomycin, eniposide, epirubicin, etoposide, idarubicin, irinotecan (CPT-11) and mitoxantrone, topotecan, irinotecan), corticosteroids (cortisone, dexamethasone, hydrocortisone, methylpednisolone, prednisone, and prenisolone); growth factor signal transduction kinase inhibitors; mitochondrial dysfunction inducers and caspase activators; chromatin disruptors.

These chemotherapeutic agents may be used by themselves with a sirtuin-modulating compound described herein as inducing cell death or reducing lifespan or increasing sensitivity to stress and/or in combination with other chemotherapeutics agents. Many combinatorial therapies have been developed, including but not limited to those listed in Table 1.

**Table 1: Exemplary combinatorial therapies for the treatment of cancer.**

| **Name** | **Therapeutic agents** |
|---|---|
| ABV | Doxorubicin, Bleomycin, Vinblastine |
| ABVD | Doxorubicin, Bleomycin, Vinblastine, Dacarbazine |
| AC (Breast) | Doxorubicin, Cyclophosphamide |
| AC (Sarcoma) | Doxorubicin, Cisplatin |
| AC (Neuroblastoma) | Cyclophosphamide, Doxorubicin |
| ACE | Cyclophosphamide, Doxorubicin, Etoposide |
| ACe | Cyclophosphamide, Doxorubicin |
| AD | Doxorubicin, Dacarbazine |
| AP | Doxorubicin, Cisplatin |
| ARAC-DNR | Cytarabine, Daunorubicin |
| B-CAVe | Bleomycin, Lomustine, Doxorubicin, Vinblastine |
| BCVPP | Carmustine, Cyclophosphamide, Vinblastine, Procarbazine, Prednisone |
| BEACOPP | Bleomycin, Etoposide, Doxorubicin, Cyclophosphamide, Vincristine, Procarbazine, Prednisone, Filgrastim |
| BEP | Bleomycin, Etoposide, Cisplatin |
| BIP | Bleomycin, Cisplatin, Ifosfamide, Mesna |
| BOMP | Bleomycin, Vincristine, Cisplatin, Mitomycin |
| CA | Cytarabine, Asparaginase |
| CABO | Cisplatin, Methotrexate, Bleomycin, Vincristine |
| CAF | Cyclophosphamide, Doxorubicin, Fluorouracil |
| CAL-G | Cyclophosphamide, Daunorubicin, Vincristine, Prednisone, Asparaginase |
| CAMP | Cyclophosphamide, Doxorubicin, Methotrexate, Procarbazine |
| CAP | Cyclophosphamide, Doxorubicin, Cisplatin |
| CaT | Carboplatin, Paclitaxel |
| CAV | Cyclophosphamide, Doxorubicin, Vincristine |
| CAVE ADD | CAV and Etoposide |
| CA-VP16 | Cyclophosphamide, Doxorubicin, Etoposide |
| CC | Cyclophosphamide, Carboplatin |
| CDDP/VP-16 | Cisplatin, Etoposide |
| CEF | Cyclophosphamide, Epirubicin, Fluorouracil |
| CEPP(B) | Cyclophosphamide, Etoposide, Prednisone, with or without/ Bleomycin |
| CEV | Cyclophosphamide, Etoposide, Vincristine |
| CF | Cisplatin, Fluorouracil or Carboplatin Fluorouracil |
| CHAP | Cyclophosphamide or Cyclophosphamide, Altretamine, Doxorubicin, Cisplatin |
| ChlVPP | Chlorambucil, Vinblastine, Procarbazine, Prednisone |
| CHOP | Cyclophosphamide, Doxorubicin, Vincristine, Prednisone |
| CHOP-BLEO | Add Bleomycin to CHOP |
| CISCA | Cyclophosphamide, Doxorubicin, Cisplatin |
| CLD-BOMP | Bleomycin, Cisplatin, Vincristine, Mitomycin |
| CMF | Methotrexate, Fluorouracil, Cyclophosphamide |
| CMFP | Cyclophosphamide, Methotrexate, Fluorouracil, Prednisone |
| CMFVP | Cyclophosphamide, Methotrexate, Fluorouracil, Vincristine, Prednisone |
| CMV | Cisplatin, Methotrexate, Vinblastine |
| CNF | Cyclophosphamide, Mitoxantrone, Fluorouracil |
| CNOP | Cyclophosphamide, Mitoxantrone, Vincristine, Prednisone |
| COB | Cisplatin, Vincristine, Bleomycin |
| CODE | Cisplatin, Vincristine, Doxorubicin, Etoposide |
| COMLA | Cyclophosphamide, Vincristine, Methotrexate, Leucovorin, Cytarabine |
| COMP | Cyclophosphamide, Vincristine, Methotrexate, Prednisone |
| Cooper Regimen | Cyclophosphamide, Methotrexate, Fluorouracil, Vincristine, Prednisone |
| COP | Cyclophosphamide, Vincristine, Prednisone |
| COPE | Cyclophosphamide, Vincristine, Cisplatin, Etoposide |
| COPP | Cyclophosphamide, Vincristine, Procarbazine, Prednisone |
| CP(Chronic lymphocytic leukemia) | Chlorambucil, Prednisone |
| CP (Ovarian Cancer) | Cyclophosphamide, Cisplatin |
| CT | Cisplatin, Paclitaxel |
| CVD | Cisplatin, Vinblastine, Dacarbazine |
| CVI | Carboplatin, Etoposide, Ifosfamide, Mesna |
| CVP | Cyclophosphamide, Vincristine, Prednisome |
| CVPP | Lomustine, Procarbazine, Prednisone |
| CYVADIC | Cyclophosphamide, Vincristine, Doxorubicin, Dacarbazine |
| DA | Daunorubicin, Cytarabine |
| DAT | Daunorubicin, Cytarabine, Thioguanine |
| DAV | Daunorubicin, Cytarabine, Etoposide |
| DCT | Daunorubicin, Cytarabine, Thioguanine |
| DHAP | Cisplatin, Cytarabine, Dexamethasone |
| DI | Doxorubicin, Ifosfamide |
| DTIC/Tamoxifen | Dacarbazine, Tamoxifen |
| DVP | Daunorubicin, Vincristine, Prednisone |
| EAP | Etoposide, Doxorubicin, Cisplatin |
| EC | Etoposide, Carboplatin |
| EFP | Etoposie, Fluorouracil, Cisplatin |
| ELF | Etoposide, Leucovorin, Fluorouracil |
| EMA 86 | Mitoxantrone, Etoposide, Cytarabine |
| EP | Etoposide, Cisplatin |
| EVA | Etoposide, Vinblastine |
| FAC | Fluorouracil, Doxorubicin, Cyclophosphamide |
| FAM | Fluorouracil, Doxorubicin, Mitomycin |
| FAMTX | Methotrexate, Leucovorin, Doxorubicin |
| FAP | Fluorouracil, Doxorubicin, Cisplatin |
| F-CL | Fluorouracil, Leucovorin |
| FEC | Fluorouracil, Cyclophosphamide, Epirubicin |
| FED | Fluorouracil, Etoposide, Cisplatin |
| FL | Flutamide, Leuprolide |
| FZ | Flutamide, Goserelin acetate implant |
| HDMTX | Methotrexate, Leucovorin |
| Hexa-CAF | Altretamine, Cyclophosphamide, Methotrexate, Fluorouracil |
| ICE-T | Ifosfamide, Carboplatin, Etoposide, Paclitaxel, Mesna |
| IDMTX/6-MP | Methotrexate, Mercaptopurine, Leucovorin |
| IE | Ifosfamide, Etoposie, Mesna |
| IfoVP | Ifosfamide, Etoposide, Mesna |
| IPA | Ifosfamide, Cisplatin, Doxorubicin |
| M-2 | Vincristine, Carmustine, Cyclophosphamide, Prednisone, Melphalan |
| MAC-III | Methotrexate, Leucovorin, Dactinomycin, Cyclophosphamide |
| MACC | Methotrexate, Doxorubicin, Cyclophosphamide, Lomustine |
| MACOP-B | Methotrexate, Leucovorin, Doxorubicin, Cyclophosphamide, Vincristine, Bleomycin, Prednisone |
| MAID | Mesna, Doxorubicin, Ifosfamide, Dacarbazine |
| m-BACOD | Bleomycin, Doxorubicin, Cyclophosphamide, Vincristine, Dexamethasone, Methotrexate, Leucovorin |
| MBC | Methotrexate, Bleomycin, Cisplatin |
| MC | Mitoxantrone, Cytarabine |
| MF | Methotrexate, Fluorouracil, Leucovorin |
| MICE | Ifosfamide, Carboplatin, Etoposide, Mesna |
| MINE | Mesna, Ifosfamide, Mitoxantrone, Etoposide |
| mini-BEAM | Carmustine, Etoposide, Cytarabine, Melphalan |
| MOBP | Bleomycin, Vincristine, Cisplatin, Mitomycin |
| MOP | Mechlorethamine, Vincristine, Procarbazine |
| MOPP | Mechlorethamine, Vincristine, Procarbazine, Prednisone |
| MOPP/ABV | Mechlorethamine, Vincristine, Procarbazine, Prednisone, Doxorubicin, Bleomycin, Vinblastine |
| MP (multiple myeloma) | Melphalan, Prednisone |
| MP (prostate cancer) | Mitoxantrone, Prednisone |
| MTX/6-MO | Methotrexate, Mercaptopurine |
| MTX/6-MP/VP | Methotrexate, Mercaptopurine, Vincristine, Prednisone |
| MTX-CDDPAdr | Methotrexate, Leucovorin, Cisplatin, Doxorubicin |
| MV (breast cancer) | Mitomycin, Vinblastine |
| MV (acute myelocytic leukemia) | Mitoxantrone, Etoposide |
| M-VAC Methotrexate | Vinblastine, Doxorubicin, Cisplatin |
| MVP Mitomycin | Vinblastine, Cisplatin |
| MVPP | Mechlorethamine, Vinblastine, Procarbazine, Prednisone |
| NFL | Mitoxantrone, Fluorouracil, Leucovorin |
| NOVP | Mitoxantrone, Vinblastine, Vincristine |
| OPA | Vincristine, Prednisone, Doxorubicin |
| OPPA | Add Procarbazine to OPA. |
| PAC | Cisplatin, Doxorubicin |
| PAC-I | Cisplatin, Doxorubicin, Cyclophosphamide |
| PA-CI | Cisplatin, Doxorubicin |
| PC | Paclitaxel, Carboplatin or Paclitaxel, Cisplatin |
| PCV | Lomustine, Procarbazine, Vincristine |
| PE | Paclitaxel, Estramustine |
| PFL | Cisplatin, Fluorouracil, Leucovorin |
| POC | Prednisone, Vincristine, Lomustine |
| ProMACE | Prednisone, Methotrexate, Leucovorin, Doxorubicin, Cyclophosphamide, Etoposide |
| ProMACE/cytaBOM | Prednisone, Doxorubicin, Cyclophosphamide, Etoposide, Cytarabine, Bleomycin, Vincristine, Methotrexate, Leucovorin, Cotrimoxazole |
| PRoMACE/MOPP | Prednisone, Doxorubicin, Cyclophosphamide, Etoposide, Mechlorethamine, Vincristine, Procarbazine, Methotrexate, Leucovorin |
| Pt/VM | Cisplatin, Teniposide |
| PVA | Prednisone, Vincristine, Asparaginase |
| PVB | Cisplatin, Vinblastine, Bleomycin |
| PVDA | Prednisone, Vincristine, Daunorubicin, Asparaginase |
| SMF | Streptozocin, Mitomycin, Fluorouracil |
| TAD | Mechlorethamine, Doxorubicin, Vinblastine, Vincristine, Bleomycin, Etoposide, Prednisone |
| TCF | Paclitaxel, Cisplatin, Fluorouracil |
| TIP | Paclitaxel, Ifosfamide, Mesna, Cisplatin |
| TTT | Methotrexate, Cytarabine, Hydrocortisone |
| Topo/CTX | Cyclophosphamide, Topotecan, Mesna |
| VAB-6 | Cyclophosphamide, Dactinomycin, Vinblastine, Cisplatin, Bleomycin |
| VAC | Vincristine, Dactinomycin, Cyclophosphamide |
| VACAdr | Vincristine, Cyclophosphamide, Doxorubicin, Dactinomycin, Vincristine |
| VAD | Vincristine, Doxorubicin, Dexamethasone |
| VATH | Vinblastine, Doxorubicin, Thiotepa, Flouxymesterone |
| VBAP | Vincristine, Carmustine, Doxorubicin, Prednisone |
| VBCMP | Vincristine, Carmustine, Melphalan, Cyclophosphamide, Prednisone |
| VC | Vinorelbine, Cisplatin |
| VCAP | Vincristine, Cyclophosphamide, Doxorubicin, Prednisone |
| VD | Vinorelbine, Doxorubicin |
| VelP | Vinblastine, Cisplatin, Ifosfamide, Mesna |
| VIP | Etoposide, Cisplatin, Ifosfamide, Mesna |
| VM | Mitomycin, Vinblastine |
| VMCP | Vincristine, Melphalan, Cyclophosphamide, Prednisone |
| VP | Etoposide, Cisplatin |
| V-TAD | Etoposide, Thioguanine, Daunorubicin, Cytarabine |
| 5+2 | Cytarabine, Daunorubicin, Mitoxantrone |
| 7 + 3 | Cytarabine with/, Daunorubicin or Idarubicin or Mitoxantrone |
| "8 in 1" | Methylprednisolone, Vincristine, Lomustine, Procarbazine, Hydroxyurea, Cisplatin, Cytarabine, Dacarbazine |

In addition to conventional chemotherapeutics, the sirtuin-modulating compounds described herein as capable of inducing cell death or reducing lifespan can also be used with antisense RNA, RNAi or other polynucleotides to inhibit the expression of the cellular components that contribute to unwanted cellular proliferation that are targets of conventional chemotherapy. Such targets are, merely to illustrate, growth factors, growth factor receptors, cell cycle regulatory proteins, transcription factors, or signal transduction kinases.

Combination therapies comprising sirtuin-modulating compounds and a conventional chemotherapeutic agent may be advantageous over combination therapies known in the art because the combination allows the conventional chemotherapeutic agent to exert greater effect at lower dosage. In a preferred embodiment, the effective dose (ED₅₀) for a chemotherapeutic agent, or combination of conventional chemotherapeutic agents, when used in combination with a sirtuin-modulating compound is at least 2 fold less than the ED₅₀ for the chemotherapeutic agent alone, and even more preferably at 5 fold, 10 fold or even 25 fold less. Conversely, the therapeutic index (TI) for such chemotherapeutic agent or combination of such chemotherapeutic agent when used in combination with a sirtuin-modulating compound described herein can be at least 2 fold greater than the TI for conventional chemotherapeutic regimen alone, and even more preferably at 5 fold, 10 fold or even 25 fold greater.

### Neuronal Diseases/Disorders

In certain aspects, sirtuin-modulating compounds that increase the level and/or activity of a sirtuin protein can be used to treat patients suffering from neurodegenerative diseases, and traumatic or mechanical injury to the central nervous system (CNS) or peripheral nervous system (PNS). Neurodegenerative disease typically involves reductions in the mass and volume of the human brain, which may be due to the atrophy and/or death of brain cells, which are far more profound than those in a healthy person that are attributable to aging. Neurodegenerative diseases evolve gradually, after a long period of normal brain function, due to progressive degeneration (e.g., nerve cell dysfunction and death) of specific brain regions. The actual onset of brain degeneration may precede clinical expression by many years. Examples of neurodegenerative diseases include, but are not limited to, Alzheimer's disease (AD), Parkinson's disease (PD), Huntington disease (HD), amyotrophic lateral sclerosis (ALS; Lou Gehrig's disease), diffuse Lewy body disease, chorea-acanthocytosis, primary lateral sclerosis, ocular diseases (ocular neuritis), chemotherapy-induced neuropathies (e.g., from vincristine, paclitaxel, bortezomib), diabetes-induced neuropathies and Friedreich's ataxia. Sirtuin-modulating compounds that increase the level and/or activity of a sirtuin protein can be used to treat these disorders and others as described below.

AD is a chronic, incurable, and unstoppable CNS disorder that occurs gradually, resulting in memory loss, unusual behavior, personality changes, and a decline in thinking abilities. These losses are related to the death of specific types of brain cells and the breakdown of connections between them. AD has been described as childhood development in reverse. In most people with AD, symptoms appear after the age 60. The earliest symptoms include loss of recent memory, faulty judgment, and changes in personality. Later in the disease, those with AD may forget how to do simple tasks like washing their hands. Eventually people with AD lose all reasoning abilities and become dependent on other people for their everyday care. Finally, the disease becomes so debilitating that patients are bedridden and typically develop coexisting illnesses.

PD is a chronic, incurable, and unstoppable CNS disorder that occurs gradually and results in uncontrolled body movements, rigidity, tremor, and gait difficulties. These motor system problems are related to the death of brain cells in an area of the brain that produces dopamine, a chemical that helps control muscle activity. In most people with PD, symptoms appear after age 50. The initial symptoms of PD are a pronounced tremor affecting the extremities, notably in the hands or lips. Subsequent characteristic symptoms of PD are stiffness or slowness of movement, a shuffling walk, stooped posture, and impaired balance. There are wide ranging secondary symptoms such as memory loss, dementia, depression, emotional changes, swallowing difficulties, abnormal speech, sexual dysfunction, and bladder and bowel problems. These symptoms will begin to interfere with routine activities, such as holding a fork or reading a newspaper. Finally, people with PD become so profoundly disabled that they are bedridden.

ALS (motor neuron disease) is a chronic, incurable, and unstoppable CNS disorder that attacks the motor neurons, components of the CNS that connect the brain to the skeletal muscles. In ALS, the motor neurons deteriorate and eventually die, and though a person's brain normally remains fully functioning and alert, the command to move never reaches the muscles. Most people who get ALS are between 40 and 70 years old. The first motor neurons that weaken are those leading to the arms or legs. Those with ALS may have trouble walking, they may drop things, fall, slur their speech, and laugh or cry uncontrollably. Eventually the muscles in the limbs begin to atrophy from disuse. This muscle weakness will become debilitating and a person will need a wheel chair or become unable to function out of bed.

The causes of these neurological diseases have remained largely unknown. They are conventionally defined as distinct diseases, yet clearly show extraordinary similarities in basic processes and commonly demonstrate overlapping symptoms far greater than would be expected by chance alone. Current disease definitions fail to properly deal with the issue of overlap and a new classification of the neurodegenerative disorders has been called for.

HD is another neurodegenerative disease resulting from genetically programmed degeneration of neurons in certain areas of the brain. This degeneration causes uncontrolled movements, loss of intellectual faculties, and emotional disturbance. HD is a familial disease, passed from parent to child through a dominant mutation in the wild-type gene. Some early symptoms of HD are mood swings, depression, irritability or trouble driving, learning new things, remembering a fact, or making a decision. As the disease progresses, concentration on intellectual tasks becomes increasingly difficult and the patient may have difficulty feeding himself or herself and swallowing.

Tay-Sachs disease and Sandhoff disease are glycolipid storage diseases caused by the lack of lysosomal β-hexosaminidase (Gravel et al., in The Metabolic Basis of Inherited Disease, eds. Scriver et al., McGraw-Hill, New York, pp. 2839-2879, 1995). In both disorders, GM2 ganglioside and related glycolipidssubstrates for β-hexosaminidase accumulate in the nervous system and trigger acute neurodegeneration. In the most severe forms, the onset of symptoms begins in early infancy. A precipitous neurodegenerative course then ensues, with affected infants exhibiting motor dysfunction, seizure, visual loss, and deafness. Death usually occurs by 2-5 years of age. Neuronal loss through an apoptotic mechanism has been demonstrated (Huang et al., Hum. Mol. Genet. 6: 1879-1885, 1997).

It is well-known that apoptosis plays a role in AIDS pathogenesis in the immune system. However, HIV-1 also induces neurological disease. Shi et al. (J. Clin. Invest. 98: 1979-1990, 1996) examined apoptosis induced by HIV-1 infection of the CNS in an in vitro model and in brain tissue from AIDS patients, and found that HIV-1 infection of primary brain cultures induced apoptosis in neurons and astrocytes in vitro. Apoptosis of neurons and astrocytes was also detected in brain tissue from 10/11 AIDS patients, including 5/5 patients with HIV-1 dementia and 4/5 nondemented patients.

Neuronal loss is also a salient feature of prion diseases, such as Creutzfeldt-Jakob disease in human, BSE in cattle (mad cow disease), Scrapie Disease in sheep and goats, and feline spongiform encephalopathy (FSE) in cats. Sirtuin-modulating compounds that increase the level and/or activity of a sirtuin protein may be useful for treating or preventing neuronal loss due to these prior diseases.

In another embodiment, a sirtuin-modulating compound that increases the level and/or activity of a sirtuin protein may be used to treat or prevent any disease or disorder involving axonopathy. Distal axonopathy is a type of peripheral neuropathy that results from some metabolic or toxic derangement of peripheral nervous system (PNS) neurons. It is the most common response of nerves to metabolic or toxic disturbances, and as such may be caused by metabolic diseases such as diabetes, renal failure, deficiency syndromes such as malnutrition and alcoholism, or the effects of toxins or drugs. The most common cause of distal axonopathy is diabetes, and the most common distal axonopathy is diabetic neuropathy. The most distal portions of axons are usually the first to degenerate, and axonal atrophy advances slowly towards the nerve's cell body. If the noxious stimulus is removed, regeneration is possible, though prognosis decreases depending on the duration and severity of the stimulus. Those with distal axonopathies usually present with symmetrical stocking-glove sensori-motor disturbances. Deep tendon reflexes and autonomic nervous system (ANS) functions are also lost or diminished in affected areas.

Diabetic neuropathies are neuropathic disorders that are associated with diabetes mellitus. These conditions usually result from diabetic microvascular injury involving small blood vessels that supply nerves (vasa nervorum). Relatively common conditions which may be associated with diabetic neuropathy include third nerve palsy; mononeuropathy; mononeuropathy multiplex; diabetic amyotrophy; a painful polyneuropathy; autonomic neuropathy; and thoracoabdominal neuropathy. Clinical manifestations of diabetic neuropathy include, for example, sensorimotor polyneuropathy such as numbness, sensory loss, dysesthesia and nighttime pain; autonomic neuropathy such as delayed gastric emptying or gastroparesis; and cranial neuropathy such as oculomotor (3rd) neuropathies or Mononeuropathies of the thoracic or lumbar spinal nerves.

Peripheral neuropathy is the medical term for damage to nerves of the peripheral nervous system, which may be caused either by diseases of the nerve or from the side-effects of systemic illness. Peripheral neuropathies vary in their presentation and origin, and may affect the nerve or the neuromuscular junction. Major causes of peripheral neuropathy include seizures, nutritional deficiencies, and HIV, though diabetes is the most likely cause. Mechanical pressure from staying in one position for too long, a tumor, intraneural hemorrhage, exposing the body to extreme conditions such as radiation, cold temperatures, or toxic substances can also cause peripheral neuropathy.

In an exemplary embodiment, a sirtuin-modulating compound that increases the level and/or activity of a sirtuin protein may be used to treat or prevent multiple sclerosis (MS), including relapsing MS and monosymptomatic MS, and other demyelinating conditions, such as, for example, chromic inflammatory demyelinating polyneuropathy (CIDP), or symptoms associated therewith.

MS is a chronic, often disabling disease of the central nervous system. Various and converging lines of evidence point to the possibility that the disease is caused by a disturbance in the immune function, although the cause of this disturbance has not been established. This disturbance permits cells of the immune system to "attack" myelin, the fat containing insulating sheath that surrounds the nerve axons located in the central nervous system ("CNS"). When myelin is damaged, electrical pulses cannot travel quickly or normally along nerve fiber pathways in the brain and spinal cord. This results in disruption of normal electrical conductivity within the axons, fatigue and disturbances of vision, strength, coordination, balance, sensation, and bladder and bowel function.

As such, MS is now a common and well-known neurological disorder that is characterized by episodic patches of inflammation and demyelination which can occur anywhere in the CNS. However, almost always without any involvement of the peripheral nerves associated therewith. Demyelination produces a situation analogous to that resulting from cracks or tears in an insulator surrounding an electrical cord. That is, when the insulating sheath is disrupted, the circuit is "short circuited" and the electrical apparatus associated therewith will function intermittently or nor at all. Such loss of myelin surrounding nerve fibers results in short circuits in nerves traversing the brain and the spinal cord that thereby result in symptoms of MS. It is further found that such demyelination occurs in patches, as opposed to along the entire CNS. In addition, such demyelination may be intermittent. Therefore, such occurrences are disseminated in both time and space.

It is believed that the pathogenesis involves a local disruption of the blood brain barrier which causes a localized immune and inflammatory response, with consequent damage to myelin and hence to neurons.

Clinically, MS exists in both sexes and can occur at any age. However, its most common presentation is in the relatively young adult, often with a single focal lesion such as a damage of the optic nerve, an area of anesthesia (loss of sensation), or paraesthesia (localize loss of feeling), or muscular weakness. In addition, vertigo, double vision, localized pain, incontinence, and pain in the arms and legs may occur upon flexation of the neck, as well as a large variety of less common symptoms.

An initial attack of MS is often transient, and it may be weeks, months, or years before a further attack occurs. Some individuals may enjoy a stable, relatively event free condition for a great number of years, while other less fortunate ones may experience a continual downhill course ending in complete paralysis. There is, most commonly, a series of remission and relapses, in which each relapse leaves a patient somewhat worse than before. Relapses may be triggered by stressful events, viral infections or toxins. Therein, elevated body temperature, i.e., a fever, will make the condition worse, or as a reduction of temperature by, for example, a cold bath, may make the condition better.

In yet another embodiment, a sirtuin-modulating compound that increases the level and/or activity of a sirtuin protein may be used to treat trauma to the nerves, including, trauma due to disease, injury (including surgical intervention), or environmental trauma (e.g., neurotoxins, alcoholism, etc.).

Sirtuin-modulating compounds that increase the level and/or activity of a sirtuin protein may also be useful to prevent, treat, and alleviate symptoms of various PNS disorders, such as the ones described below. The PNS is composed of the nerves that lead to or branch off from the CNS. The peripheral nerves handle a diverse array of functions in the body, including sensory, motor, and autonomic functions. When an individual has a peripheral neuropathy, nerves of the PNS have been damaged. Nerve damage can arise from a number of causes, such as disease, physical injury, poisoning, or malnutrition. These agents may affect either afferent or efferent nerves. Depending on the cause of damage, the nerve cell axon, its protective myelin sheath, or both may be injured or destroyed.

The term "peripheral neuropathy" encompasses a wide range of disorders in which the nerves outside of the brain and spinal cord-peripheral nerves-have been damaged. Peripheral neuropathy may also be referred to as peripheral neuritis, or if many nerves are involved, the terms polyneuropathy or polyneuritis may be used.

Peripheral neuropathy is a widespread disorder, and there are many underlying causes. Some of these causes are common, such as diabetes, and others are extremely rare, such as acrylamide poisoning and certain inherited disorders. The most common worldwide cause of peripheral neuropathy is leprosy. Leprosy is caused by the bacterium Mycobacterium leprae, which attacks the peripheral nerves of affected people.

Leprosy is extremely rare in the United States, where diabetes is the most commonly known cause of peripheral neuropathy. It has been estimated that more than 17 million people in the United States and Europe have diabetes-related polyneuropathy. Many neuropathies are idiopathic; no known cause can be found. The most common of the inherited peripheral neuropathies in the United States is Charcot-Marie-Tooth disease, which affects approximately 125,000 persons.

Another of the better known peripheral neuropathies is Guillain-Barré syndrome, which arises from complications associated with viral illnesses, such as cytomegalovirus, Epstein-Barr virus, and human immunodeficiency virus (HIV), or bacterial infection, including Campylobacter jejuni and Lyme disease. The worldwide incidence rate is approximately 1.7 cases per 100,000 people annually. Other well-known causes of peripheral neuropathies include chronic alcoholism, infection of the varicella-zoster virus, botulism, and poliomyelitis. Peripheral neuropathy may develop as a primary symptom, or it may be due to another disease. For example, peripheral neuropathy is only one symptom of diseases such as amyloid neuropathy, certain cancers, or inherited neurologic disorders. Such diseases may affect the PNS and the CNS, as well as other body tissues.

Other PNS diseases treatable with sirtuin-modulating compounds that increase the level and/or activity of a sirtuin protein include: Brachial Plexus Neuropathies (diseases of the cervical and first thoracic roots, nerve trunks, cords, and peripheral nerve components of the brachial plexus. Clinical manifestations include regional pain, paresthesia; muscle weakness, and decreased sensation in the upper extremity. These disorders may be associated with trauma, including birth injuries; thoracic outlet syndrome; neoplasms, neuritis, radiotherapy; and other conditions. See Adams et al., Principles of Neurology, 6th ed, pp1351-2); Diabetic Neuropathies (peripheral, autonomic, and cranial nerve disorders that are associated with diabetes mellitus). These conditions usually result from diabetic microvascular injury involving small blood vessels that supply nerves (vasa nervorum). Relatively common conditions which may be associated with diabetic neuropathy include third nerve palsy; mononeuropathy; mononeuropathy multiplex; diabetic amyotrophy; a painful polyneuropathy; autonomic neuropathy; and thoracoabdominal neuropathy (see Adams et al., Principles of Neurology, 6th ed, p1325); mononeuropathies (disease or trauma involving a single peripheral nerve in isolation, or out of proportion to evidence of diffuse peripheral nerve dysfunction). Mononeuropathy multiplex refers to a condition characterized by multiple isolated nerve injuries. Mononeuropathies may result from a wide variety of causes, including ischemia; traumatic injury; compression; connective tissue diseases; cumulative trauma disorders; and other conditions; Neuralgia (intense or aching pain that occurs along the course or distribution of a peripheral or cranial nerve); Peripheral Nervous System Neoplasms (neoplasms which arise from peripheral nerve tissue). This includes neurofibromas; Schwannomas; granular cell tumors; and malignant peripheral nerve sheath tumors. See DeVita Jr et al., Cancer: Principles and Practice of Oncology, 5th ed, pp1750-1); and Nerve Compression Syndromes (mechanical compression of nerves or nerve roots from internal or external causes). These may result in a conduction block to nerve impulses, due to, for example, myelin sheath dysfunction, or axonal loss. The nerve and nerve sheath injuries may be caused by ischemia; inflammation; or a direct mechanical effect; Neuritis (a general term indicating inflammation of a peripheral or cranial nerve). Clinical manifestation may include pain; paresthesias; paresis; or hyperthesia; Polyneuropathies (diseases of multiple peripheral nerves). The various forms are categorized by the type of nerve affected (e.g., sensory, motor, or autonomic), by the distribution of nerve injury (e.g., distal vs. proximal), by nerve component primarily affected (e.g., demyelinating vs. axonal), by etiology, or by pattern of inheritance.

In one embodiment, a combination drug regimen may include drugs or compounds for the treatment or prevention of neurodegenerative disorders or secondary conditions associated with these conditions. Thus, a combination drug regimen may include one or more sirtuin-modulating compounds that increase the level and/or activity of a sirtuin protein and one or more anti-neurodegeneration agents. For example, one or more sirtuin-modulating compounds can be combined with an effective amount of one or more of: L-DOPA; a dopamine agonist; an adenosine A_{2A} receptor antagonists; a COMT inhibitor; a MAO inhibitor; an NOS inhibitor; a sodium channel antagonist; a selective N-methyl D-aspartate (NMDA) receptor antagonists; an AMPA/kainate receptor antagonist; a calcium channel antagonist; a GABA-A receptor agonist; an acetyl-choline esterase inhibitor; a matrix metalloprotease inhibitor; an inhibitor of p38 MAP kinase or c-jun-N-terminal kinases; TPA; NDA antagonists; beta-interferons; growth factors; glutamate inhibitors; and/or as part of a cell therapy.

Exemplary N-NOS inhibitors include 4-(6-amino-pyridin-2-yl)-3-methoxyphenol 6-[4-(2-dimethylamino-ethoxy)-2-methoxy-phenyl]-pyridin-2-yl-amine, 6-[4-(2-dimethylamino-ethoxy)-2,3-dimet-hyl-phenyl]-pyridin-2-yl-amine, 6-[4-(2-pyrrolidinyl-ethoxy)-2,3-dimethyl-p-henyl]-pyridin-2-yl-amine, 6-[4-(4-(n-methyl)piperidinyloxy)-2,3-dimethyl-p-henyl]-pyridin-2-yl-amine, 6-[4-(2-dimethylamino-ethoxy)-3-methoxy-phenyl]-pyridin-2-yl-amine, 6-[4-(2-pyrrolidinyl-ethoxy)-3-methoxy-phenyl]-pyridin-2-yl-amine, 6-{4-[2-(6,7-dimethoxy-3,4-dihydro-1h-isoquinolin-2-yl)-ethoxy]-3-methoxy-phenyl}-pyridin-2-yl-amine, 6-{3-methoxy-4-[2-(4-phenethyl-piper-azin-1-yl)-ethoxy]-phenyl}-pyridin-2-yl-amine, 6-{3-methoxy-4-[2-(4-methyl-piperazin-1-yl)-ethoxy]-phenyl}-pyridin-2-yl-amine, 6-{4-[2-(4-dimethylamin-o-piperidin-1-yl)-ethoxy]-3-methoxy-phenyl}-pyridin-2-yl-amine, 6-[4-(2-dimethylamino-ethoxy)-3-ethoxy-phenyl]-pyridin-2-yl-amine, 6-[4-(2-pyrrolidinyl-ethoxy)-3-ethoxy-phenyl]-pyridin-2-yl-amine, 6-[4-(2-dimethylamino-ethoxy)-2-isopropyl-phenyl]-pyridin-2-yl-amine, 4-(6-amino-pyridin-yl)-3-cyclopropyl-phenol 6-[2-cyclopropyl-4-(2-dimethy-lamino-ethoxy)-phenyl]-pyridin-2-yl-amine, 6-[2-cyclopropyl-4-(2-pyrrolidin-1-yl-ethoxy)-phenyl]-pyridin-2-yl-amine, 3-[3-(6-amino-pyridin-2yl)-4-cycl-opropyl-phenoxy]-pyrrolidine-1-carboxylic acid tert-butyl ester 6-[2-cyclopropyl-4-(1-methyl-pyrrolidin-3-yl-oxy)-phenyl]-pyridin-2-yl-amine, 4-(6-amino-pyridin-2-yl)-3-cyclobutyl-phenol 6-[2-cyclobutyl-4-(2-dime-thylamino-ethoxy)-phenyl]-pyridin-2-yl-amine, 6-[2-cyclobutyl-4-(2-pyrrolid-in-1-yl-ethoxy)-phenyl]-pyridin-2-yl-amine, 6-[2-cyclobutyl-4-(1-methyl-pyr-rolidin-3-yl-oxy)-phenyl]-pyridin-2-yl-amine, 4-(6-amino-pyridin-2-yl)-3-cyclopentyl-phenol 6-[2-cyclopentyl-4-(2-dimethylamino-ethoxy)-phenyl]-pyrid-in-2-yl-amine, 6-[2-cyclopentyl-4-(2-pyrrolidin-lyl-ethoxy)-phenyl]-pyridin-2-yl-amine, 3-[4-(6-amino-pyridin-2yl)-3-mcthoxy-phcnoxy]-pyrrolidinc-1-ca-rboxylic acid tert butyl ester 6-[4-(1-methyl-pyrrolidin-3-yl-oxy)-2-metho-xy-phenyl]-pyridin-2-yl-amine, 4-[4-(6-amino-pyridin-2yl)-3-methoxy-phenoxy-]-piperidine-1-carboxylic acid tert butyl ester 6-[2-methoxy-4-(1-methyl-p-iperidin-4-yl-oxy)-phenyl]-pyridin-2-yl-amine, 6-[4-(allyloxy)-2-methoxy-ph-enyl]-pyridin-2-yl-amine, 4-(6-amino-pyridin-2-yl)-3-methoxy-6-allyl-phenol 12 and 4-(6-amino-pyridin-2-yl)-3-methoxy-2-allylphenol 13 4-(6-amino-pyridin-2-yl)-3-methoxy-6-propyl-phenol 6-[4-(2-dimethylamino-ethoxy)-2-methoxy-5-propyl-phenyl]-pyridin-yl-amine, 6-[2-isopropyl-4-(pyrrolidin-3-yl-oxy)-phenyl]-pyridin-2-yl-amine, 6-[2-isopropyl-4-(piperidin-3-yl-oxy)-phenyl]-pyridin-2-yl-amine, 6-[2-isopropyl-4-(1-methyl-azetidin-3-yl-oxy)-phenyl]-pyridin-2-yl-amine, 6-[2-isopropyl-4-(1-methyl-piperidin-4-yl-oxy)-phenyl]-pyridin-2-yl-amine, 6-[2-isopropyl-4-(1-methyl-pyrrolidin-3-yl-oxy)-phenyl]-pyridin-2-yl-amin-e 6-[2-isopropyl-4-(1-methyl-pyrrolidin-3-yl-oxy)-phenyl]-pyridin-2-yl-amine, 6-[2-isopropyl-4-(2-methyl-2-aza-bicyclo[2.2.1]hept-5-yl-oxy)-phenyl]-p-yridin-2-yl-amine, 6-[4-(2-dimethylamino-ethoxy)-2-methoxy-phenyl]-pyridin-2-yl-amine, 6- {4-[2-(benzyl-methyl-amino)-ethoxy]-2-methoxy-phenyl}-pyridin-2-yl-amine, 6-[2-methoxy-4-(2-pyrrolidin-1-yl-ethoxy)-phenyl]-pyridin-2-yl-amine, 2-(6-amino-pyridin-2-yl)-5-(2-dimethylamino-ethoxy)-phenol 2-[4-(6-amino-pyridin-2-yl)-3-methoxy-phenoxy]-acetamide 6-[4-(2-amino-ethoxy)-2-methoxy-phenyl]-pyridin-2-yl-amine, 6-{4-[2-(3,4-dihydro-1h-isoquinolin-2-yl)-ethoxy]-2-methoxy-phenyl}-pyrid-in-2-yl-amine, 2-[4-(6-amino-pyridin-2-yl)-3-methoxy-phenoxy]-ethanol 6- {2-methoxy-4-[2-(2,2,6,6-tetramethyl-piperidin-1-yl)-ethoxy]-phenyl}-py-ridin-2-yl-amine, 6- {4-[2-(2,5-dimethyl-pyrrolidin-1-yl)-ethoxy]-2-methoxy-phenyl}-pyridin-2-yl-amine, 6- {4-[2-(2,5-dimethyl-pyrrolidin-1-yl)-ethoxy]-2-methoxy-phenyl}-pyridin-2-yl-amine, 2-[4-(6-amino-pyridin-2-yl)-3-methoxy-phenoxy]-1-(2,2,6,6-tetramethyl-piperidin-1-yl)-ethanone 6-[2-methoxy-4-(1-methylpyrrolidin-2-yl-methoxy)-phenyl]-pyridin-2-yl-amine, 6-[4-(2-dimethylamino-ethoxy)-2-propoxy-phenyl]-pyridin-2-yl-amine, 6-{4-[2-(benzyl-methyl-amino)-ethoxy]-2-propoxy-phenyl}-pyridin-2-yl-amin-e 6-[4-(2-ethoxy-ethoxy)-2-methoxy-phenyl]-pyridin-2-yl-amine, 6-[4-(2-dimethylamino-ethoxy)-2-isopropoxy-phenyl]-pyridin-2-yl-amine, 6-[4-(2-ethoxy-ethoxy)-2-isopropoxy-phenyl]-pyridin-2-yl-amine, 6-[2-methoxy-4-(3-methyl-butoxy)-phenyl]-pyridin-2-yl-amine, 6-[4-(2-dimethylamino-ethoxy)-2-ethoxy-phenyl]-pyridin-2-yl-amine, 6-{4-[2-(benzyl-methyl-amino)-ethoxy]-2-ethoxy-phenyl}-pyridin-2-yl-amine, 6-[2-ethoxy-4-(3-methyl-butoxy)-phenyl]-pyridin-2-yl-amine, 1-(6-amino-3-aza-bicyclo[3.1.0]hex-3-yl)-2-[4-(6-amino-pyridin-2-yl)-3-et-hoxy-phenoxy]-ethanone 6-[2-ethoxy-4-(2-pyrrolidin-1-yl-ethoxy)-phenyl]-py-ridin-2-yl-amine, 3- {2-[4-(6-amino-pyridin-2-yl)-3-ethoxy-phenoxy]-ethyl} -3-aza-bicyclo[3.1.0]hex-6-yl-amine, 1-(6-amino-3-aza-bicyclo[3.1.0]hex-3-yl)-2-[4-(6-amino-pyridin-2-yl)-3-methoxy-phenoxy]-ethanone 3-{2-[4-(6-amino-pyridin-2-yl)-3-methoxy-phenoxy]-ethyl}-3-aza-bicyclo[3.-1.0]hex-6-yl-amine, 6-[2-isopropoxy-4-(2-pyrrolidin-1-yl-ethoxy)-phenyl]-py-ridin-2-yl-amine, 6-{4-[2-(benzyl-methyl-amino)-ethoxy]-2-isopropoxy-phenyl-}-pyridin-2-yl-amine, 6-[4-(2-dimethylamino-ethoxy)-2-methoxy-5-propyl-phen-yl]-pyridin-2-yl-amine, 6-[5-allyl-4-(2-dimethylamino-ethoxy)-2-methoxy-phe-nyl]-pyridin-2-yl-amine, 6-[5-allyl-2-methoxy-4-(2-pyrrolidin-1-yl-ethoxy)-phenyl]-pyridin-2-yl-amine, 6-[3-allyl-4-(2-dimethylamino-ethoxy)-2-methoxy-phenyl]-pyridin-2-yl-amine, 6-[2-methoxy-4-(pyrrolidin-3-yl-oxy)-phenyl]-p-yridin-2-yl-amine, 6-[2-methoxy-4-(1-methyl-pyrrolidin-3-yl-oxy)-phenyl]-py-ridin-2-yl-amine, 6-[2-ethoxy-4-(pyrrolidin-3-yl-oxy)-phenyl]-pyridin-2-yl-amine, 6-[2-isopropoxy-4-(pyrrolidin-3-yl-oxy)-phenyl]-pyridin-2-yl-amine, 6-[2-methoxy-4-(piperidin-4-yl-oxy)-phenyl]-pyridin-2-yl-amine, 6-[2-methoxy-4-(2,2,6,6-tetramethyl-piperidin-4-yl-oxy)-phenyl]-pyridin-2-yl-amine, 6-[2-isopropoxy-4-(pyrrolidin-3-yl-oxy)-phenyl]-pyridin-2-yl-amine, 3-[4-(6-amino-pyridin-2-yl)-3-methoxy-phenoxy]-azetidine-1-carboxylic acid tert-butyl ester 6-[4-(azetidin-3-yl-oxy)-2-methoxy-phenyl]-pyridin-2-yl-amine, 6-[2-methoxy-4-(1-methyl-azetidin-3-yl-oxy)-phenyl]-pyridin-2-y-1-amine, 6-[2-isopropoxy-4-(pyrrolidin-3-yl-oxy)-phenyl]-pyridin-2-yl-amine, 6-[2-isopropoxy-4-(pyrrolidin-3-yl-oxy)-phenyl]-pyridin-2-yl-amine, 6-[2-methoxy-4-(pyrrolidin-3-yl-oxy)-phenyl]-pyridin-2-yl-amine, 6-[2-methoxy-4-(1-methyl-pyrrolidin-3-yl-oxy)-phenyl]-pyridin-2-yl-amine, 6-[2-methoxy-4-(1-methyl-pyrrolidin-3-yl-oxy)-phenyl]-pyridin-2-yl-amine, 6-[2-methoxy-4-(2-methyl-2-aza-bicyclo[2.2.1]hept-5-yl-oxy)-phenyl]-pyrid-in-2-yl-amine, 6-[2-methoxy-4-(1-methyl-piperidin-4-yl-oxy)-phenyl]-pyridin-2-yl-amine 6-[4-(1-ethyl-piperidin-4-yl-oxy)-2-methoxy-phenyl]-pyridin-2-yl-amine, 6-[5-allyl-2-methoxy-4-(1-methyl-pyrrolidin-3-yl-oxy)-phenyl]-pyr-idin-2-yl-amine, 6-[4-(2-dimethylamino-ethoxy)-2,6-dimethyl-phenyl]-pyridin-2-yl-amine, 6-[2,6-dimethyl-4-(3-piperidin-1-yl-propoxy)-phenyl]-pyridin-2-yl-amine, 6-[2,6-dimethyl-4-(2-pyrrolidin-1-yl-ethoxy)-phenyl]-pyridin-2-y-1-amine, 6- {2,6-dimethyl-4-[3-(4-methyl-piperazin-1-yl)-propoxy]-phenyl}-py-ridin-2-yl-amine, 6-[2,6-dimethyl-4-(2-morpholin-4-yl-ethoxy)-phenyl]-pyrid-in-2-yl-amine, 6-{4-[2-(benzyl-methyl-amino)-ethoxy]-2,6-dimethyl-phenyl}-p-yridin-2-yl-amine, 2-[4-(6-amino-pyridin-2-yl)-3,5-dimethyl-phenoxy]-acetam-ide 6-[4-(2-amino-ethoxy)-2,6-dimethyl-phenyl]-pyridin-2-yl-amine, 6-[2-isopropyl-4-(2-pyrrolidin-1-yl-ethoxy)-phenyl]-pyridin-2-yl-amine, 2-(2,5-dimethyl-pyrrolidin-1-yl)-6-[2-isopropyl-4-(2-pyrrolidin-1-yl-etho-xy)-phenyl]-pyridine 6-{4-[2-(3,5-dimethyl-piperidin-1-yl)-ethoxy]-2-isopr-opyl-phenyl}-pyridin-2-yl-amine, 6-[4-(2-dimethylamino-ethoxy)-2-isopropyl-phenyl]-pyridin-2-yl-amine, 6-[2-tert-butyl-4-(2-dimethylamino-ethoxy)-phen-yl]-pyridin-2-yl-amine, 6-[2-tert-butyl-4-(2-pyrrolidin-1-yl-ethoxy)-phenyl-]-pyridin-2-yl-amine, 6-[4-(2-pyrrolidinyl-ethoxy)-2,5-dimethyl-phenyl]-pyr-idin-2-yl-amine, 6-[4-(2-dimethylamino-ethoxy)-2,5-dimethyl-phenyl]-pyridin-2-yl-amine, 6-[4-(2-(4-phenethylpiperazin-1-yl)-ethoxy)-2,5-dimethyl-pheny-l]-pyridin-2-yl-amine, 6-[2-cyclopropyl-4-(2-dimethylamino-1-methyl-ethoxy)-phenyl]-pyridin-2-yl-amine, 6-[cyclo butyl-4-(2-dimethylamino-1-methyl-etho-xy)-phenyl]-pyridin-2-yl-amine, 6-[4-(allyloxy)-2-cyclobutyl-phenyl]-pyridi-n-2ylamine, 2-allyl-4-(6-amino-pyridin-2-yl)-3-cyclobutyl-phenol and 2-allyl-4-(6-amino-pyridin-2-yl)-5-cyclobutyl-phenol 4-(6-amino-pyridin-2yl)-5-cyclobutyl-2-propyl-phenol 4-(6-amino-pyridin-2yl)-3-cyclobutyl-2-propyl-phenol 6-[2-cyclobutyl-4-(2-dimethylamino-1-methyl-ethoxy)-5-propyl-phenyl]-pyri-din-2-yl-amine, 6-[2-cyclobutyl-4-(2-dimethylamino-1-methyl-ethoxy)-3-propy-1-phenyl]-pyridin-2-yl-amine, 6-[2-cyclobutyl-4-(2-dimethylamino-ethoxy)-5-propyl-phenyl]-pyridin-2-yl-amine, 6-[2-cyclobutyl-4-(2-dimethylamino-ethox-y)-3-propyl-phenyl]-pyridin-2-yl-amine, 6-[2-cyclobutyl-4-(1-methyl-pyrroli-din-3-yl-oxy)-5-propyl-phenyl]-pyridin-2-yl-amine, 6-[cyclobutyl-4-(1-methy-l-pyrrolidin-3-yl-oxy)-3-propyl-phenyl]-pyridin-2-yl-amine, 2-(4-benzyloxy-5-hydroxy-2-methoxy-phenyl)-6-(2,5-dimethyl-pyrrol-1-yl)-p-yridine 6-[4-(2-dimethylamino-ethoxy)-5-ethoxy-2-methoxy-phenyl]-pyridin-2-yl-amine, 6-[5-ethyl-2-methoxy-4-(1-methyl-piperidin-4-yl-oxy)-phenyl]-pyr-idin-2-yl-amine, 6-[5-ethyl-2-methoxy-4-(piperidin-4-yl-oxy)-phenyl]-pyridi-n-2-yl-amine, 6-[2,5-dimethoxy-4-(1-methyl-pyrrolidin-3-yl-oxy)-phenyl]-pyr-idin-2-yl-amine, 6-[4-(2-dimethylamino-ethoxy)-5-ethyl-2-methoxy-phenyl]-py-ridin-2-yl-amine.

Exemplary NMDA receptor antagonist include (+)-(1S, 2S)-1-(4-hydroxy-phenyl)-2-(4-hydroxy-4-phenylpiperidino)-1-pro-panol, (1S, 2S)-1-(4-hydroxy-3-methoxyphenyl)-2-(4-hydroxy-4-phenylpiperi-dino)-1-propanol, (3R, 4S)-3-(4-(4-fluorophenyl)-4-hydroxypiperidin-1-yl-)-chroman-4,7-diol, (1R*, 2R*)-1-(4-hydroxy-3-methylphenyl)-2-(4-(4-fluoro-phenyl)-4-hydroxypiperidin-1-yl)-propan-1-ol-mesylate or a pharmaceutically acceptable acid addition salt thereof.

Exemplary dopamine agonists include ropininole; L-dopa decarboxylase inhibitors such as carbidopa or benserazide, bromocriptine, dihydroergocryptine, etisulergine, AF-14, alaptide, pergolide, piribedil; dopamine D1 receptor agonists such as A-68939, A-77636, dihydrexine, and SKF-38393; dopamine D2 receptor agonists such as carbergoline, lisuride, N-0434, naxagolide, PD-118440, pramipexole, quinpirole and ropinirole; dopamine/β-adrenegeric receptor agonists such as DPDMS and dopexamine; dopamine/5-HT uptake inhibitor/5-HT-1A agonists such as roxindole; dopamine/opiate receptor agonists such as NIH-10494; α2-adrenergic antagonist/dopamine agonists such as terguride; α2-adrenergic antagonist/dopamine D2 agonists such as ergolines and talipexole; dopamine uptake inhibitors such as GBR-12909, GBR-13069, GYKI-52895, and NS-2141; monoamine oxidase-B inhibitors such as selegiline, N-(2-butyl)-N-methylpropargylamine, N-methyl-N-(2-pentyl)propargylamine, AGN-1133, ergot derivatives, lazabemide, LU-53439, MD-280040 and mofegiline; and COMT inhibitors such as CGP-28014.

Exemplary acetyl cholinesterase inhibitors include donepizil, 1-(2-methyl-1H-benzimida-zol-5-yl)-3-[1-(phenylmethyl)-4-piperidinyl]-1-propanone; 1-(2-phenyl-1H-benzimidazol-5-yl)-3-[1-(phenylmethyl)-4-piperidinyl]-1-pr-opanone; 1-(1-ethyl-2-methyl-1H-benzimidazol-5-yl)-3-[1-(phenylmethyl)-4-p-iperidinyl]-1-propanone; 1-(2-methyl-6-benzothiazolyl)-3-[1-(phenylmethyl)-4-piperidinyl]-1-propanone; 1-(2-methyl-6-benzothiazolyl)-3-[1-[(2-methyl-4-thiazolyl)methyl]-4-piperidinyl]-1-propanone; 1-(5-methyl-benzo[b]thie-n-2-yl)-3-[1-(phenylmethyl)4-piperidinyl]-1-propanone; 1-(6-methyl-benzo[b]thien-2-yl)-3-[1-(phenylmethyl)-4-piperidinyl]-1-prop-anone; 1-(3,5-dimethyl-benzo[b]thien-2-yl)-3-[1-(phenylmethyl)-4-piperidin-yl]-1-propanone; 1-(benzo[b]thien-2-yl)-3-[1-(phenylmethyl)-4-piperidinyl]-1-propanone; 1-(benzofuran-2-yl)-3-[1-(phenylmethyl)-4-piperidinyl]-1-pro-panone; 1-(1-phenylsulfonyl-6-methyl-indol-2-yl)-3-[1-(phenylmethyl)-4-pip-eridinyl]-1-propanone; 1-(6-methyl-indol-2-yl)-3-[1-(phenylmethyl)-4-piper-idinyl]-1-propanone; 1-(-phenylsulfonyl-5-amino-indol-2-yl)-3-[1-(phenylm-ethyl)-4-piperidinyl]-1-propanone; 1-(5-amino-indol-2-yl)-3-[1-(phenylmet-hyl)-4-piperidinyl]-1-propanone; and 1-(5-acetylamino-indol-2-yl)-3-[1-(ph-enylmethyl)-4-piperidinyl]-1-propanone; 1-(6-quinolyl)-3-[1-(phenylmethyl)-4-piperidinyl]-1-propanone; 1-(5-indolyl)-3-[1-(phenylmethyl)-4-piperidiny-1]-1-propanone; 1-(5-benzthienyl)-3-[1-(phenylmethyl)-4-piperidinyl]-1-pro-panone; 1-(6-quinazolyl)-3-[1-(phenylmethyl)-4-piperidinyl]-1-propanone; 1-(6-benzoxazolyl)-3-[1-(phenylmethyl)-4-piperidinyl]-1-propanone; 1-(5-benzofuranyl)-3-[1-(phenylmethyl)-4-piperidinyl]-1-propanone; 1-(5-methyl-bnzimidazol-2-yl)-3-[1-(phenylmethyl)-4-piperidinyl]-1-propa-none; 1-(6-methyl-benzimidazol-2-yl)-3-[1-(phenylmethyl)-4-piperidinyl]-1-propanone; 1-(5-chloro-benzo[b]thien-2-yl)-3-[1-(phenylmethyl)-4-piperidin-yl]-1-propanone; 1-(5-azaindol-2-yl)-3-[1-(phenylmethyl)4-piperidinyl]-1-p-ropanone; 1-(6-azabenzo[b]thien-2-yl)-3-[1-(phenylmethyl)-4-piperidinyl]-1-propanone; 1-(1H-2-oxo-pyrrolo[2',3',5,6]benzo[b]thieno-2-yl)-3-[1-(phenylmethyl)-4-piperidinyl]-1-propanone; 1-(6-methyl-benzothiazol-2-yl)-3-[1-(phenylmethyl)-4-piperidinyl]-1-propanone; 1-(6-methoxy-indol-2-yl)-3-[1-(phenylmethyl)-4-piperidinyl]-1-propanone; 1-(6-methoxy-benzo[b]thien-2-yl)-3-[1-(phenylmethyl)-4-piperidinyl]-1-pro-panone; 1-(6-acetylamino-benzo[b]thien-2-yl)-3-[1-(phenylmethyl)-4-piperid-inyl]-1-propanone; 1-(5-acetylamino-benzo[b]thien-2-yl)-3-[1-(phenylmethyl-)-4-piperidinyl]-1-propanone; 6-hydroxy-3-[2-[1-(phenylmethyl)-4-piperidin-yl]ethyl] - 1,2-benzisoxazole; 5-methyl-3-[2-[1-(phenylmethyl)-4-piperidinyl-]ethyl]-1,2-benzisoxazole; 6-methoxy-3[2-[1(phenylmethyl)-4-piperidinyl]et-hyl]-1,2-benzisoxazole; 6-acetamide-3-[2-[1-(phenylmethyl)-4-piperidinyl]-ethyl]-1,2-benzisoxazole; 6-amino-3-[2-[1-(phenymethyl)-4-piperidinyl]ethy-1]-1,2-benzisoxazole; 6-(4-morpholinyl)-3-[2-1-(phenylmethyl)-4-piperidin-yl]ethyl]-1,2-benzisoxazole; 5,7-dihydro-3-[2-[1-(phenylmethyl)-4-piperidi-nyl]ethyl]-6H-pyrrolo[4,5-f]-1,2-benzisoxazol-6-one; 3-[2-[1-(phenylmethyl)-4-piperidinyl]ethyl]-1,2-benzisothiazole; 3-[2-[1-(phenylmethyl)-4-piperidinyl]ethenyl]-1,2-benzisoxazole; 6-phenylamino-3-[2-[1-(phenylmethyl)-4-piperidinyl]ethyl]-1,2,-benzisoxaz-ole; 6-(2-thiazoly)-3-[2-[1-(phenylmethyl)-4-piperidinyl]ethyl]-1,2-benzis-oxazole; 6-(2-oxazolyl)-3-[2-[1 -(phenylmethyl)-4-piperidinyl]ethyl]-1,2-benzisoxazole; 6-pyrrolidinyl-3-[2-[1-(phenylmethyl)-4-piperidinyl]ethyl]-1,-2-benzisoxazole; 5,7-dihydro-5,5-dimethyl-3-[2-[1-(phenylmethyl)-4-piperid-inyl]ethyl]-6H-pyrrolo[4,5-f]-1,2-benzisoxazole-6-one; 6,8-dihydro-3-[2-[1-(phenylmethyl)-4-piperidinyl]ethyl]-7H-pyrrolo[5,4-g]-1,2-benzisoxazole-7-one; 3-[2-[1-(phenylmethyl)-4-piperidinyl]ethyl]-5,6,-8-trihydro-7H-isoxazolo[4,5-g]-quinolin-7-one; 1-benzyl-4-((5,6-dimethoxy-1-indanon)-2-yl)methylpiperidine, 1-benzyl-4-((5,6-dimethoxy-1-indanon)-2-ylidenyl)methylpiperidine, 1-benzyl-4-((5-methoxy-1-indanon)-2-yl)methylp-iperidine, 1-benzyl-4-((5,6-diethoxy-1-indanon)-2-yl)methylpiperidine, 1-benzyl-4-((5,6-methnylenedioxy-1-indanon)-2-yl)methylpiperidine, 1-(m-nitrobenzyl)-4-((5,6-dimethoxy-1-indanon)-2-yl)methylpiperidine, 1-cyclohexymethyl-4-((5,6-dimethoxy-1-indanon)-2-yl)methylpiperidine, 1-(m-florobenzyl)-4-((5,6-dimethoxy-1-indanon)-2-yl)methylpiperidine, 1-benzyl-4-((5,6-dimethoxy-1-indanon)-2-yl)propylpiperidine, and 1-benzyl-4-((5-isopropoxy-6-methoxy-1-indanon)-2-yl)methylpiperidine.

Exemplary calcium channel antagonists include diltiazem, omega-conotoxin GVIA, methoxyverapamil, amlodipine, felodipine, lacidipine, and mibefradil.

Exemplary GABA-A receptor modulators include clomethiazole; IDDB; gaboxadol (4,5,6,7-tetrahydroisoxazolo[5,4-c]pyridin-3-ol); ganaxolone (3-alpha-hydroxy-3-beta-methyl-5-alpha-pregnan-20-one); fengabine (2-[(butylimino)-(2-chlorophenyl)methyl]-4-chlorophenol); 2-(4-methoxyphenyl)-2,5,6,7,8,9-hexahydro-pyrazolo[4,3-c]cinnolin-3-one; 7-cyclobutyl-6-(2-methyl-2H-1,2,4-triazol-3-ylmethoxy)-3-phenyl-1,2,4-triazolo[4,3-b]pyridazine; (3-fluoro-4-methylphenyl)-N-({-1 -[(2-methylphenyl)methyl]-benzimidazol-2-yl}methyl)-N-pentylcarboxamide; and 3-(aminomethyl)-5-methylhexanoic acid.

Exemplary potassium channel openers include diazoxide, flupirtine, pinacidil, levcromakalim, rilmakalim, chromakalim, PCO-400 and SKP-450 (2-[2"(1", 3"-dioxolone)-2-methyl]-4-(2'-oxo-1'-pyrrolidinyl)-6-nitro-2H-1-benzopyra-n).

Exemplary AMPA/kainate receptor antagonists include 6-cyano-7-nitroquinoxalin-2,3-di-one (CNQX); 6-nitro-7-sulphamoylbenzo[f]quinoxaline-2,3-dione (NBQX); 6,7-dinitroquinoxaline-2,3-dione (DNQX); 1-(4-aminophenyl)-4-methyl-7,8-m-ethylenedioxy-5H-2,3-benzodiazepine hydrochloride; and 2,3-dihydroxy-6-nitro-7-sulfamoylbenzo-[f]quinoxaline.

Exemplary sodium channel antagonists include ajmaline, procainamide, flecainide and riluzole.

Exemplary matrix-metalloprotease inhibitors include 4-[4-(4-fluorophenoxy)benzenesulfonylamino]tetrahydropyran-4-carboxylic acid hydroxyamide; 5-Methyl-5-(4-(4'-fluorophenoxy)-phenoxy)-pyrimidine-2,4,6-trione; 5-n-Butyl-5-(4-(4'-fluorophenoxy)-phenoxy)-pyrimidine-2,4,6-trione and prinomistat.

Exemplary inhibitors of p38 MAP kinase and c-jun-N-terminal kinases include pyridyl imidazoles, such as PD 169316, isomeric PD 169316, SB 203580, SB 202190, SB 220026, and RWJ 67657. Others are described in US Patent 6,288,089, and incorporated by reference herein.

In an exemplary embodiment, a combination therapy for treating or preventing MS comprises a therapeutically effective amount of one or more sirtuin-modulating compounds that increase the level and/or activity of a sirtuin protein and one or more of Avonex^{®} (interferon beta-1a), Tysabri^{®} (natalizumab), or Fumaderm^{®} (BG-12/Oral Fumarate).

In another embodiment, a combination therapy for treating or preventing diabetic neuropathy or conditions associated therewith comprises a therapeutically effective amount of one or more sirtuin-modulating compounds that increase the level and/or activity of a sirtuin protein and one or more of tricyclic antidepressants (TCAs) (including, for example, imipramine, amytriptyline, desipramine and nortriptyline), serotonin reuptake inhibitors (SSRIs) (including, for example, fluoxetine, paroxetine, sertralene, and citalopram) and antiepileptic drugs (AEDs) (including, for example, gabapentin, carbamazepine, and topimirate).

### Blood Coagulation Disorders

In other aspects, sirtuin-modulating compounds that increase the level and/or activity of a sirtuin protein can be used to treat or prevent blood coagulation disorders (or hemostatic disorders). As used interchangeably herein, the terms "hemostasis", "blood coagulation," and "blood clotting" refer to the control of bleeding, including the physiological properties of vasoconstriction and coagulation. Blood coagulation assists in maintaining the integrity of mammalian circulation after injury, inflammation, disease, congenital defect, dysfunction or other disruption. After initiation of clotting, blood coagulation proceeds through the sequential activation of certain plasma proenzymes to their enzyme forms (see, for example, Coleman, R. W. et al. (eds.) Hemostasis and Thrombosis, Second Edition, (1987)). These plasma glycoproteins, including Factor XII, Factor XI, Factor IX, Factor X, Factor VII, and prothrombin, are zymogens of serine proteases. Most of these blood clotting enzymes are effective on a physiological scale only when assembled in complexes on membrane surfaces with protein cofactors such as Factor VIII and Factor V. Other blood factors modulate and localize clot formation, or dissolve blood clots. Activated protein C is a specific enzyme that inactivates procoagulant components. Calcium ions are involved in many of the component reactions. Blood coagulation follows either the intrinsic pathway, where all of the protein components are present in blood, or the extrinsic pathway, where the cell-membrane protein tissue factor plays a critical role. Clot formation occurs when fibrinogen is cleaved by thrombin to form fibrin. Blood clots are composed of activated platelets and fibrin.

Further, the formation of blood clots does not only limit bleeding in case of an injury (hemostasis), but may lead to serious organ damage and death in the context of atherosclerotic diseases by occlusion of an important artery or vein. Thrombosis is thus blood clot formation at the wrong time and place. It involves a cascade of complicated and regulated biochemical reactions between circulating blood proteins (coagulation factors), blood cells (in particular platelets), and elements of an injured vessel wall.

Accordingly, the present invention provides anticoagulation and antithrombotic treatments aiming at inhibiting the formation of blood clots in order to prevent or treat blood coagulation disorders, such as myocardial infarction, stroke, loss of a limb by peripheral artery disease or pulmonary embolism.

As used interchangeably herein, "modulating or modulation of hemostasis" and "regulating or regulation of hemostasis" includes the induction (e.g., stimulation or increase) of hemostasis, as well as the inhibition (e.g., reduction or decrease) of hemostasis.

In one aspect, the invention provides a method for reducing or inhibiting hemostasis in a subject by administering a sirtuin-modulating compound that increases the level and/or activity of a sirtuin protein. The compositions and methods disclosed herein are useful for the treatment or prevention of thrombotic disorders. As used herein, the term "thrombotic disorder" includes any disorder or condition characterized by excessive or unwanted coagulation or hemostatic activity, or a hypercoagulable state. Thrombotic disorders include diseases or disorders involving platelet adhesion and thrombus formation, and may manifest as an increased propensity to form thromboses, e.g., an increased number of thromboses, thrombosis at an early age, a familial tendency towards thrombosis, and thrombosis at unusual sites. Examples of thrombotic disorders include, but are not limited to, thromboembolism, deep vein thrombosis, pulmonary embolism, stroke, myocardial infarction, miscarriage, thrombophilia associated with anti-thrombin III deficiency, protein C deficiency, protein S deficiency, resistance to activated protein C, dysfibrinogenemia, fibrinolytic disorders, homocystinuria, pregnancy, inflammatory disorders, myeloproliferative disorders, arteriosclerosis, angina, e.g., unstable angina, disseminated intravascular coagulation, thrombotic thrombocytopenic purpura, cancer metastasis, sickle cell disease, glomerular nephritis, and drug induced thrombocytopenia (including, for example, heparin induced thrombocytopenia). In addition, sirtuin-modulating compounds that increase the level and/or activity of a sirtuin protein may be administered to prevent thrombotic events or to prevent reocclusion during or after therapeutic clot lysis or procedures such as angioplasty or surgery.

In another embodiment, a combination drug regimen may include drugs or compounds for the treatment or prevention of blood coagulation disorders or secondary conditions associated with these conditions. Thus, a combination drug regimen may include one or more sirtuin-modulating compounds that increase the level and/or activity of a sirtuin protein and one or more anti-coagulation or anti-thrombosis agents. For example, one or more sirtuin-modulating compounds can be combined with an effective amount of one or more of: aspirin, heparin, and oral Warfarin that inhibits Vit K-dependent factors, low molecular weight heparins that inhibit factors X and II, thrombin inhibitors, inhibitors of platelet GP IIbIIIa receptors, inhibitors of tissue factor (TF), inhibitors of human von Willebrand factor, inhibitors of one or more factors involved in hemostasis (in particular in the coagulation cascade). In addition, sirtuin-modulating compounds that increase the level and/or activity of a sirtuin protein can be combined with thrombolytic agents, such as t-PA, streptokinase, reptilase, TNK-t-PA, and staphylokinase.

### Weight Control

In another aspect, sirtuin-modulating compounds that increase the level and/or activity of a sirtuin protein may be used for treating or preventing weight gain or obesity in a subject. For example, sirtuin-modulating compounds that increase the level and/or activity of a sirtuin protein may be used, for example, to treat or prevent hereditary obesity, dietary obesity, hormone related obesity, obesity related to the administration of medication, to reduce the weight of a subject, or to reduce or prevent weight gain in a subject. A subject in need of such a treatment may be a subject who is obese, likely to become obese, overweight, or likely to become overweight. Subjects who are likely to become obese or overweight can be identified, for example, based on family history, genetics, diet, activity level, medication intake, or various combinations thereof.

In yet other embodiments, sirtuin-modulating compounds that increase the level and/or activity of a sirtuin protein may be administered to subjects suffering from a variety of other diseases and conditions that may be treated or prevented by promoting weight loss in the subject. Such diseases include, for example, high blood pressure, hypertension, high blood cholesterol, dyslipidemia, type 2 diabetes, insulin resistance, glucose intolerance, hyperinsulinemia, coronary heart disease, angina pectoris, congestive heart failure, stroke, gallstones, cholescystitis and cholelithiasis, gout, osteoarthritis, obstructive sleep apnea and respiratory problems, some types of cancer (such as endometrial, breast, prostate, and colon), complications of pregnancy, poor female reproductive health (such as menstrual irregularities, infertility, irregular ovulation), bladder control problems (such as stress incontinence); uric acid nephrolithiasis; psychological disorders (such as depression, eating disorders, distorted body image, and low self esteem). Stunkard AJ, Wadden TA. (Editors) Obesity: theory and therapy, Second Edition. New York: Raven Press, 1993. Finally, patients with AIDS can develop lipodystrophy or insulin resistance in response to combination therapies for AIDS.

In another embodiment, sirtuin-modulating compounds that increase the level and/or activity of a sirtuin protein may be used for inhibiting adipogenesis or fat cell differentiation, whether in vitro or in vivo. In particular, high circulating levels of insulin and/or insulin like growth factor (IGF) 1 will be prevented from recruiting preadipocytes to differentiate into adipocytes. Such methods may be used for treating or preventing obesity.

In other embodiments, sirtuin-modulating compounds that increase the level and/or activity of a sirtuin protein may be used for reducing appetite and/or increasing satiety, thereby causing weight loss or avoidance of weight gain. A subject in need of such a treatment may be a subject who is overweight, obese or a subject likely to become overweight or obese. The method may comprise administering daily or, every other day, or once a week, a dose, e.g., in the form of a pill, to a subject. The dose may be an "appetite reducing dose."

In other embodiments, a sirtuin-modulating compound that decreases the level and/or activity of a sirtuin protein may be used to stimulate appetite and/or weight gain. A method may comprise administering to a subject, such as a subject in need thereof, a pharmaceutically effective amount of a sirtuin-modulating agent that decreases the level and/or activity of a sirtuin protein, such as SIRT1. A subject in need of such a treatment may be a subject who has cachexia or may be likely to develop cachexia. A combination of agents may also be administered. A method may further comprise monitoring in the subject the state of the disease or of activation of sirtuins, for example, in adipose tissue.

Methods for stimulating fat accumulation in cells may be used in vitro, to establish cell models of weight gain, which may be used, e.g., for identifying other drugs that prevent weight gain.

Also provided are methods for modulating adipogenesis or fat cell differentiation, whether in vitro or in vivo. In particular, high circulating levels of insulin and/or insulin like growth factor (IGF) 1 will be prevented from recruiting preadipocytes to differentiate into adipocytes. Such methods may be used to modulate obesity. A method for stimulating adipogenesis may comprise contacting a cell with a sirtuin-modulating agent that decreases the level and/or activity of a sirtuin protein.

In another embodiment, the invention provides methods of decreasing fat or lipid metabolism in a subject by administering a sirtuin-modulating compound that decreases the level and/or activity of a sirtuin protein. The method includes administering to a subject an amount of a sirtuin-modulating compound, e.g., in an amount effective to decrease mobilization of fat to the blood from WAT cells and/or to decrease fat burning by BAT cells.

Methods for promoting appetite and/or weight gain may include, for example, prior identifying a subject as being in need of decreased fat or lipid metabolism, e.g., by weighing the subject, determining the BMI of the subject, or evaluating fat content of the subject or sirtuin activity in cells of the subject. The method may also include monitoring the subject, e.g., during and/or after administration of a sirtuin-modulating compound. The administering can include one or more dosages, e.g., delivered in boluses or continuously. Monitoring can include evaluating a hormone or a metabolite. Exemplary hormones include leptin, adiponectin, resistin, and insulin. Exemplary metabolites include triglyercides, cholesterol, and fatty acids.

In one embodiment, a sirtuin-modulating compound that decreases the level and/or activity of a sirtuin protein may be used to modulate (e.g., increase) the amount of subcutaneous fat in a tissue, e.g., in facial tissue or in other surface-associated tissue of the neck, hand, leg, or lips. The sirtuin-modulating compound may be used to increase the rigidity, water retention, or support properties of the tissue. For example, the sirtuin-modulating compound can be applied topically, e.g., in association with another agent, e.g., for surface-associated tissue treatment. The sirtuin-modulating compound may also be injected subcutaneously, e.g., within the region where an alteration in subcutaneous fat is desired.

A method for modulating weight may further comprise monitoring the weight of the subject and/or the level of modulation of sirtuins, for example, in adipose tissue.

In an exemplary embodiment, sirtuin-modulating compounds that increase the level and/or activity of a sirtuin protein may be administered as a combination therapy for treating or preventing weight gain or obesity. For example, one or more sirtuin-modulating compounds that increase the level and/or activity of a sirtuin protein may be administered in combination with one or more anti-obesity agents. Exemplary anti-obesity agents include, for example, phenylpropanolamine, ephedrine, pseudoephedrine, phentermine, a cholecystokinin-A agonist, a monoamine reuptake inhibitor (such as sibutramine), a sympathomimetic agent, a serotonergic agent (such as dexfenfluramine or fenfluramine), a dopamine agonist (such as bromocriptine), a melanocyte-stimulating hormone receptor agonist or mimetic, a melanocyte-stimulating hormone analog, a cannabinoid receptor antagonist, a melanin concentrating hormone antagonist, the OB protein (leptin), a leptin analog, a leptin receptor agonist, a galanin antagonist or a GI lipase inhibitor or decreaser (such as orlistat). Other anorectic agents include bombesin agonists, dehydroepiandrosterone or analogs thereof, glucocorticoid receptor agonists and antagonists, orexin receptor antagonists, urocortin binding protein antagonists, agonists of the glucagon-like peptide-1 receptor such as Exendin and ciliary neurotrophic factors such as Axokine.

In another embodiment, sirtuin-modulating compounds that increase the level and/or activity of a sirtuin protein may be administered to reduce drug-induced weight gain. For example, a sirtuin-modulating compound that increases the level and/or activity of a sirtuin protein may be administered as a combination therapy with medications that may stimulate appetite or cause weight gain, in particular, weight gain due to factors other than water retention. Examples of medications that may cause weight gain, include for example, diabetes treatments, including, for example, sulfonylureas (such as glipizide and glyburide), thiazolidinediones (such as pioglitazone and rosiglitazone), meglitinides, nateglinide, repaglinide, sulphonylurea medicines, and insulin; anti-depressants, including, for example, tricyclic antidepressants (such as amitriptyline and imipramine), irreversible monoamine oxidase inhibitors (MAOIs), selective serotonin reuptake inhibitors (SSRIs), bupropion, paroxetine, and mirtazapine; steroids, such as, for example, prednisone; hormone therapy; lithium carbonate; valproic acid; carbamazepine; chlorpromazine; thiothixene; beta blockers (such as propranolo); alpha blockers (such as clonidine, prazosin and terazosin); and contraceptives including oral contraceptives (birth control pills) or other contraceptives containing estrogen and/or progesterone (Depo-Provera, Norplant, Ortho), testosterone or Megestrol. In another exemplary embodiment, sirtuin-modulating compounds that increase the level and/or activity of a sirtuin protein may be administered as part of a smoking cessation program to prevent weight gain or reduce weight already gained.

### Metabolic Disorders/Diabetes

In another aspect, sirtuin-modulating compounds that increase the level and/or activity of a sirtuin protein may be used for treating or preventing a metabolic disorder, such as insulin-resistance, a pre-diabetic state, type II diabetes, and/or complications thereof Administration of a sirtuin-modulating compounds that increases the level and/or activity of a sirtuin protein may increase insulin sensitivity and/or decrease insulin levels in a subject. A subject in need of such a treatment may be a subject who has insulin resistance or other precursor symptom of type II diabetes, who has type II diabetes, or who is likely to develop any of these conditions. For example, the subject may be a subject having insulin resistance, e.g., having high circulating levels of insulin and/or associated conditions, such as hyperlipidemia, dyslipogenesis, hypercholesterolemia, impaired glucose tolerance, high blood glucose sugar level, other manifestations of syndrome X, hypertension, atherosclerosis and lipodystrophy.

In an exemplary embodiment, sirtuin-modulating compounds that increase the level and/or activity of a sirtuin protein may be administered as a combination therapy for treating or preventing a metabolic disorder. For example, one or more sirtuin-modulating compounds that increase the level and/or activity of a sirtuin protein may be administered in combination with one or more anti-diabetic agents. Exemplary anti-diabetic agents include, for example, an aldose reductase inhibitor, a glycogen phosphorylase inhibitor, a sorbitol dehydrogenase inhibitor, a protein tyrosine phosphatase 1B inhibitor, a dipeptidyl protease inhibitor, insulin (including orally bioavailable insulin preparations), an insulin mimetic, metformin, acarbose, a peroxisome proliferator-activated receptor-γ (PPAR-γ) ligand such as troglitazone, rosaglitazone, pioglitazone or GW-1929, a sulfonylurea, glipazide, glyburide, or chlorpropamide wherein the amounts of the first and second compounds result in a therapeutic effect. Other anti-diabetic agents include a glucosidase inhibitor, a glucagon-like peptide-1 (GLP-1), insulin, a PPAR α/γ dual agonist, a meglitimide and an αP2 inhibitor. In an exemplary embodiment, an anti-diabetic agent may be a dipeptidyl peptidase IV (DP-IV or DPP-IV) inhibitor, such as, for example LAF237 from Novartis (NVP DPP728; 1-[[[2-[(5-cyanopyridin-2-yl)amino] ethyl]amino]acetyl]-2- cyano-(S)- pyrrolidine) or MK-04301 from Merck (see e.g., Hughes et al., Biochemistry 38: 11597-603 (1999)).

### Inflammatory Diseases

In other aspects, sirtuin-modulating compounds that increase the level and/or activity of a sirtuin protein can be used to treat or prevent a disease or disorder associated with inflammation. Sirtuin-modulating compounds that increase the level and/or activity of a sirtuin protein may be administered prior to the onset of, at, or after the initiation of inflammation. When used prophylactically, the compounds are preferably provided in advance of any inflammatory response or symptom. Administration of the compounds may prevent or attenuate inflammatory responses or symptoms.

Exemplary inflammatory conditions include, for example, multiple sclerosis, rheumatoid arthritis, psoriatic arthritis, degenerative joint disease, spondouloarthropathies, gouty arthritis, systemic lupus erythematosus, juvenile arthritis, rheumatoid arthritis, osteoarthritis, osteoporosis, diabetes (e.g., insulin dependent diabetes mellitus or juvenile onset diabetes), menstrual cramps, cystic fibrosis, inflammatory bowel disease, irritable bowel syndrome, Crohn's disease, mucous colitis, ulcerative colitis, gastritis, esophagitis, pancreatitis, peritonitis, Alzheimer's disease, shock, ankylosing spondylitis, gastritis, conjunctivitis, pancreatis (acute or chronic), multiple organ injury syndrome (e.g., secondary to septicemia or trauma), myocardial infarction, atherosclerosis, stroke, reperfusion injury (e.g., due to cardiopulmonary bypass or kidney dialysis), acute glomerulonephritis, vasculitis, thermal injury (i.e., sunburn), necrotizing enterocolitis, granulocyte transfusion associated syndrome, and/or Sjogren's syndrome. Exemplary inflammatory conditions of the skin include, for example, eczema, atopic dermatitis, contact dermatitis, urticaria, schleroderma, psoriasis, and dermatosis with acute inflammatory components.

In another embodiment, sirtuin-modulating compounds that increase the level and/or activity of a sirtuin protein may be used to treat or prevent allergies and respiratory conditions, including asthma, bronchitis, pulmonary fibrosis, allergic rhinitis, oxygen toxicity, emphysema, chronic bronchitis, acute respiratory distress syndrome, and any chronic obstructive pulmonary disease (COPD). The compounds may be used to treat chronic hepatitis infection, including hepatitis B and hepatitis C.

Additionally, sirtuin-modulating compounds that increase the level and/or activity of a sirtuin protein may be used to treat autoimmune diseases and/or inflammation associated with autoimmune diseases such as organ-tissue autoimmune diseases (e.g., Raynaud's syndrome), scleroderma, myasthenia gravis, transplant rejection, endotoxin shock, sepsis, psoriasis, eczema, dermatitis, multiple sclerosis, autoimmune thyroiditis, uveitis, systemic lupus erythematosis, Addison's disease, autoimmune polyglandular disease (also known as autoimmune polyglandular syndrome), and Grave's disease.

In certain embodiments, one or more sirtuin-modulating compounds that increase the level and/or activity of a sirtuin protein may be taken alone or in combination with other compounds useful for treating or preventing inflammation. Exemplary anti-inflammatory agents include, for example, steroids (e.g., cortisol, cortisone, fludrocortisone, prednisone, 6-alpha-methylprednisone, triamcinolone, betamethasone or dexamethasone), nonsteroidal antiinflammatory drugs (NSAIDS (e.g., aspirin, acetaminophen, tolmetin, ibuprofen, mefenamic acid, piroxicam, nabumetone, rofecoxib, celecoxib, etodolac or nimesulide). In another embodiment, the other therapeutic agent is an antibiotic (e.g., vancomycin, penicillin, amoxicillin, ampicillin, cefotaxime, ceftriaxone, cefixime, rifampinmetronidazole, doxycycline or streptomycin). In another embodiment, the other therapeutic agent is a PDE4 inhibitor (e.g., roflumilast or rolipram). In another embodiment, the other therapeutic agent is an antihistamine (e.g., cyclizine, hydroxyzine, promethazine or diphenhydramine). In another embodiment, the other therapeutic agent is an anti-malarial (e.g., artemisinin, artemether, artsunate, chloroquine phosphate, mefloquine hydrochloride, doxycycline hyclate, proguanil hydrochloride, atovaquone or halofantrine). In one embodiment, the other therapeutic agent is drotrecogin alfa.

Further examples of anti-inflammatory agents include, for example, aceclofenac, acemetacin, e-acetamidocaproic acid, acetaminophen, acetaminosalol, acetanilide, acetylsalicylic acid, S-adenosylmethionine, alclofenac, alclometasone, alfentanil, algestone, allylprodine, alminoprofen, aloxiprin, alphaprodine, aluminum bis(acetylsalicylate), amcinonide, amfenac, aminochlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antrafenine, apazone, beclomethasone, bendazac, benorylate, benoxaprofen, benzpiperylon, benzydamine, benzylmorphine, bermoprofen, betamethasone, betamethasone-17-valerate, bezitramide, .alpha.-bisabolol, bromfenac, p-bromoacetanilide, 5-bromosalicylic acid acetate, bromosaligenin, bucetin, bucloxic acid, bucolome, budesonide, bufexamac, bumadizon, buprenorphine, butacetin, butibufen, butorphanol, carbamazepine, carbiphene, carprofen, carsalam, chlorobutanol, chloroprednisone, chlorthenoxazin, choline salicylate, cinchophen, cinmetacin, ciramadol, clidanac, clobetasol, clocortolone, clometacin, clonitazene, clonixin, clopirac, cloprednol, clove, codeine, codeine methyl bromide, codeine phosphate, codeine sulfate, cortisone, cortivazol, cropropamide, crotethamide, cyclazocine, deflazacort, dehydrotestosterone, desomorphine, desonide, desoximetasone, dexamethasone, dexamethasone-21-isonicotinate, dexoxadrol, dextromoramide, dextropropoxyphene, deoxycorticosterone, dezocine, diampromide, diamorphone, diclofenac, difenamizole, difenpiramide, diflorasone, diflucortolone, diflunisal, difluprednate, dihydrocodeine, dihydrocodeinone enol acetate, dihydromorphine, dihydroxyaluminum acetylsalicylate, dimenoxadol, dimepheptanol, dimethylthiambutene, dioxaphetyl butyrate, dipipanone, diprocetyl, dipyrone, ditazol, droxicam, emorfazone, enfenamic acid, enoxolone, epirizole, eptazocine, etersalate, ethenzamide, ethoheptazine, ethoxazene, ethylmethylthiambutene, ethylmorphine, etodolac, etofenamate, etonitazene, eugenol, felbinac, fenbufen, fenclozic acid, fendosal, fenoprofen, fentanyl, fentiazac, fepradinol, feprazone, floctafenine, fluazacort, flucloronide, flufenamic acid, flumethasone, flunisolide, flunixin, flunoxaprofen, fluocinolone acetonide, fluocinonide, fluocinolone acetonide, fluocortin butyl, fluocortolone, fluoresone, fluorometholone, fluperolone, flupirtine, fluprednidene, fluprednisolone, fluproquazone, flurandrenolide, flurbiprofen, fluticasone, formocortal, fosfosal, gentisic acid, glafenine, glucametacin, glycol salicylate, guaiazulene, halcinonide, halobetasol, halometasone, haloprednone, heroin, hydrocodone, hydrocortamate, hydrocortisone, hydrocortisone acetate, hydrocortisone succinate, hydrocortisone hemisuccinate, hydrocortisone 21-lysinate, hydrocortisone cypionate, hydromorphone, hydroxypethidine, ibufenac, ibuprofen, ibuproxam, imidazole salicylate, indomethacin, indoprofen, isofezolac, isoflupredone, isoflupredone acetate, isoladol, isomethadone, isonixin, isoxepac, isoxicam, ketobemidone, ketoprofen, ketorolac, p-lactophenetide, lefetamine, levallorphan, levorphanol, levophenacyl-morphan, lofentanil, lonazolac, lornoxicam, loxoprofen, lysine acetylsalicylate, mazipredone, meclofenamic acid, medrysone, mefenamic acid, meloxicam, meperidine, meprednisone, meptazinol, mesalamine, metazocine, methadone, methotrimeprazine, methylprednisolone, methylprednisolone acetate, methylprednisolone sodium succinate, methylprednisolone suleptnate, metiazinic acid, metofoline, metopon, mofebutazone, mofezolac, mometasone, morazone, morphine, morphine hydrochloride, morphine sulfate, morpholine salicylate, myrophine, nabumetone, nalbuphine, nalorphine, 1-naphthyl salicylate, naproxen, narceine, nefopam, nicomorphine, nifenazone, niflumic acid, nimesulide, 5'-nitro-2'-propoxyacetanilide, norlevorphanol, normethadone, normorphine, norpipanone, olsalazine, opium, oxaceprol, oxametacine, oxaprozin, oxycodone, oxymorphone, oxyphenbutazone, papaveretum, paramethasone, paranyline, parsalmide, pentazocine, perisoxal, phenacetin, phenadoxone, phenazocine, phenazopyridine hydrochloride, phenocoll, phenoperidine, phenopyrazone, phenomorphan, phenyl acetylsalicylate, phenylbutazone, phenyl salicylate, phenyramidol, piketoprofen, piminodine, pipebuzone, piperylone, pirazolac, piritramide, piroxicam, pirprofen, pranoprofen, prednicarbate, prednisolone, prednisone, prednival, prednylidene, proglumetacin, proheptazine, promedol, propacetamol, properidine, propiram, propoxyphene, propyphenazone, proquazone, protizinic acid, proxazole, ramifenazone, remifentanil, rimazolium metilsulfate, salacetamide, salicin, salicylamide, salicylamide o-acetic acid, salicylic acid, salicylsulfuric acid, salsalate, salverine, simetride, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tixocortol, tolfenamic acid, tolmetin, tramadol, triamcinolone, triamcinolone acetonide, tropesin, viminol, xenbucin, ximoprofen, zaltoprofen and zomepirac.

In an exemplary embodiment, a sirtuin-modulating compound that increases the level and/or activity of a sirtuin protein may be administered with a selective COX-2 inhibitor for treating or preventing inflammation. Exemplary selective COX-2 inhibitors include, for example, deracoxib, parecoxib, celecoxib, valdecoxib, rofecoxib, etoricoxib, lumiracoxib, 2-(3,5-difluorophenyl)-3-[4-(methylsulfonyl)phenyl]-2-cyclopenten-1-one, (S)-6,8-dichloro-2-(trifluoromethyl)-2H-1-benzopyran-3-carboxylic acid, 2-(3,4-difluorophenyl)-4-(3-hydroxy-3-methyl-1-butoxy)-5-[4-(methylsulfonyl)phenyl]-3-(2H)-pyridazinone, 4-[5-(4-fluorophenyl)-3-(trifluoromethyl)-1H-pyrazol-1-yl]benzenesulfonamide, tert-butyl 1 benzyl-4-[(4-oxopiperidin-1-yl}sulfonyl]piperidine-4-carboxylate, 4-[5-(phenyl)-3-(trifluoromethyl)-1H-pyrazol-1-yl]benzenesulfonamide, salts and prodrugs thereof.

### Flushing

In another aspect, sirtuin-modulating compounds that increase the level and/or activity of a sirtuin protein may be used for reducing the incidence or severity of flushing and/or hot flashes which are symptoms of a disorder. For instance, the subject method includes the use of sirtuin-modulating compounds that increase the level and/or activity of a sirtuin protein, alone or in combination with other agents, for reducing incidence or severity of flushing and/or hot flashes in cancer patients. In other embodiments, the method provides for the use of sirtuin-modulating compounds that increase the level and/or activity of a sirtuin protein to reduce the incidence or severity of flushing and/or hot flashes in menopausal and post-menopausal woman.

In another aspect, sirtuin-modulating compounds that increase the level and/or activity of a sirtuin protein may be used as a therapy for reducing the incidence or severity of flushing and/or hot flashes which are side-effects of another drug therapy, e.g., drug-induced flushing. In certain embodiments, a method for treating and/or preventing drug-induced flushing comprises administering to a patient in need thereof a formulation comprising at least one flushing inducing compound and at least one sirtuin-modulating compound that increases the level and/or activity of a sirtuin protein. In other embodiments, a method for treating drug induced flushing comprises separately administering one or more compounds that induce flushing and one or more sirtuin-modulating compounds, e.g., wherein the sirtuin-modulating compound and flushing inducing agent have not been formulated in the same compositions. When using separate formulations, the sirtuin-modulating compound may be administered (1) at the same as administration of the flushing inducing agent, (2) intermittently with the flushing inducing agent, (3) staggered relative to administration of the flushing inducing agent, (4) prior to administration of the flushing inducing agent, (5) subsequent to administration of the flushing inducing agent, and (6) various combination thereof. Exemplary flushing inducing agents include, for example, niacin, faloxifene, antidepressants, anti-psychotics, chemotherapeutics, calcium channel blockers, and antibiotics.

In one embodiment, sirtuin-modulating compounds that increase the level and/or activity of a sirtuin protein may be used to reduce flushing side effects of a vasodilator or an antilipemic agent (including anticholesteremic agents and lipotropic agents). In an exemplary embodiment, a sirtuin-modulating compound that increases the level and/or activity of a sirtuin protein may be used to reduce flushing associated with the administration of niacin.

Nicotinic acid, 3-pyridinecarboxylic acid or niacin, is an antilipidemic agent that is marketed under, for example, the trade names Nicolar^{®}, SloNiacin^{®}, Nicobid^{®} and Time Release Niacin^{®}. Nicotinic acid has been used for many years in the treatment of lipidemic disorders such as hyperlipidemia, hypercholesterolemia and atherosclerosis. This compound has long been known to exhibit the beneficial effects of reducing total cholesterol, low density lipoproteins or "LDL cholesterol," triglycerides and apolipoprotein a (Lp(a)) in the human body, while increasing desirable high density lipoproteins or "HDL cholesterol".

Typical doses range from about 1 gram to about 3 grams daily. Nicotinic acid is normally administered two to four times per day after meals, depending upon the dosage form selected. Nicotinic acid is currently commercially available in two dosage forms. One dosage form is an immediate or rapid release tablet which should be administered three or four times per day. Immediate release ("IR") nicotinic acid formulations generally release nearly all of their nicotinic acid within about 30 to 60 minutes following ingestion. The other dosage form is a sustained release form which is suitable for administration two to four times per day. In contrast to IR formulations, sustained release ("SR") nicotinic acid formulations are designed to release significant quantities of drug for absorption into the blood stream over specific timed intervals in order to maintain therapeutic levels of nicotinic acid over an extended period such as 12 or 24 hours after ingestion.

As used herein, the term "nicotinic acid" is meant to encompass nicotinic acid or a compound other than nicotinic acid itself which the body metabolizes into nicotinic acid, thus producing essentially the same effect as nicotinic acid. Exemplary compounds that produce an effect similar to that of nicotinic acid include, for example, nicotinyl alcohol tartrate, d-glucitol hexanicotinate, aluminum nicotinate, niceritrol and d,1-alpha-tocopheryl nicotinate. Each such compound will be collectively referred to herein as "nicotinic acid."

In another embodiment, the invention provides a method for treating and/or preventing hyperlipidemia with reduced flushing side effects. The method comprises the steps of administering to a subject in need thereof a therapeutically effective amount of nicotinic acid and a sirtuin-modulating compound that increases the level and/or activity of a sirtuin protein in an amount sufficient to reduce flushing. In an exemplary embodiment, the nicotinic acid and/or sirtuin-modulating compound may be administered nocturnally.

In another representative embodiment, the method involves the use of sirtuin-modulating compounds that increase the level and/or activity of a sirtuin protein to reduce flushing side effects of raloxifene. Raloxifene acts like estrogen in certain places in the body, but is not a hormone. It helps prevent osteoporosis in women who have reached menopause. Osteoporosis causes bones to gradually grow thin, fragile, and more likely to break. Evista slows down the loss of bone mass that occurs with menopause, lowering the risk of spine fractures due to osteoporosis. A common side effect of raloxifene is hot flashes (sweating and flushing). This can be uncomfortable for women who already have hot flashes due to menopause.

In another representative embodiment, the method involves the use of sirtuin-modulating compounds that increase the level and/or activity of a sirtuin protein to reduce flushing side effects of antidepressants or anti-psychotic agent. For instance, sirtuin-modulating compounds that increase the level and/or activity of a sirtuin protein can be used in conjunction (administered separately or together) with a serotonin reuptake inhibitor, a 5HT2 receptor antagonist, an anticonvulsant, a norepinephrine reuptake inhibitor, an α-adrenoreceptor antagonist, an NK-3 antagonist, an NK-1 receptor antagonist, a PDE4 inhibitor, an Neuropeptide Y5 Receptor Antagonists, a D4 receptor antagonist, a 5HT1A receptor antagonist, a 5HT1D receptor antagonist, a CRF antagonist, a monoamine oxidase inhibitor, or a sedative-hypnotic drug.

In certain embodiments, sirtuin-modulating compounds that increase the level and/or activity of a sirtuin protein may be used as part of a treatment with a serotonin reuptake inhibitor (SRI) to reduce flushing. In certain preferred embodiments, the SRI is a selective serotonin reuptake inhibitor (SSRI), such as a fluoxetinoid (fluoxetine, norfluoxetine) or a nefazodonoid (nefazodone, hydroxynefazodone, oxonefazodone). Other exemplary SSRI's include duloxetine, venlafaxine, milnacipran, citalopram, fluvoxamine, paroxetine and sertraline. The sirtuin-modulating compound that increases the level and/or activity of a sirtuin protein can also be used as part of a treatment with sedative-hypnotic drug, such as selected from the group consisting of a benzodiazepine (such as alprazolam, chlordiazepoxide, clonazepam, chlorazepate, clobazam, diazepam, halazepam, lorazepam, oxazepam and prazepam), zolpidem, and barbiturates. In still other embodiments, a sirtuin-modulating compound that increases the level and/or activity of a sirtuin protein may be used as part of a treatment with a 5-HT1A receptor partial agonist, such as selected from the group consisting of buspirone, flesinoxan, gepirone and ipsapirone. Sirtuin-modulating compounds that increase the level and/or activity of a sirtuin protein can also used as part of a treatment with a norepinephrine reuptake inhibitor, such as selected from tertiary amine tricyclics and secondary amine tricyclics. Exemplary tertiary amine tricyclics include amitriptyline, clomipramine, doxepin, imipramine and trimipramine. Exemplary secondary amine tricyclics include amoxapine, desipramine, maprotiline, nortriptyline and protriptyline. In certain embodiments, sirtuin-modulating compounds that increase the level and/or activity of a sirtuin protein may be used as part of a treatment with a monoamine oxidase inhibitor, such as selected from the group consisting of isocarboxazid, phenelzine, tranylcypromine, selegiline and moclobemide.

In still another representative embodiment, sirtuin-modulating compounds that increase the level and/or activity of a sirtuin protein may be used to reduce flushing side effects of chemotherapeutic agents, such as cyclophosphamide, tamoxifen.

In another embodiment, sirtuin-modulating compounds that increase the level and/or activity of a sirtuin protein may be used to reduce flushing side effects of calcium channel blockers, such as amlodipine.

In another embodiment, sirtuin-modulating compounds that increase the level and/or activity of a sirtuin protein may be used to reduce flushing side effects of antibiotics. For example, sirtuin-modulating compounds that increase the level and/or activity of a sirtuin protein can be used in combination with levofloxacin. Levofloxacin is used to treat infections of the sinuses, skin, lungs, ears, airways, bones, and joints caused by susceptible bacteria. Levofloxacin also is frequently used to treat urinary infections, including those resistant to other antibiotics, as well as prostatitis. Levofloxacin is effective in treating infectious diarrheas caused by E. coli, campylobacter jejuni, and shigella bacteria. Levofloxacin also can be used to treat various obstetric infections, including mastitis.

### Other Uses

Sirtuin-modulating compounds that increase the level and/or activity of a sirtuin protein may be used for treating or preventing viral infections (such as infections by influenza, herpes or papilloma virus) or as antifungal agents. In certain embodiments, sirtuin-modulating compounds that increase the level and/or activity of a sirtuin protein may be administered as part of a combination drug therapy with another therapeutic agent for the treatment of viral diseases, including, for example, acyclovir, ganciclovir and zidovudine. In another embodiment, sirtuin-modulating compounds that increase the level and/or activity of a sirtuin protein may be administered as part of a combination drug therapy with another anti-fungal agent including, for example, topical anti-fungals such as ciclopirox, clotrimazole, econazole, miconazole, nystatin, oxiconazole, terconazole, and tolnaftate, or systemic anti-fungal such as fluconazole (Diflucan), itraconazole (Sporanox), ketoconazole (Nizoral), and miconazole (Monistat I.V.).

Subjects that may be treated as described herein include eukaryotes, such as mammals, e.g., humans, ovines, bovines, equines, porcines, canines, felines, non-human primate, mice, and rats. Cells that may be treated include eukaryotic cells, e.g., from a subject described above, or plant cells, yeast cells and prokaryotic cells, e.g., bacterial cells. For example, modulating compounds may be administered to farm animals to improve their ability to withstand farming conditions longer.

Sirtuin-modulating compounds that increase the level and/or activity of a sirtuin protein may also be used to increase lifespan, stress resistance, and resistance to apoptosis in plants. In one embodiment, a compound is applied to plants, e.g., on a periodic basis, or to fungi. In another embodiment, plants are genetically modified to produce a compound. In another embodiment, plants and fruits are treated with a compound prior to picking and shipping to increase resistance to damage during shipping. Plant seeds may also be contacted with compounds described herein, e.g., to preserve them.

In other embodiments, sirtuin-modulating compounds that increase the level and/or activity of a sirtuin protein may be used for modulating lifespan in yeast cells. Situations in which it may be desirable to extend the lifespan of yeast cells include any process in which yeast is used, e.g., the making of beer, yogurt, and bakery items, e.g., bread. Use of yeast having an extended lifespan can result in using less yeast or in having the yeast be active for longer periods of time. Yeast or other mammalian cells used for recombinantly producing proteins may also be treated as described herein.

Sirtuin-modulating compounds that increase the level and/or activity of a sirtuin protein may also be used to increase lifespan, stress resistance and resistance to apoptosis in insects. In this embodiment, compounds would be applied to useful insects, e.g., bees and other insects that are involved in pollination of plants. In a specific embodiment, a compound would be applied to bees involved in the production of honey. Generally, the methods described herein may be applied to any organism, e.g., eukaryote, that may have commercial importance. For example, they can be applied to fish (aquaculture) and birds (e.g., chicken and fowl).

Higher doses of sirtuin-modulating compounds that increase the level and/or activity of a sirtuin protein may also be used as a pesticide by interfering with the regulation of silenced genes and the regulation of apoptosis during development. In this embodiment, a compound may be applied to plants using a method known in the art that ensures the compound is bio-available to insect larvae, and not to plants.

At least in view of the link between reproduction and longevity (Longo and Finch, Science, 2002), sirtuin-modulating compounds that increase the level and/or activity of a sirtuin protein can be applied to affect the reproduction of organisms such as insects, animals and microorganisms.

### 4. Assays

Yet other methods contemplated herein include screening methods for identifying compounds or agents that modulate sirtuins. An agent may be a nucleic acid, such as an aptamer. Assays may be conducted in a cell based or cell free format. For example, an assay may comprise incubating (or contacting) a sirtuin with a test agent under conditions in which a sirtuin can be modulated by an agent known to modulate the sirtuin, and monitoring or determining the level of modulation of the sirtuin in the presence of the test agent relative to the absence of the test agent. The level of modulation of a sirtuin can be determined by determining its ability to deacetylate a substrate. Exemplary substrates are acetylated peptides which can be obtained from BIOMOL (Plymouth Meeting, PA). Preferred substrates include peptides of p53, such as those comprising an acetylated K382. A particularly preferred substrate is the Fluor de Lys-SIRT1 (BIOMOL), i.e., the acetylated peptide Arg-His-Lys-Lys. Other substrates are peptides from human histones H3 and H4 or an acetylated amino acid. Substrates may be fluorogenic. The sirtuin may be SIRT1 or Sir2 or a portion thereof. For example, recombinant SIRT1 can be obtained from BIOMOL. The reaction may be conducted for about 30 minutes and stopped, e.g., with nicotinamide. The HDAC fluorescent activity assay/drug discovery kit (AK-500, BIOMOL Research Laboratories) may be used to determine the level of acetylation. Similar assays are described in Bitterman et al. (2002) J. Biol. Chem. 277:45099. The level of modulation of the sirtuin in an assay may be compared to the level of modulation of the sirtuin in the presence of one or more (separately or simultaneously) compounds described herein, which may serve as positive or negative controls. Sirtuins for use in the assays may be full length sirtuin proteins or portions thereof. Since it has been shown herein that activating compounds appear to interact with the N-terminus of SIRT1, proteins for use in the assays include N-terminal portions of sirtuins, e.g., about amino acids 1-176 or 1-255 of SIRT1; about amino acids 1-174 or 1-252 of Sir2.

In one embodiment, a screening assay comprises (i) contacting a sirtuin with a test agent and an acetylated substrate under conditions appropriate for the sirtuin to deacetylate the substrate in the absence of the test agent; and (ii) determining the level of acetylation of the substrate, wherein a lower level of acetylation of the substrate in the presence of the test agent relative to the absence of the test agent indicates that the test agent stimulates deacetylation by the sirtuin, whereas a higher level of acetylation of the substrate in the presence of the test agent relative to the absence of the test agent indicates that the test agent inhibits deacetylation by the sirtuin.

Methods for identifying an agent that modulates, e.g., stimulates or inhibits, sirtuins *in vivo* may comprise (i) contacting a cell with a test agent and a substrate that is capable of entering a cell in the presence of an inhibitor of class I and class II HDACs under conditions appropriate for the sirtuin to deacetylate the substrate in the absence of the test agent; and (ii) determining the level of acetylation of the substrate, wherein a lower level of acetylation of the substrate in the presence of the test agent relative to the absence of the test agent indicates that the test agent stimulates deacetylation by the sirtuin, whereas a higher level of acetylation of the substrate in the presence of the test agent relative to the absence of the test agent indicates that the test agent inhibits deacetylation by the sirtuin. A preferred substrate is an acetylated peptide, which is also preferably fluorogenic, as further described herein. The method may further comprise lysing the cells to determine the level of acetylation of the substrate. Substrates may be added to cells at a concentration ranging from about 1µM to about 10mM, preferably from about 10µM to 1mM, even more preferably from about 100µM to 1mM, such as about 200µM. A preferred substrate is an acetylated lysine, e.g., ε-acetyl lysine (Fluor de Lys, FdL) or Fluor de Lys-SIRT1. A preferred inhibitor of class I and class II HDACs is trichostatin A (TSA), which may be used at concentrations ranging from about 0.01 to 100µM, preferably from about 0.1 to 10µM, such as 1µM. Incubation of cells with the test compound and the substrate may be conducted for about 10 minutes to 5 hours, preferably for about 1-3 hours. Since TSA inhibits all class I and class II HDACs, and that certain substrates, e.g., Fluor de Lys, is a poor substrate for SIRT2 and even less a substrate for SIRT3-7, such an assay may be used to identify modulators of SIRT1 *in vivo.*

### 5. Pharmaceutical Compositions

The sirtuin-modulating compounds described herein may be formulated in a conventional manner using one or more physiologically acceptable carriers or excipients. For example, sirtuin-modulating compounds and their physiologically acceptable salts and solvates may be formulated for administration by, for example, injection, inhalation or insufflation (either through the mouth or the nose) or oral, buccal, sublingual, transdermal, nasal, parenteral or rectal administration. In one embodiment, a sirtuin-modulating compound may be administered locally, at the site where the target cells are present, i.e., in a specific tissue, organ, or fluid (e.g., blood, cerebrospinal fluid, etc.).

Sirtuin-modulating compounds can be formulated for a variety of modes of administration, including systemic and topical or localized administration. Techniques and formulations generally may be found in Remington's Pharmaceutical Sciences, Meade Publishing Co., Easton, PA. For parenteral administration, injection is preferred, including intramuscular, intravenous, intraperitoneal, and subcutaneous. For injection, the compounds can be formulated in liquid solutions, preferably in physiologically compatible buffers such as Hank's solution or Ringer's solution. In addition, the compounds may be formulated in solid form and redissolved or suspended immediately prior to use. Lyophilized forms are also included.

For oral administration, the pharmaceutical compositions may take the form of, for example, tablets, lozanges, or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., ationd oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate. Preparations for oral administration may be suitably formulated to give controlled release of the active compound.

For administration by inhalation (e.g., pulmonary delivery), sirtuin-modulating compounds may be conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g., gelatin, for use in an inhaler or insufflator may be formulated containing a powder mix of the compound and a suitable powder base such as lactose or starch.

Sirtuin-modulating compounds may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

Sirtuin-modulating compounds may also be formulated in rectal compositions such as suppositories or retention enemas, e.g., containing conventional suppository bases such as cocoa butter or other glycerides.

In addition to the formulations described previously, sirtuin-modulating compounds may also be formulated as a depot preparation. Such long acting formulations may be administered by implantation (for example subcutaneously or intramuscularly) or by intramuscular injection. Thus, for example, sirtuin-modulating compounds may be formulated with suitable polymeric or hydrophobic materials (for example as an emulsion in an acceptable oil) or ion exchange resins, or as sparingly soluble derivatives, for example, as a sparingly soluble salt.

Pharmaceutical compositions (including cosmetic preparations) may comprise from about 0.00001 to 100% such as from 0.001 to 10% or from 0.1% to 5% by weight of one or more sirtuin-modulating compounds described herein.

In one embodiment, a sirtuin-modulating compound described herein, is incorporated into a topical formulation containing a topical carrier that is generally suited to topical drug administration and comprising any such material known in the art. The topical carrier may be selected so as to provide the composition in the desired form, e.g., as an ointment, lotion, cream, microemulsion, gel, oil, solution, or the like, and may be comprised of a material of either naturally occurring or synthetic origin. It is preferable that the selected carrier not adversely affect the active agent or other components of the topical formulation. Examples of suitable topical carriers for use herein include water, alcohols and other nontoxic organic solvents, glycerin, mineral oil, silicone, petroleum jelly, lanolin, fatty acids, vegetable oils, parabens, waxes, and the like.

Formulations may be colorless, odorless ointments, lotions, creams, microemulsions and gels.

Sirtuin-modulating compounds may be incorporated into ointments, which generally are semisolid preparations which are typically based on petrolatum or other petroleum derivatives. The specific ointment base to be used, as will be appreciated by those skilled in the art, is one that will provide for optimum drug delivery, and, preferably, will provide for other desired characteristics as well, e.g., emolliency or the like. As with other carriers or vehicles, an ointment base should be inert, stable, nonirritating and nonsensitizing. As explained in Remington's (*supra*) ointment bases may be grouped in four classes: oleaginous bases; emulsifiable bases; emulsion bases; and water-soluble bases. Oleaginous ointment bases include, for example, vegetable oils, fats obtained from animals, and semisolid hydrocarbons obtained from petroleum. Emulsifiable ointment bases, also known as absorbent ointment bases, contain little or no water and include, for example, hydroxystearin sulfate, anhydrous lanolin and hydrophilic petrolatum. Emulsion ointment bases are either water-in-oil (W/O) emulsions or oil-in-water (O/W) emulsions, and include, for example, cetyl alcohol, glyceryl monostearate, lanolin and stearic acid. Exemplary water-soluble ointment bases are prepared from polyethylene glycols (PEGs) of varying molecular weight; again, reference may be had to Remington's, *supra,* for further information.

Sirtuin-modulating compounds may be incorporated into lotions, which generally are preparations to be applied to the skin surface without friction, and are typically liquid or semiliquid preparations in which solid particles, including the active agent, are present in a water or alcohol base. Lotions are usually suspensions of solids, and may comprise a liquid oily emulsion of the oil-in-water type. Lotions are preferred formulations for treating large body areas, because of the ease of applying a more fluid composition. It is generally necessary that the insoluble matter in a lotion be finely divided. Lotions will typically contain suspending agents to produce better dispersions as well as compounds useful for localizing and holding the active agent in contact with the skin, e.g., methylcellulose, sodium carboxymethylcellulose, or the like. An exemplary lotion formulation for use in conjunction with the present method contains propylene glycol mixed with a hydrophilic petrolatum such as that which may be obtained under the trademark Aquaphor^{RTM} from Beiersdorf, Inc. (Norwalk, Conn.).

Sirtuin-modulating compounds may be incorporated into creams, which generally are viscous liquid or semisolid emulsions, either oil-in-water or water-in-oil. Cream bases are water-washable, and contain an oil phase, an emulsifier and an aqueous phase. The oil phase is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation, as explained in Remington's, *supra,* is generally a nonionic, anionic, cationic or amphoteric surfactant.

Sirtuin-modulating compounds may be incorporated into microemulsions, which generally are thermodynamically stable, isotropically clear dispersions of two immiscible liquids, such as oil and water, stabilized by an interfacial film of surfactant molecules (Encyclopedia of Pharmaceutical Technology (New York: Marcel Dekker, 1992), volume 9). For the preparation of microemulsions, surfactant (emulsifier), co-surfactant (co-emulsifier), an oil phase and a water phase are necessary. Suitable surfactants include any surfactants that are useful in the preparation of emulsions, e.g., emulsifiers that are typically used in the preparation of creams. The co-surfactant (or "co-emulsifer") is generally selected from the group of polyglycerol derivatives, glycerol derivatives and fatty alcohols. Preferred emulsifier/co-emulsifier combinations are generally although not necessarily selected from the group consisting of: glyceryl monostearate and polyoxyethylene stearate; polyethylene glycol and ethylene glycol palmitostearate; and caprilic and capric triglycerides and oleoyl macrogolglycerides. The water phase includes not only water but also, typically, buffers, glucose, propylene glycol, polyethylene glycols, preferably lower molecular weight polyethylene glycols (e.g., PEG 300 and PEG 400), and/or glycerol, and the like, while the oil phase will generally comprise, for example, fatty acid esters, modified vegetable oils, silicone oils, mixtures of mono-di- and triglycerides, mono- and di-esters of PEG (e.g., oleoyl macrogol glycerides), etc.

Sirtuin-modulating compounds may be incorporated into gel formulations, which generally are semisolid systems consisting of either suspensions made up of small inorganic particles (two-phase systems) or large organic molecules distributed substantially uniformly throughout a carrier liquid (single phase gels). Single phase gels can be made, for example, by combining the active agent, a carrier liquid and a suitable gelling agent such as tragacanth (at 2 to 5%), sodium alginate (at 2-10%), gelatin (at 2-15%), methylcellulose (at 3-5%), sodium carboxymethylcellulose (at 2-5%), carbomer (at 0.3-5%) or polyvinyl alcohol (at 10-20%) together and mixing until a characteristic semisolid product is produced. Other suitable gelling agents include methylhydroxycellulo se, polyoxyethylene-polyoxypropylene, hydroxyethylcellulose and gelatin. Although gels commonly employ aqueous carrier liquid, alcohols and oils can be used as the carrier liquid as well.

Various additives, known to those skilled in the art, may be included in formulations, e.g., topical formulations. Examples of additives include, but are not limited to, solubilizers, skin permeation enhancers, opacifiers, preservatives (e.g., anti-oxidants), gelling agents, buffering agents, surfactants (particularly nonionic and amphoteric surfactants), emulsifiers, emollients, thickening agents, stabilizers, humectants, colorants, fragrance, and the like. Inclusion of solubilizers and/or skin permeation enhancers is particularly preferred, along with emulsifiers, emollients and preservatives. An optimum topical formulation comprises approximately: 2 wt. % to 60 wt. %, preferably 2 wt. % to 50 wt. %, solubilizer and/or skin permeation enhancer; 2 wt. % to 50 wt. %, preferably 2 wt. % to 20 wt. %, emulsifiers; 2 wt. % to 20 wt. % emollient; and 0.01 to 0.2 wt. % preservative, with the active agent and carrier (e.g., water) making of the remainder of the formulation.

A skin permeation enhancer serves to facilitate passage of therapeutic levels of active agent to pass through a reasonably sized area of unbroken skin. Suitable enhancers are well known in the art and include, for example: lower alkanols such as methanol ethanol and 2-propanol; alkyl methyl sulfoxides such as dimethylsulfoxide (DMSO), decylmethylsulfoxide (C₁₀ MSO) and tetradecylmethyl sulfboxide; pyrrolidones such as 2-pyrrolidone, N-methyl-2-pyrrolidone and N-(-hydroxyethyl)pyrrolidone; urea; N,N-diethyl-m-toluamide; C₂ -C₆ alkanediols; miscellaneous solvents such as dimethyl formamide (DMF), N,N-dimethylacetamide (DMA) and tetrahydrofurfuryl alcohol; and the 1-substituted azacycloheptan-2-ones, particularly 1-n-dodecylcyclazacycloheptan-2-one (laurocapram; available under the trademark Azone^{RTM} from Whitby Research Incorporated, Richmond, Va.).

Examples of solubilizers include, but are not limited to, the following: hydrophilic ethers such as diethylene glycol monoethyl ether (ethoxydiglycol, available commercially as Transcutol^{RTM}) and diethylene glycol monoethyl ether oleate (available commercially as Softcutol^{RTM}); polyethylene castor oil derivatives such as polyoxy 35 castor oil, polyoxy 40 hydrogenated castor oil, etc.; polyethylene glycol, particularly lower molecular weight polyethylene glycols such as PEG 300 and PEG 400, and polyethylene glycol derivatives such as PEG-8 caprylic/capric glycerides (available commercially as Labrasol^{RTM}); alkyl methyl sulfoxides such as DMSO; pyrrolidones such as 2-pyrrolidone and N-methyl-2-pyrrolidone; and DMA. Many solubilizers can also act as absorption enhancers. A single solubilizer may be incorporated into the formulation, or a mixture of solubilizers may be incorporated therein.

Suitable emulsifiers and co-emulsifiers include, without limitation, those emulsifiers and co-emulsifiers described with respect to microemulsion formulations. Emollients include, for example, propylene glycol, glycerol, isopropyl myristate, polypropylene glycol-2 (PPG-2) myristyl ether propionate, and the like.

Other active agents may also be included in formulations, e.g., other anti-inflammatory agents, analgesics, antimicrobial agents, antifungal agents, antibiotics, vitamins, antioxidants, and sunblock agents commonly found in sunscreen formulations including, but not limited to, anthranilates, benzophenones (particularly benzophenone-3), camphor derivatives, cinnamates (e.g., octyl methoxycinnamate), dibenzoyl methanes (e.g., butyl methoxydibenzoyl methane), p-aminobenzoic acid (PABA) and derivatives thereof, and salicylates (e.g., octyl salicylate).

In certain topical formulations, the active agent is present in an amount in the range of approximately 0.25 wt. % to 75 wt. % of the formulation, preferably in the range of approximately 0.25 wt. % to 30 wt. % of the formulation, more preferably in the range of approximately 0.5 wt. % to 15 wt. % of the formulation, and most preferably in the range of approximately 1.0 wt. % to 10 wt. % of the formulation.

Topical skin treatment compositions can be packaged in a suitable container to suit its viscosity and intended use by the consumer. For example, a lotion or cream can be packaged in a bottle or a roll-ball applicator, or a propellant-driven aerosol device or a container fitted with a pump suitable for finger operation. When the composition is a cream, it can simply be stored in a non-deformable bottle or squeeze container, such as a tube or a lidded jar. The composition may also be included in capsules such as those described in U.S. Pat. No. 5,063,507. Accordingly, also provided are closed containers containing a cosmetically acceptable composition as herein defined.

In an alternative embodiment, a pharmaceutical formulation is provided for oral or parenteral administration, in which case the formulation may comprises a modulating compound-containing microemulsion as described above, but may contain alternative pharmaceutically acceptable carriers, vehicles, additives, etc. particularly suited to oral or parenteral drug administration. Alternatively, a modulating compound-containing microemulsion may be administered orally or parenterally substantially as described above, without modification.

Phospholipids complexes, e.g., resveratrol-phospholipid complexes, and their preparation are described in U.S. Patent Application Publication No. 2004/116386. Methods for stabilizing active components using polyol/polymer microcapsules, and their preparation are described in US20040108608. Processes for dissolving lipophilic compounds in aqueous solution with amphiphilic block copolymers are described in WO 04/035013.

Conditions of the eye can be treated or prevented by, e.g., systemic, topical, intraocular injection of a sirtuin-modulating compound, or by insertion of a sustained release device that releases a sirtuin-modulating compound. A sirtuin-modulating compound that increases or decreases the level and/or activity of a sirtuin protein may be delivered in a pharmaceutically acceptable ophthalmic vehicle, such that the compound is maintained in contact with the ocular surface for a sufficient time period to allow the compound to penetrate the corneal and internal regions of the eye, as for example the anterior chamber, posterior chamber, vitreous body, aqueous humor, vitreous humor, cornea, iris/ciliary, lens, choroid/retina and sclera. The pharmaceutically-acceptable ophthalmic vehicle may, for example, be an ointment, vegetable oil or an encapsulating material. Alternatively, the compounds of the invention may be injected directly into the vitreous and aqueous humour. In a further alternative, the compounds may be administered systemically, such as by intravenous infusion or injection, for treatment of the eye.

Sirtuin-modulating compounds described herein may be stored in oxygen free environment according to methods in the art. For example, resveratrol or analog thereof can be prepared in an airtight capsule for oral administration, such as Capsugel from Pfizer, Inc.

Cells, e.g., treated *ex vivo* with a sirtuin-modulating compound, can be administered according to methods for administering a graft to a subject, which may be accompanied, e.g., by administration of an immunosuppressant drug, e.g., cyclosporin A. For general principles in medicinal formulation, the reader is referred to Cell Therapy: Stem Cell Transplantation, Gene Therapy, and Cellular Immunotherapy, by G. Morstyn & W. Sheridan eds, Cambridge University Press, 1996; and Hematopoietic Stem Cell Therapy, E. D. Ball, J. Lister & P. Law, Churchill Livingstone, 2000.

Toxicity and therapeutic efficacy of sirtuin-modulating compounds can be determined by standard pharmaceutical procedures in cell cultures or experimental animals. The LD₅₀ is the dose lethal to 50% of the population. The ED₅₀ is the dose therapeutically effective in 50% of the population. The dose ratio between toxic and therapeutic effects (LD₅₀/ED₅₀) is the therapeutic index. Sirtuin-modulating compounds that exhibit large therapeutic indexes are preferred. While sirtuin-modulating compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds may lie within a range of circulating concentrations that include the ED₅₀ with little or no toxicity. The dosage may vary within this range depending upon the dosage form employed and the route of administration utilized. For any compound, the therapeutically effective dose can be estimated initially from cell culture assays. A dose may be formulated in animal models to achieve a circulating plasma concentration range that includes the IC₅₀ (i.e., the concentration of the test compound that achieves a half-maximal inhibition of symptoms) as determined in cell culture. Such information can be used to more accurately determine useful doses in humans. Levels in plasma may be measured, for example, by high performance liquid chromatography.

### 6. Kits

Also provided herein are kits, e.g., kits for therapeutic purposes or kits for modulating the lifespan of cells or modulating apoptosis. A kit may comprise one or more sirtuin-modulating compounds, e.g., in premeasured doses. A kit may optionally comprise devices for contacting cells with the compounds and instructions for use. Devices include syringes, stents and other devices for introducing a sirtuin-modulating compound into a subject (e.g., the blood vessel of a subject) or applying it to the skin of a subject.

The practice of the present methods will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press: 1989); DNA Cloning, Volumes I and II (D. N. Glover ed., 1985); Oligonucleotide Synthesis (M. J. Gait ed., 1984); Mullis et al. U.S. Patent No: 4,683,195; Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Manipulating the Mouse Embryo, (Cold Spring Harbor Laboratory Press, Cold Spring Harbor, N.Y., 1986).

### EXEMPLIFICATION

The invention now being generally described, it will be more readily understood by reference to the following examples which are included merely for purposes of illustration of certain aspects and embodiments of the present invention, and are not intended to limit the invention in any way.

### EXAMPLE 1: Mammalian cell based assay:

As described herein, nicotinamide riboside and its analogs may directly or indirectly modulate sirtuins, such as human SIRT1. This may include analogs of nicotinamide riboside, particularly compounds that are metabolized, hydrolyzed or otherwise converted to nicotinamide riboside *in vivo.* The ability of such compounds, or products thereof, to modulate sirtuin activity may be examined using the cell based assay described below.

A human embryonic kidney cell line that stably expresses the beta-lactamase gene under the regulation of an NFκB response element (NFκB-bla HEK 293T CellSensor Cell Line, Invitrogen Corp., CA) responds to stimulation with Tumor Necrosis Factor-alpha (TNFa) leading to activation of the NFκB signaling pathway and subsequent beta-lactamase expression. Expression of beta-lactamase is quantified using a fluorescence resonance energy transfer (FRET)-based substrate (LiveBLAzer-FRET B/G Substrate, Invitrogen Inc., CA). The substrate is a lipophilic, esterified compound that readily enters the reporter cell line. Upon cleavage by endogenous cytoplasmic esterases, the substrate is converted into a negatively charged substrate that is retained in the cytosol. Beta-lactamase cleavage spatially separates the two chromophors of the substrate disrupting FRET and producing a blue fluorescence signal at 450 nm (upon excitation at 409 nm). In the absence of beta-lactamase cleavage, the substrate produces a green fluourescence signal at 520 nm (upon excitation at 409 nm). The ratio of blue to green fluorescence increases with increasing beta-lactamase activity.

NFκB gene expression regulatory activity is modulated by SIRT1 (Yeung et al., EMBO J., 23(12):2369, 2004). SIRT1 deacetylates the RelA/p65 subunit of NκKB at lysine 310 thereby inhibiting NFkB stimulated transcription. SIRT1 activation (e.g., by resveratrol) inhibits endogenous NFκB activation by TNFa thereby decreasing beta-lactamase expression in the reporter cell line. Conversley, SIRT1 inhibition (e.g., by sirtinol) may lead to an even higher level of endogenous NFκB activation by TNFa and subsequent increased beta-lactamase expression in the reporter cell line. The degree of sirtuin modulation in the presence of a test compound may be determined by monitoring a change in the ratio of blue to green fluorescence signal produced by the FRET substrate, e.g., a decrease in the ratio of blue to green fluorescence signal is indicative of a sirtuin activator and an increase in the ratio blue to green fluorescence signal is indicative of a sirtuin inhibitor. The degree of sirtuin activity in the presence of a test compound may be compared to the level of sirtuin activity in a control (e.g., in the absence of a test compound or in the presence of a compound having known activity).

### EXAMPLE 2: Yeast based assay:

A yeast *Saccharomyces cerevisiae,* haploid strain that contains a chromosomal deletion of the *qns1* gene has been shown to be dependent on exogenous nicotinamide riboside as its only source of de novo NAD biosynthesis (Bieganowski and Brenner, 2004). This strain can therefore be used to screen for nicotinamide riboside analogs that are capable of supporting growth of this yeast strain. A yeast haploid strain containing a *qns1* deletion is transformed with a single plasmid containing both a wild type *QNS1* gene and a *URA3* gene. Methods for transforming *S. cerevisiae,* are commonly known to those skilled in the art. The plasmid *QNS1* gene allows for growth of the haploid yeast strain in the absence of nicotinamide riboside, or analogs thereof. Yeast cells are grown at 30°C and plated on media containing 5-fluoroorotic acid (1 mg/ml), which selects against the *URA3* plasmid and therefore leads to the loss of the functional *QNS1* gene. Consequently, the growth of the *qns1* haploid yeast is dependent on exogenous nicotinamide riboside, or analogs thereof. For example, media containing 1 mg/ml 5-fluoroorotic acid may be supplemented with about 10 µM of nicotinamide riboside. Alternatively, nicotinamide riboside analogs, particularly compounds that are metabolized, hydrolyzed or otherwise converted to nicotinamide riboside in vivo, may allow the growth of *qns1* yeast in the presence of 5-fluoroorotic acid.

### EXAMPLE 3: Nicotinamide riboside kinase assay:

Nicotinamide riboside and analogs thereof, particularly compounds that are metabolized, hydrolyzed or otherwise converted to nicotinamide riboside in vivo, may be a substrate for nicotinamide riboside kinase, NRK, upon entering a target cell. An assay for the human NRK enzyme has been described (Sasiak et al., 1996). Human NRK is purified from human placentas as described. Reaction mixtures containing NRK consist of 50 mM Tris-HCl (pH 7.8), 5mM MgCl₂, 1 mM dithiothreitol, 8mM ATP (pH 7), and 10 to 100 µM ³H-labeled nicotinamide riboside, or an analog thereof. 0.4 mg/ml bovine serum albumin may also be added to the reaction mixture. The reaction mixture is incubated for 30-60 minutes at 37°C after which aliquots are removed and applied to Whatman DE81 filter paper disks. The reaction product of NRK may be separated from the remaining ³H-labeled nicotinamide riboside or substrate analog by thin-layer chromatography. This product can be quantified by phosphoimager analysis. To distinguish NRK activity from other non-specific phosphorylating activities, unlabeled nicotinamide riboside or substrate analog is added to a duplicate reaction mixture at approximately 100 µM, which results in inhibition of phosphorylation by NRK but not by other kinases. The product of this reaction is used to approximate non-specific activity in the reaction.

### EXAMPLE 4: Nicotinamide mononucleotide adenylyl transferase assay:

Nicotinamide riboside kinase (NRK) phosphorylates nicotinamide to yield nicotinamide mononucleotide (NMN). NMN is used as a substrate for nicotinamide mononucleotide adenyl transferase (NMNAT) in de novo NAD biosynthesis. As described in Example 4, NRK may also phosphorylate nicotinamide riboside analogs, particularly those that are metabolized, hydrolyzed or otherwise converted to nicotinamide riboside in vivo. NMNAT may utilize these products as a substrate to produce NAD or an analog thereof. An assay for the human NMNAT enzyme has been described (Schweiger et al., 2001). NMNAT is purified from human placentas as described in Magni et al., 1997.

Activity of NMNAT is measured by monitoring the increase in absorbance at 340 nm caused by the reduction of NAD to NADH, or analogs thereof. The reaction is performed in 16 mM semicarbacide-HCl, 0.625% (v/v) ethanol, 30 mM HEPES buffer (pH 7.4), 12.25 mM MgCl₂, 1.17 mM ATP, and 15 U alcohol dehydrogenase (Sigma). The reaction is started by adding the phosphorylated nicotinamide riboside or an analog thereof to a final concentration of 0.625 mM.

### EXAMPLE 5: Coupled enzyme assay reaction of NRK and NMNAT:

A single assay that combines NRK and NMNAT may allow the conversion of nicotinamide riboside or analogs thereof, particularly compounds that are metabolized, hydrolyzed or otherwise converted to nicotinamide riboside in vivo, to be converted in two steps to NAD or an analog of NAD. These products may directly or indirectly activate sirtuins, such as human SIRT1, in a target cell. The reaction can be evaluated by monitoring NMNAT activity by monitoring the increase in absorbance at 340 nm caused by the reduction of NAD to NADH, or analogs thereof.

### EXAMPLE 6: Nicotinamide Riboside is Neuroprotective for Retinal Ganglion Cells During Acute Optic Neuritis

### Background

Optic neuritis is an inflammatory disorder of the optic nerve that is commonly associated with the central nervous system autoimmune-mediated demyelinating disease multiple sclerosis (MS). Patients with optic neuritis typically have progressive visual loss over 1-2 weeks, then recover most or all of their vision over several weeks. Over 40% of patients do have some persistent visual changes (decreased acuity, color vision, contrast sensitivity or visual field), and patients with repeated episodes of optic neuritis have increased likelihood of permanent visual loss. Recent studies have suggested that neuronal damage in lesions of MS and optic neuritis are responsible for permanent dysfunction.

Experimental autoimmune encephalomyelitis (EAE) is an animal model of MS induced by immunization with Proteolipid Protein (PLP). Animals mount an immune response resulting in inflammation, demyelination, and neuronal damage in the brain, spinal cord, and optic nerve, similar to MS patients. Optic neuritis induced in EAE mice leads to loss of retinal ganglion cells (RGCs), neurons whose axons form the optic nerve.

### Preliminary Studies

Techniques for labeling RGCs and for histological determination of optic neuritis have been refined for use in SJL/J mice with EAE induced by proteolipid protein peptide (PLP). A detailed evaluation of the time course of RGC loss in optic neuritis has been performed and are described below.

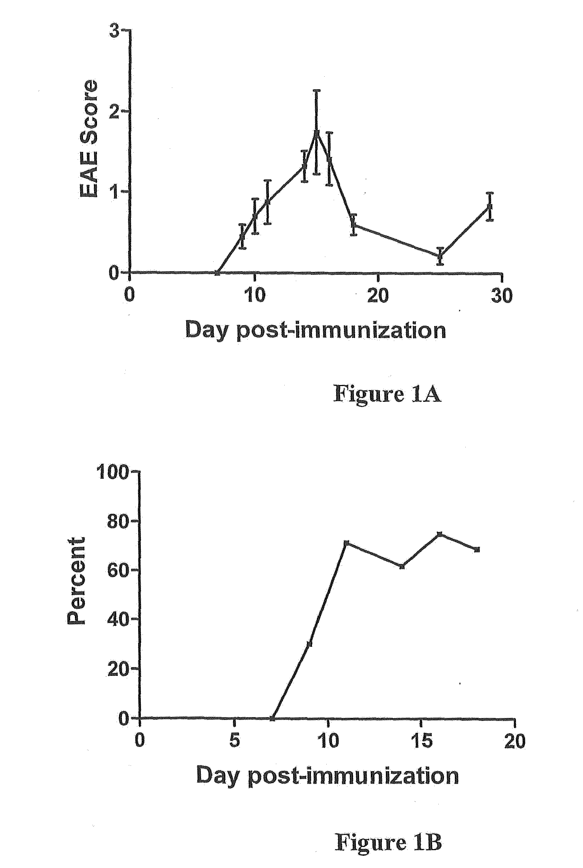

*PLP induces a relapsing*/*remitting course of EAE in SJL*/*J mice:* Mice were immunized with PLP by subcutaneous injection and observed daily for clinical signs of EAE. Results demonstrate that mice develop EAE clinical symptoms as early as day 9 after immunization and clinical symptoms peak by day 14-15 (FIG. 1A). Clinical EAE score then declines until day 25 when a second relapse of symptoms begins.

*A high incidence of optic neuritis is detected in EAE mice:* SJL/J mice immunized with PLP were sacrificed at various time points. Optic nerves were isolated, fixed, embedded in paraffin, cut and stained with hematoxylin and eosin. Optic neuritis (presence of inflammatory cell infiltrates) is detected by day 9 after immunization and reaches peak incidence of over 70% of optic nerves by day 11 (FIG. 1B).

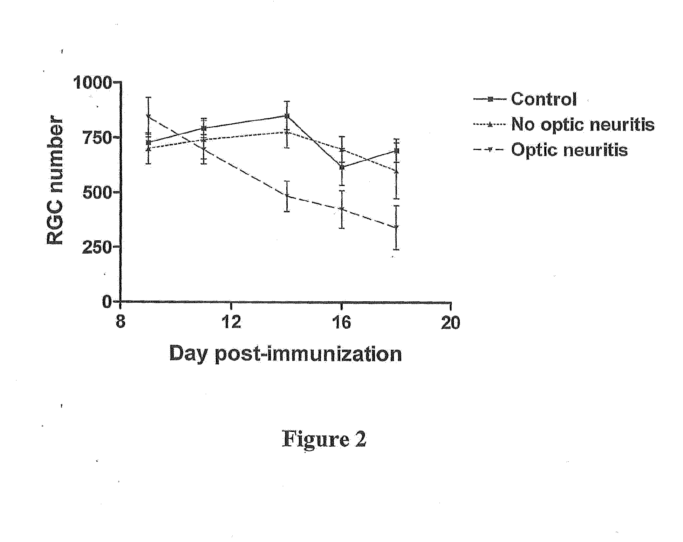

*Inflammation precedes RGC loss in eyes with optic neuritis:* RGCs were retrogradely labeled with Fluorgold (FG) by stereotactic injection into superior colliculi prior to induction of EAE. Mice were sacrificed at various times points and retinas and optic nerves were isolated. Retinas were whole mounted on glass slides and RGC numbers were counted by fluorescent microscopy. In eyes with optic neuritis, no loss of RGCs is detected at day 9 or 11 after immunization as compared to control eyes or eyes from EAE mice that did not develop optic neuritis (FIG. 2). Significant loss of RGCs is detected by day 14 (43% decrease vs. control) and progresses through day 18 (52% decrease vs. control).

*Study outline:* The neuroprotective effects of nicotinamide riboside were examined in EAE mice with optic neuritis. 6-8 week old SJL/J mice were labeled with 2.5 µl of 1.25% FG solution injected into the superior colliculi. To induce EAE, mice were immunized several days later with 300 µg PLP emulsified in complete Freund's adjuvant (CFA), and control mice (without EAE) were mock-immunized with phosphate buffered saline (PBS) in CFA. All mice received 200 ng intraperitoneal pertussis toxin (PT) on the day of immunization (day 0) and again on day 2.

Eyes were treated with nicotinamide riboside by intravitreal (ivt) injections with a volume of 0.8 µl/injection of a stock solution of either 0.1 M or 0.4 M nicotinamide riboside in PBS (Groups 2, 4 and 5). This results in an estimated final ocular concentration of nicotinamide riboside of 19 mM or 76 mM. Non-drug treatment control mice received either no ivt injections (Group 1), or mock-injections with PBS (Group 3). Treament with nicotinamide riboside, as well as PBS control injections, were given ivt on days 0, 4, 7 and 11. Mice were scored daily for clinical EAE, and were sacrificed on day 14 by overdose with ketamine and xylazine.

Retinas were dissected and whole-mounted for fluorescent microscopy. RGC numbers were quantified by counting FG-labeled cells in 12 standardized fields in each retina. Optic nerves were dissected and processed for histology. Cut sections stained by H & E were evaluated for the presence of inflammatory cells to determine acute optic neuritis. RGCs were compared between PBS-treated and nicotinamide riboside-treated eyes with optic neuritis to determine whether nicotinamide riboside prevents loss of neurons.

*Results:* There was no difference in RGC numbers between control eyes and non-EAE eyes treated with nicotinamide riboside (Groups 1 and 2). Significant RGC loss occurred in PBS treated EAE eyes with optic neuritis (268±59 RGCs; Group 3) vs. controls (691±81; Group 1), p<0.01. RGC loss was reduced by 100mM nicotinamide riboside treatment (505±36; Group 4) and completely blocked by 400mM nicotinamide riboside treatment (710±67; Group 5), p<0.01. Incidence of optic neuritis and clinical EAE did not differ between nicotinamide riboside treated mice and controls.

*Conclusion:* Nicotinamide riboside is neuroprotective for RGCs during acute optic neuritis in EAE in a dose-dependent manner. Nicotinamide riboside is not toxic to RGCs, and does not prevent inflammatory cell infiltration. Sirtuin activation has the potential therapeutic role to prevent neurodegeneration in optic neuritis and MS, and may be useful in conjunction with anti-inflammatory therapy.

### Example 7: Testing of Neuroprotective Effects of Nicotinamide Riboside and Nicotinamide Mononucleotide in a Retinal Ganglion Cell Injury Model:

### Summary:

The following example demonstartes the effect of resveratrol, NMN and nicotinamide riboside on ganglion cell survival in the Swiss white mouse retinas after intravitreal NMDA injection.

### Administration of test compounds:

Stock solutions for administration are nicotinamide riboside (125 mM in water) and NMN (125 mM in water).

### Endpoints

RGC density is determined by immunohistochemistry with brn-3 labeled retinal ganglion cells (RGC). RGCs are counted in 12 standard retinal locations per flat mount.

### Methods

### Test substance administration

On days 0, 2 and 4, 2µl of test substance or vehicle is injected into the intravitreal space of anesthetised (intraperitoneal ketamine, xylazine) to the right eye of all 3-month old adult Swiss white mice (25 to 30g, n=12 per treatment) using a microsyringe driver attached to a micropipette.

### Sham injections:

Vehicle (2µl, n=12) is injected on days 0, 2 and 4 to the right eye of all mice using a microsyringe driver attached to a micropipette. Water (n=4) will be injected days 0, 2 and 4 to the right eye of all mice using a microsyringe driver attached to a micropipette to serve as controls for nicotinamide riboside and NMN.

### RGC injury models

Intravitreal NMDA injection (100nM in 2µl) is administered to the right eye of all mice (test substance or sham injected animals) using a microsyringe driver attached to a micropipette. This injection induces reproducible RGC apoptosis, which peaks between 12 and 24 hours after injection.

### RGC density:

This is quantified from retinal flatmounts created 6 days after NMDA injection. RGCs are identified by anti-brn-3 staining³. RGC density is determined for 12 retinal locations per flat mount (3 per quadrant at set distances from the optic nerve head). To generate flatmounts, mice are perfusion fixed with 4% paraformaldehyde, eyes enucleated and fixed overnight in 4% paraformaldehyde. Retinas are then collected and placed onto subbed slides, labeled and counted.

### Mouse summary for each test substance:

Injections are performed to right eyes only (in accordance with ARVO statements for the use of animals in ophthalmic and vision research).

### Example 8: Stability of Triacetoxy Nicotinamide Riboside, NMN and Nicotinamide Riboside in Rat Plasma:

Duplicate sets of standard curves of triacetoxy nicotinamide riboside, NMN and nicotinamide riboside in rat plasma were extracted by protein precipitation. Due to sensitivities of compounds to the organic solvent used in extraction, two separate extractions were performed on the standards. The standards were extracted with ACN to obtain triacetoxy nicotinamide riboside and nicotinamide riboside standards, and extracted with MeOH to obtain NMN standards. The standards were analyzed through LC/MS.

Due to varying chromatographic properties of the compounds, two separate methods and columns were employed to resolve the compounds. The triacetoxy nicotinamide riboside standards (ACN extraction) were run on an YMC ODS-AQ 2.0 x 100 mm column, while the NMN standards (MeOH extraction) and nicotinamide riboside standards (ACN extraction) were run on an YMC ODS-AQ 4.5 x 150 mm column. The resolution procedures were as follows:
- 50 uL aliquots of each sample were taken in replicates of four.
- Added 200 uL of organic solvent to each sample.
- Vortexed samples and centrifuged at 5000 g's for 10 minutes.
- Transferred supernatant to new tubes (200 uL), and dried down in Speed-Vac.
- Reconstituted samples in 200 uL 95:5 ddH₂O:ACN, and vortexed 10 minutes on platform vortexer at setting 10. Transferred to injection vials for analysis.

The triacetoxy nicotinamide riboside curves in rat plasma showed a variation of response between the first curve and second curve injected. The first curve injected shows a higher response for triacetoxy nicotinamide riboside. Though the curves may be quantitated separately, they cannot be quantitated together. The decrease in triacetoxy nicotinamide riboside between the first and second curve may be due to hydrolysis of the compound over time. Triacetoxy nicotinamide riboside hydrolyzes into nicotinamide riboside when extracted with MeOH, and less strongly so when extracted with ACN.

The NMN curves in rat plasma showed linearity over 1-1000 ng/mL.

The nicotinamide riboside curves in rat plasma showed linearity over 5-1000 ng/mL.

To measure stability of triacetoxy nicotinamide riboside, NMN and nicotinamide riboside in rat plasma over time, the following procedure was used:
- Spiked 10 uL of 10 ug/mL compound working solutions into separate 90 uL aliquots of rat plasma, for a final concentration of 0.1mg/mL. Left samples on benchtop at ambient temperature. Samples prepared in triplicate.

- At the specified timepoints after spiking (0, 15 min, 30 min, 1 hr, 2 hr), added 400 uL of organic solvent to each sample. The nicotinamide riboside and NMN samples received methanol, the triacetoxy nicotinamide riboside samples received acetonitrile. (For the zero time point, the 400 uL of organic solvent was added to the plasma and vortexed prior to the spiking of compound stock solution)
- Vortexed samples and centrifuge.
- Transferred supernatant to new tubes (200 uL), and dried down in Speed-Vac.
- Reconstituted samples in 200 uL 95:5 ddH₂O:ACN, and vortexed. Transferred to injection vials for analysis.

### Results:

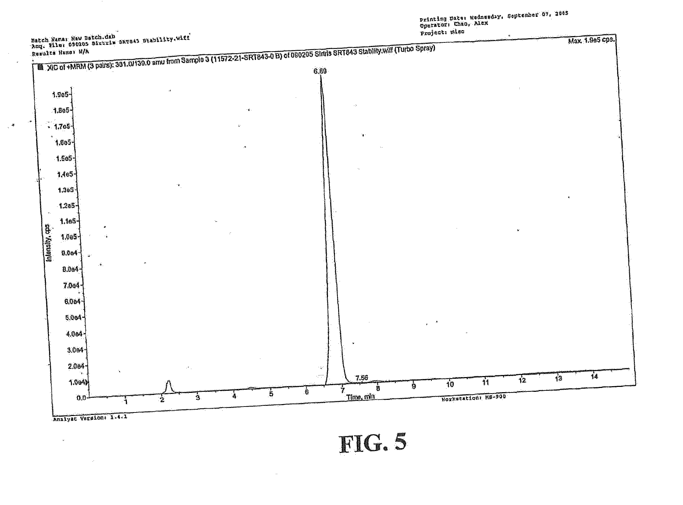

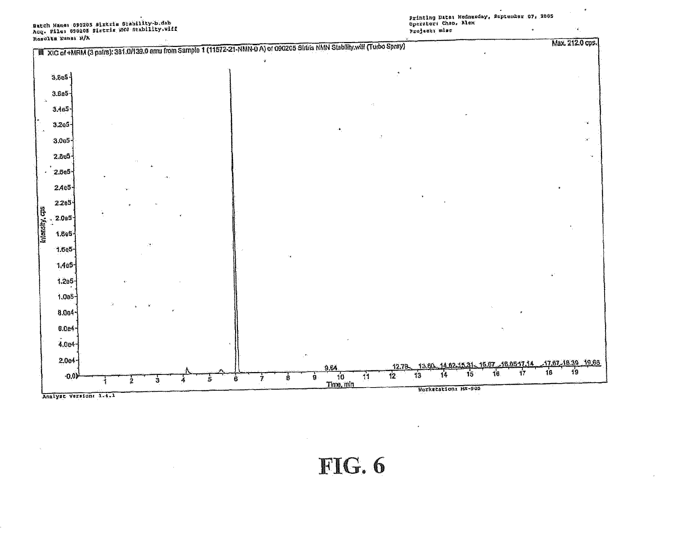

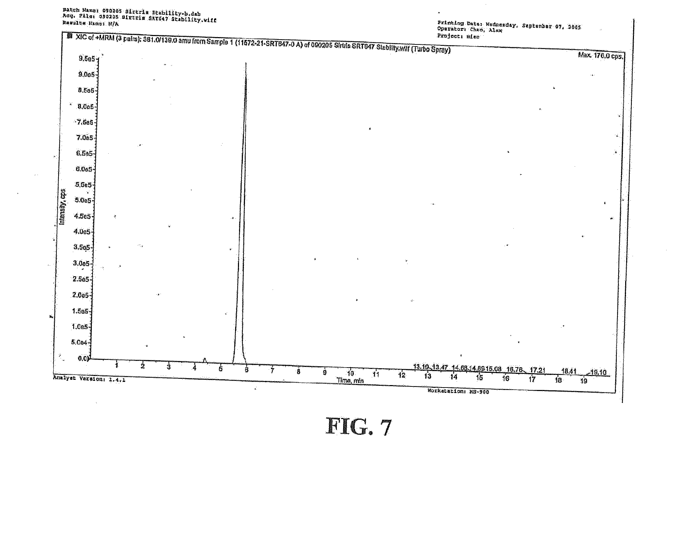

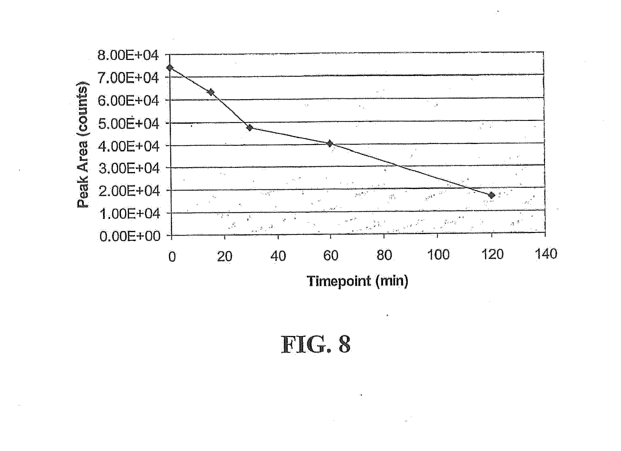

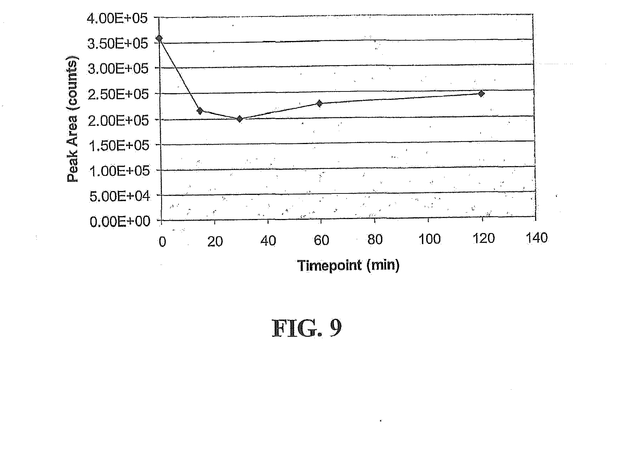

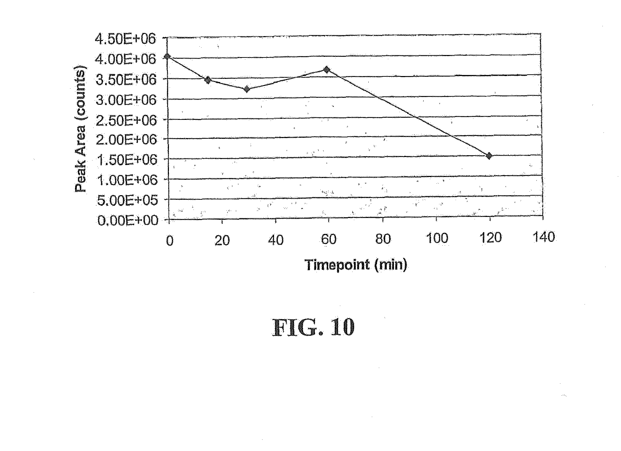

Representative chromatograms of triacetoxy nicotinamide riboside, NMN and nicotinamide riboside following extraction from rat plasma after 0 minutes are shown in FIGS. 5, 6, and 7, respectively. Stability of triacetoxy nicotinamide riboside, NMN and nicotinamide riboside in rat plasma (as determined by peak area) at indicated time points is shown in FIGS. 8, 9 and 10 respectively.

### Example 9: 7 Day Repeat Dose Tolerance Study of NMN in Mice:

Groups of male Swiss Albino mice (6 mice per dosing group) were dosed with either NMN dissolved in phosphate buffered saline (PBS) at 1000 mg/kg, 500 mg/kg, or 300 mg/kg or vehicle control (PBS) via intraperitoneal injection (IP) for 7 consecutive days. The evaluation criteria used to assess toxicity included daily clinical observations and weight gain. None of the animals showed clinical signs of toxicity associated with NMN administration at any point during the study. All animals in the study gained weight by study termination, with the exception of one control animal.

### EQUIVALENTS

The present invention provides among other things sirtuin-activating compounds and methods of use thereof. While specific embodiments of the subject invention have been discussed, the above specification is illustrative and not restrictive. Many variations of the invention will become apparent to those skilled in the art upon review of this specification. The full scope of the invention should be determined by reference to the claims, along with their full scope of equivalents, and the specification, along with such variations.

### INCORPORATION BY REFERENCE

All publications and patents mentioned herein, including those items listed below, are hereby incorporated by reference in their entirety as if each individual publication or patent was specifically and individually indicated to be incorporated by reference. In case of conflict, the present application, including any definitions herein, will control.

Also incorporated by reference in their entirety are any polynucleotide and polypeptide sequences which reference an accession number correlating to an entry in a public database, such as those maintained by The Institute for Genomic Research (TIGR) (www.tigr.org) and/or the National Center for Biotechnology Information (NCBI) (www.ncbi.nlm.nih.gov).

Also incorporated by reference are the following: PCT Publications WO 2005/002672; 2005/002555; and 2004/016726.

The invention can be further defined as in the numbered sentences below:
1. A method for promoting survival of a eukaryotic cell comprising contacting the cell with at least one compound of Structural Formula (I) or (II): 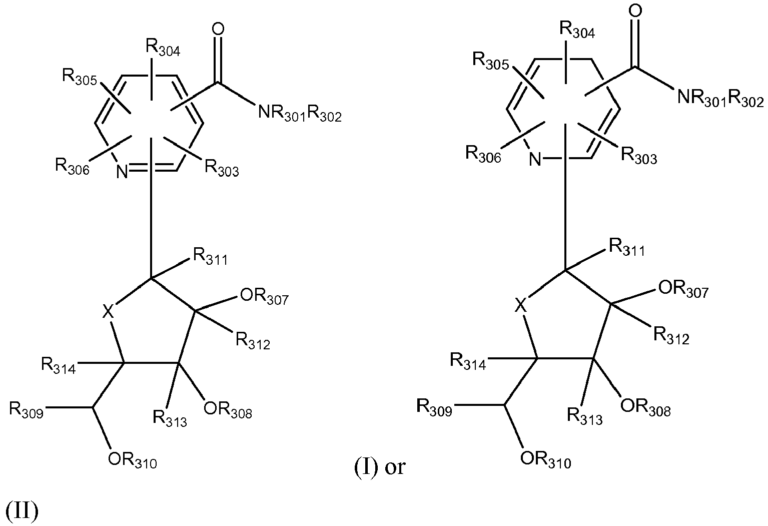 or a pharmaceutically acceptable salt thereof, wherein:
   R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
   R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
2. A method for promoting survival of a eukaryotic cell comprising contacting the cell with at least one compound of Structural Formula (III) or (IV): 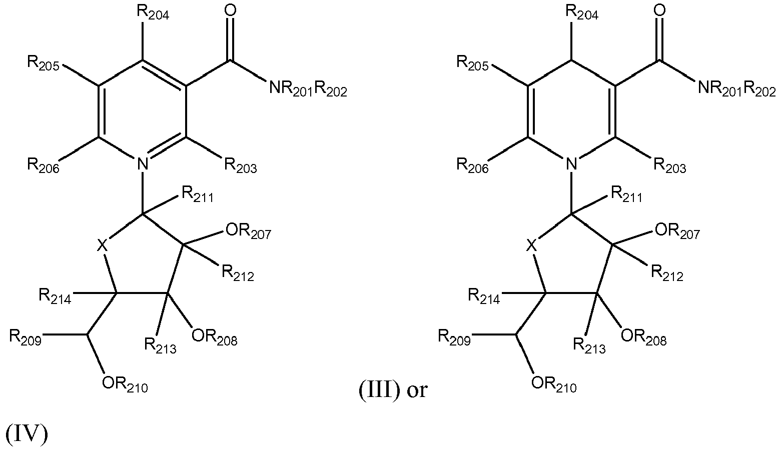 or a pharmaceutically acceptable salt thereof, wherein:
   R₂₀₁ and R₂₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₂₀₁ and R₂₀₂ taken together with the atom to which they are attached form a substituted or unsubstituted non-aromatic heterocyclic group;
   R₂₀₃, R₂₀₄, R₂₀₅ and R₂₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₂₀₇, R₂₀₈ and R₂₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₂₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₂₁₁, R₂₁₂, R₂₁₃ and R₂₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
3. The method of sentence 2, wherein at least one of R_{207,} R₂₀₈ and R₂₁₀ is a substituted or unsubstituted alkyl group, a substituted or unsubstituted aryl group, -C(O)R, -C(O)OR, -C(O)NHR, -C(S)R, -C(S)OR or -C(O)SR.
4. The method of sentence 3, wherein at least one of R₂₀₇, R₂₀₈ and R₂₁₀ is - C(O)R.
5. The method of sentence 2, wherein R₂₀₄ is a halogen or -H.
6. The method of sentence 5, wherein the halogen is -F and -H is -D.
7. The method of sentence 2, wherein said compound increases at least one of the level or activity of a sirtuin protein in the cell.
8. The method of sentence 2, wherein the compound increases the lifespan of the cell.
9. The method of sentence 2, wherein the compound increases the cell's ability to resist stress.
10. The method of sentence 9, wherein the stress is one or more of the following: heatshock, osmotic stress, high energy radiation, chemically-induced stress, DNA damage, inadequate salt level, inadequate nitrogen level, or inadequate nutrient level.
11. The method of sentence 2, wherein the compound mimics the effect of nutrient restriction on the cell.
12. The method of sentence 2, wherein the compound increases deacetylase activity of the sirtuin protein.
13. The method of sentence 2, wherein the sirtuin protein is a mammalian protein.
14. The method of sentence 2, wherein the sirtuin protein is human SIRT1.
15. The method of sentence 2, wherein the eukaryotic cell is a mammalian cell.
16. The method of sentence 2, wherein the compound does not substantially have one or more of the following activities: inhibition of PI3-kinase, inhibition of aldoreductase, inhibition of tyrosine kinase, transactivation of EGFR tyrosine kinase, coronary dilation, or spasmolytic activity, at concentrations of the compound that are effective for increasing the deacetylation activity of the SIRT1 protein.
17. The method of sentence 2, wherein the method does not include the reduction of a prodrug to an active agent by a NAD(P)H quinone reductase.
18. A method for treating or preventing a disease or disorder associated with cell death, cell dysfunction or aging in a subject, comprising administering to a subject in need thereof a therapeutically effective amount of at least one compound of Structural Formula (I) or (II): 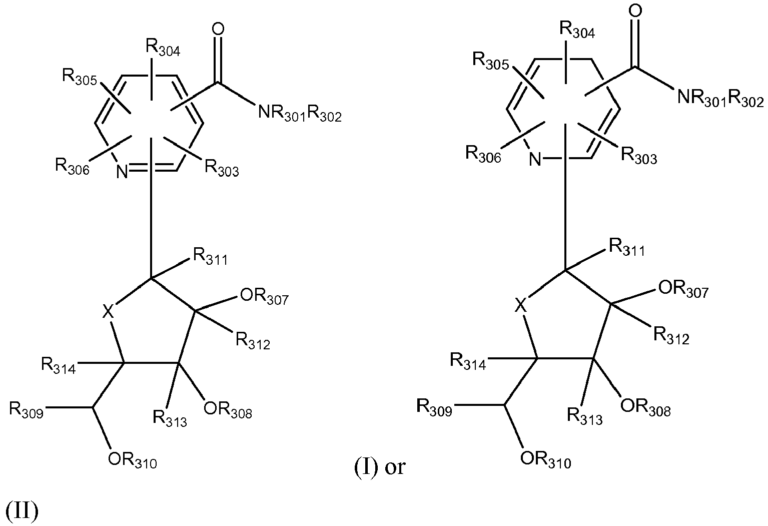 or a pharmaceutically acceptable salt thereof, wherein:
   R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
   R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
19. A method for treating or preventing a disease or disorder associated with cell death, cell dysfunction or aging in a subject, comprising administering to a subject in need thereof a therapeutically effective amount of at least one compound of Structural Formula (III) or (IV): 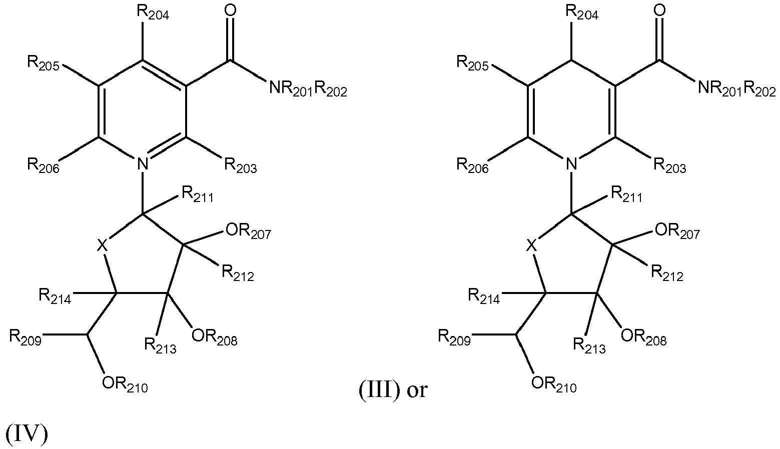 or a pharmaceutically acceptable salt thereof, wherein:
   R₂₀₁ and R₂₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₂₀₁ and R₂₀₂ taken together with the atom to which they are attached form a substituted or unsubstituted non-aromatic heterocyclic group;
   R₂₀₃, R₂₀₄, R₂₀₅ and R₂₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₂₀₇, R₂₀₈ and R₂₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₂₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₂₁₁, R₂₁₂, R₂₁₃ and R₂₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
20. The method of sentence 19, wherein at least one of R₂₀₇, R₂₀₈ and R₂₁₀ is a substituted or unsubstituted alkyl group, a substituted or unsubstituted aryl group, -C(O)R, -C(O)OR, -C(O)NHR, -C(S)R, -C(S)OR or -C(O)SR.
21. The method of sentence 20, wherein at least one of R₂₀₇, R₂₀₈ and R₂₁₀ is - C(O)R.
22. The method of sentence 19, wherein R₂₀₄ is a halogen or -H.
23. The method of sentence 22, wherein the halogen is -F and -H is -D.
24. The method of sentence 19, wherein the method does not include the reduction of a prodrug to an active agent by a NAD(P)H quinone reductase.
25. The method of sentence 19, wherein the subject is not in need of an increase in nitric oxide bioactivity.
26. The method of sentence 19, wherein the aging-related disease is stroke, a cardiovascular disease, arthritis, high blood pressure, or Alzheimer's disease.
27. The method of sentence 19, wherin the disorder associated with cell death is the result of surgery, drug therapy, chemical exposure or radiation exposure.
28. A method for treating or preventing insulin resistance, a metabolic syndrome, hypercholesterolemia, artherogenic dyslipidemia, diabetes, or complications thereof, or for increasing insulin sensitivity in a subject, comprising administering to a subject in need thereof a therapeutically effective amount of at least one compound of Structural Formula (I) or (II): 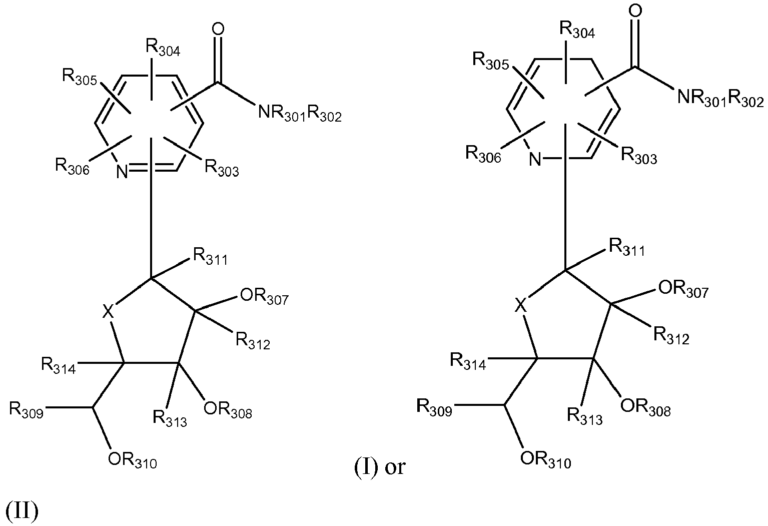 or a pharmaceutically acceptable salt thereof, wherein:
   R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
   R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2,
   provided that when X is O and R₃₀₁-R₃₀₉ and R₃₁₁-R₃₁₄ are -H, R₃₁₀ is not -H.
29. A method for treating or preventing insulin resistance, a metabolic syndrome, artherogenic dyslipidemia, hypercholesterolemia, diabetes, or complications thereof, or for increasing insulin sensitivity in a subject, comprising administering to a subject in need thereof a therapeutically effective amount of at least one compound of Structural Formula (III) or (IV): 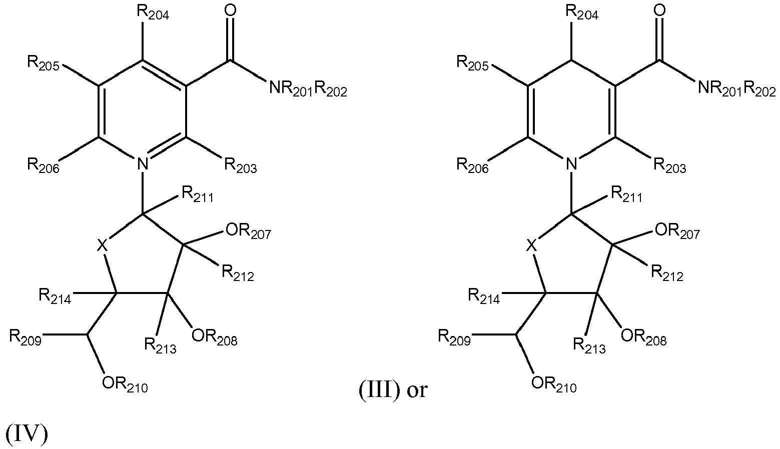 or a pharmaceutically acceptable salt thereof, wherein:
   R₂₀₁ and R₂₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₂₀₁ and R₂₀₂ taken together with the atom to which they are attached form a substituted or unsubstituted non-aromatic heterocyclic group;
   R₂₀₃, R₂₀₄, R₂₀₅ and R₂₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₂₀₇, R₂₀₈ and R₂₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₂₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₂₁₁, R₂₁₂, R₂₁₃ and R₂₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2,
   provided that when X is O and R₂₀₁-R₂₀₉ and R₂₁₁-R₂₁₄ are -H, R₂₁₀ is not -H.
30. A method for reducing the weight of a subject, or preventing weight gain in a subject, comprising administering to a subject in need thereof a therapeutically effective amount of at least one compound of Structural Formula (I) or (II): 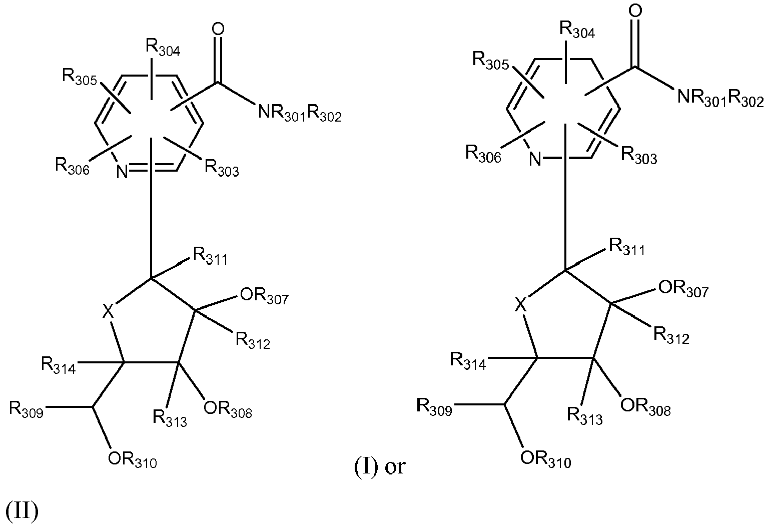 or a pharmaceutically acceptable salt thereof, wherein:
   R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
   R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
31. A method for reducing the weight of a subject, or preventing weight gain in a subject, comprising administering to a subject in need thereof a therapeutically effective amount of at least one compound of Structural Formula (III) or (IV): 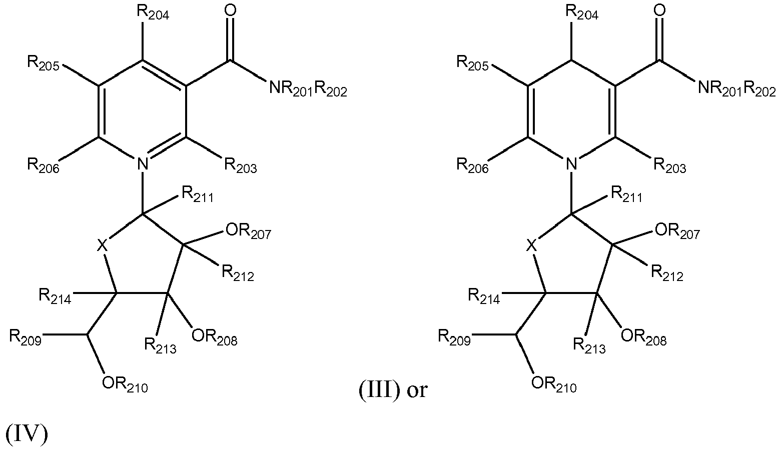 or a pharmaceutically acceptable salt thereof, wherein:
   R₂₀₁ and R₂₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₂₀₁ and R₂₀₂ taken together with the atom to which they are attached form a substituted or unsubstituted non-aromatic heterocyclic group;
   R₂₀₃, R₂₀₄, R₂₀₅ and R₂₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₂₀₇, R₂₀₈ and R₂₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₂₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₂₁₁, R₂₁₂, R₂₁₃ and R₂₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
32. A method for preventing the differentiation of a pre-adipocyte, comprising contacting the pre-adipocyte with at least one compound of Structural Formula (I) or (II) : 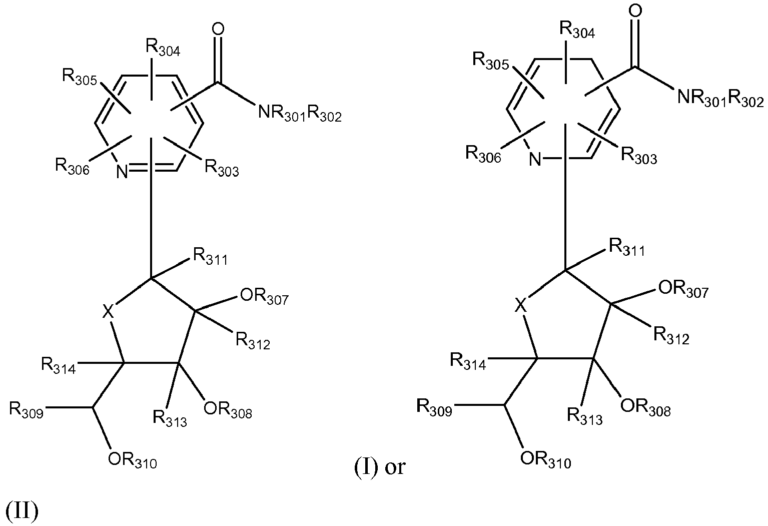 or a pharmaceutically acceptable salt thereof, wherein:
   R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
   R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
33. A method for preventing the differentiation of a pre-adipocyte, comprising contacting the pre-adipocyte with at least one compound of Structural Formula (III) or (IV): 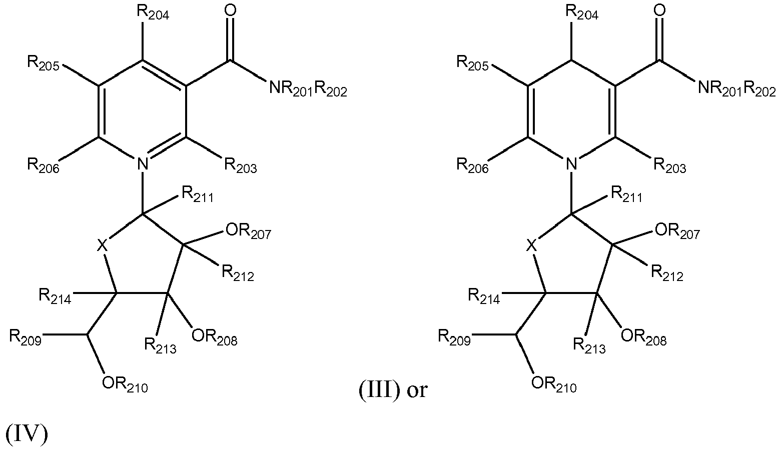 or a pharmaceutically acceptable salt thereof, wherein:
   R₂₀₁ and R₂₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₂₀₁ and R₂₀₂ taken together with the atom to which they are attached form a substituted or unsubstituted non-aromatic heterocyclic group;
   R₂₀₃, R₂₀₄, R₂₀₅ and R₂₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₂₀₇, R₂₀₈ and R₂₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₂₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₂₁₁, R₂₁₂, R₂₁₃ and R₂₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
34. A method for prolonging the lifespan of a subject comprising administering to a subject a therapeutically effective amount of at least one compound of Structural Formula (I) or (II): 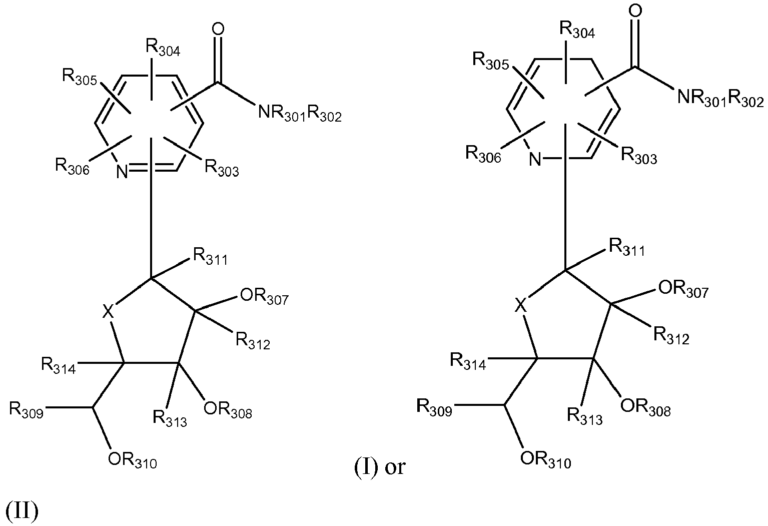 or a pharmaceutically acceptable salt thereof, wherein:
   R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
   R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
35. A method for prolonging the lifespan of a subject comprising administering to a subject a therapeutically effective amount of at least one compound of Structural Formula (III) or (IV): 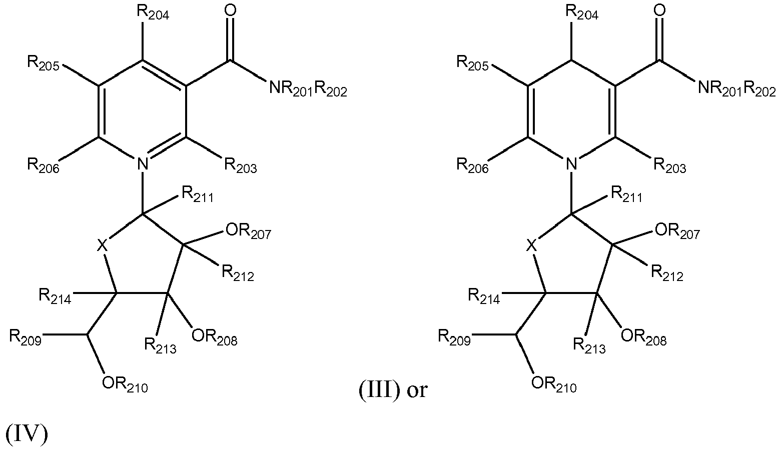 or a pharmaceutically acceptable salt thereof, wherein:
   R₂₀₁ and R₂₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₂₀₁ and R₂₀₂ taken together with the atom to which they are attached form a substituted or unsubstituted non-aromatic heterocyclic group;
   R₂₀₃, R₂₀₄, R₂₀₅ and R₂₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₂₀₇, R₂₀₈ and R₂₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₂₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₂₁₁, R₂₁₂, R₂₁₃ and R₂₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
36. The method of sentence 35, wherein the method does not include the reduction of a prodrug to an active agent by a NAD(P)H quinone reductase.
37. The method of sentence 35, wherein the subject is not in need of an increase in nitric oxide bioactivity.
38. The method of sentence 35, wherein R₂₀₆ is not -H or -NH₂ when R₂₀₁-R₂₀₅ and R₂₀₇-R₂₁₄ are each -H.
39. A method for treating or preventing a neurodegenerative disorder in a subject, comprising administering to a subject in need thereof a therapeutically effective amount of at least one compound of Structural Formula (I) or (II): 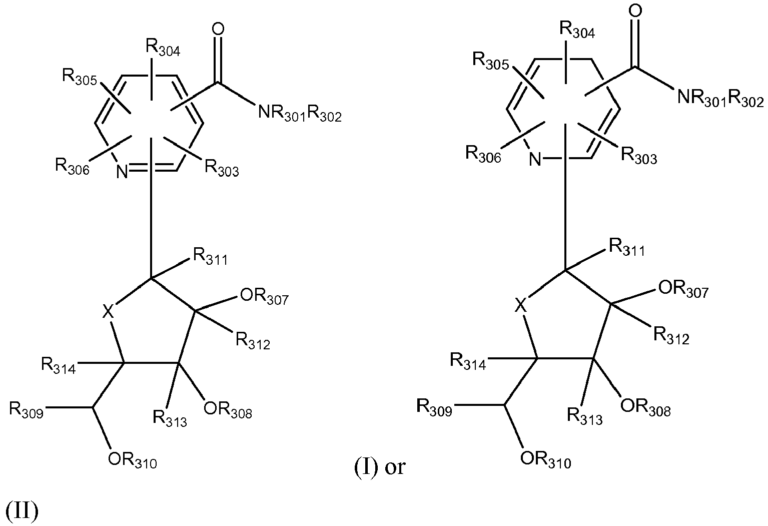 or a pharmaceutically acceptable salt thereof, wherein:
   R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
   R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
40. A method for treating or preventing a neurodegenerative disorder in a subject, comprising administering to a subject in need thereof a therapeutically effective amount of at least one compound of Structural Formula (III) or (IV): 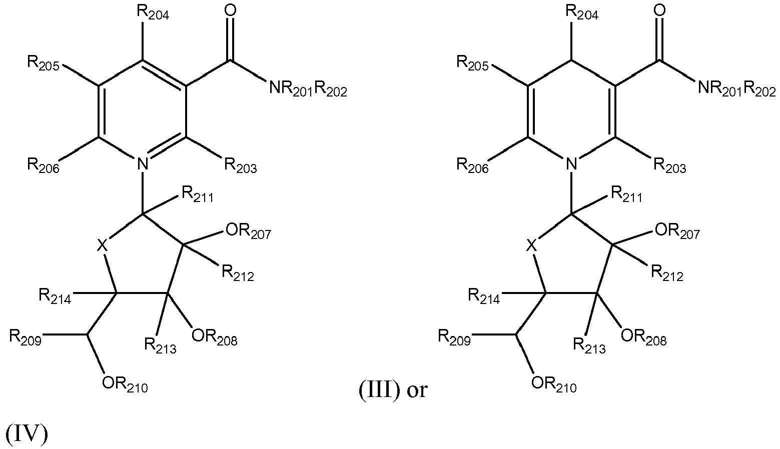 or a pharmaceutically acceptable salt thereof, wherein:
   R₂₀₁ and R₂₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₂₀₁ and R₂₀₂ taken together with the atom to which they are attached form a substituted or unsubstituted non-aromatic heterocyclic group;
   R₂₀₃, R₂₀₄, R₂₀₅ and R₂₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₂₀₇, R₂₀₈ and R₂₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₂₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₂₁₁, R₂₁₂, R₂₁₃ and R₂₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
41. The method of sentence 40, wherein the neurodegenerative disorder is selected from the group consisting of Alzheimer's disease (AD), Parkinson's disease (PD), Huntington disease (HD), amyotrophic lateral sclerosis (ALS; Lou Gehrig's disease), diffuse Lewy body disease, chorea-acanthocytosis, primary lateral sclerosis, Multiple Sclerosis (MS), ocular diseases, spinal muscle atrophy, chemotherapy-induced neuropathies, diabetes-induced neuropathies and Friedreich's ataxia.
42. A method for treating or preventing a blood coagulation disorder in a subject, comprising administering to a subject in need thereof a therapeutically effective amount of at least one compound of Structural Formula (I) or (II): 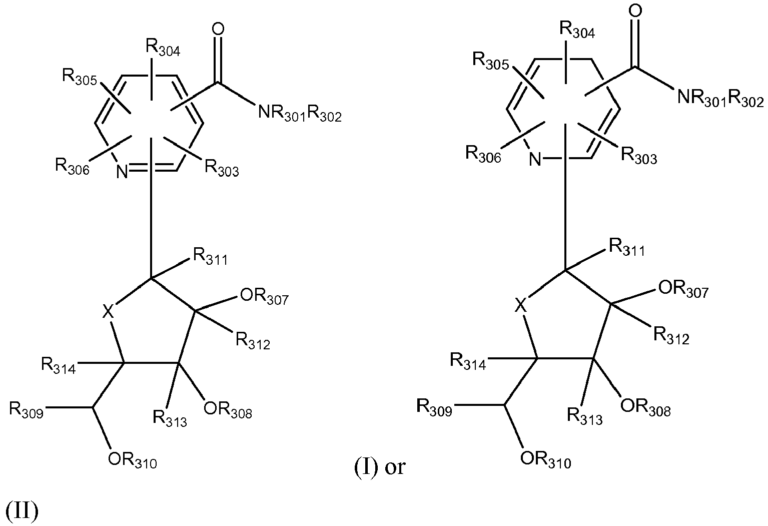 or a pharmaceutically acceptable salt thereof, wherein:
   R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
   R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
43. A method for treating or preventing a blood coagulation disorder in a subject, comprising administering to a subject in need thereof a therapeutically effective amount of at least one compound of Structural Formula (III) or (IV): 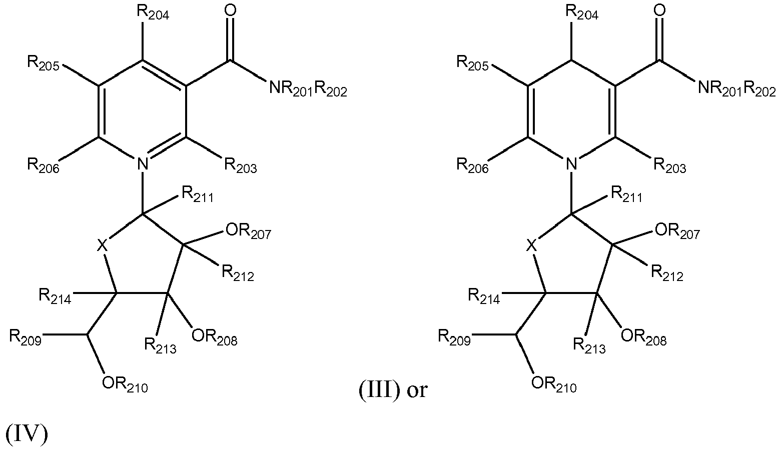 or a pharmaceutically acceptable salt thereof, wherein:
   R₂₀₁ and R₂₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₂₀₁ and R₂₀₂ taken together with the atom to which they are attached form a substituted or unsubstituted non-aromatic heterocyclic group;
   R₂₀₃, R₂₀₄, R₂₀₅ and R₂₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₂₀₇, R₂₀₈ and R₂₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₂₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₂₁₁, R₂₁₂, R₂₁₃ and R₂₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
44. The method of sentence 43, wherein the blood coagulation disorder is selected from the group consisting of thromboembolism, deep vein thrombosis, pulmonary embolism, stroke, myocardial infarction, miscarriage, thrombophilia associated with anti-thrombin III deficiency, protein C deficiency, protein S deficiency, resistance to activated protein C, dysfibrinogenemia, fibrinolytic disorders, homocystinuria, pregnancy, inflammatory disorders, myeloproliferative disorders, arteriosclerosis, angina, disseminated intravascular coagulation, thrombotic thrombocytopenic purpura, cancer metastasis, sickle cell disease, glomerular nephritis, drug induced thrombocytopenia, and re-occlusion during or after therapeutic clot lysis or procedures such as angioplasty or surgery.
45. The method of sentence 44, wherein the subject is not in need of an increase in nitric oxide bioactivity.
46. The method of sentence 44, wherein R₂₀₆ is not -H or -NH₂ when R₂₀₁-R₂₀₅ and R₂₀₇-R₂₁₄ are each -H.
47. A pharmaceutical composition comprising a pharmaceutically acceptable carrier or diluent and a compound represented by Structural Formula (V) or (VI): 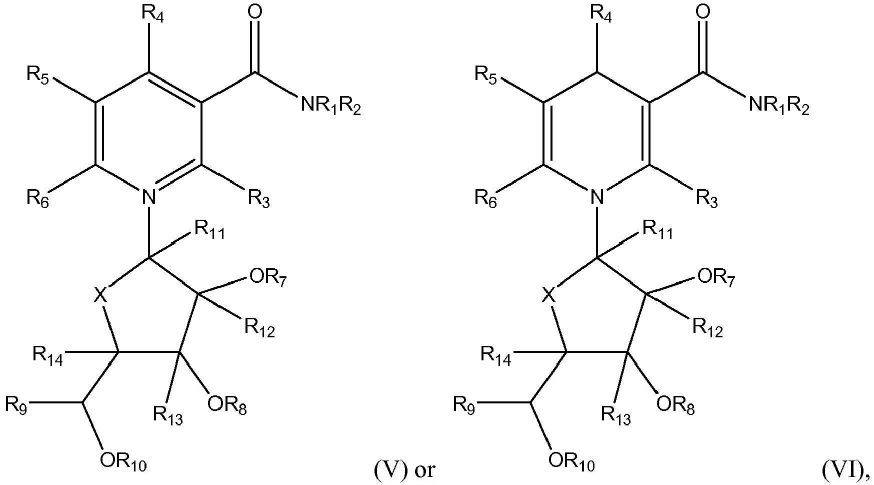 or a pharmaceutically acceptable salt thereof, wherein:
   R₁ and R₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁ and R₂ taken together with the atom to which they are attached form a substituted or unsubstituted non-aromatic heterocyclic group, provided that when one of R₁ and R₂ is -H, the other is not an alkyl group substituted by -C(O)OCH₂CH₃;
   R₃, R₄ and R₅ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₆ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRC(O)OR', -NO₂ and -NRC(O)R';
   R₇, R₈ and R₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₉ selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₁₁, R₁₂, R₁₃ and R₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2,
   provided that R₁-R₁₄ are not each -H and that R₁-R₉ and R₁₁-R₁₄ are not each -H when R₁₀ is -C(O)C₆H₅.
48. The pharmaceutical composition of sentence 47, wherein X is O.
49. The pharmaceutical composition of sentence 48, wherein R₁ is -H.
50. The pharmaceutical composition of sentence 49, wherein R₇, R₈ and R₁₀ are independently -H, -C(O)R or -C(O)OR.
51. The pharmaceutical composition of sentence 50, wherein R₉ is -H.
52. The pharmaceutical composition of sentence 51, wherein R₂ is -H.
53. The pharmaceutical composition of sentence 52, wherein R₇, R₈ and R₁₀ are independently -H or -C(O)CH₃.
54. The pharmaceutical composition of sentence 47, wherein R₄ is -H or a halogen.
55. The pharmaceutical composition of sentence 54, wherein -H is -D and the halogen is -F.
56. A pharmaceutical composition comprising a pharmaceutically acceptable carrier or diluent and a compound represented by Structural Formula (VII) or (VIII): 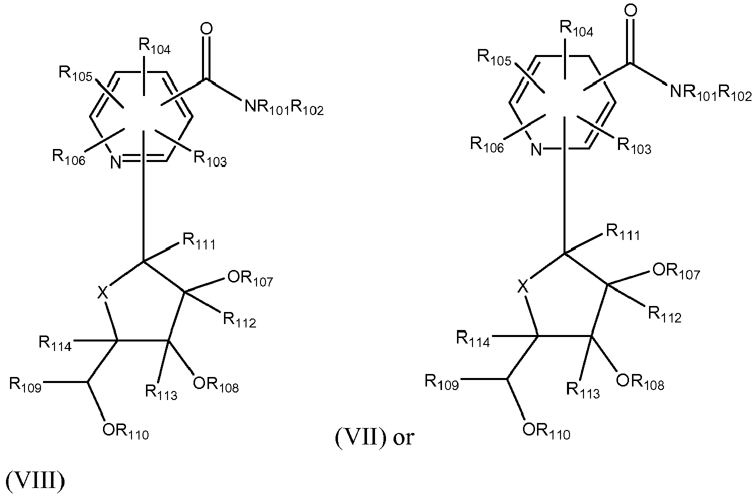 or a pharmaceutically acceptable salt thereof, wherein:
   R₁₀₁ and R₁₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁₀₁ and R₁₀₂ taken together with the atom to which they are attached form a substituted or unsubstituted non-aromatic heterocyclic group;
   R₁₀₃, R₁₀₄, R₁₀₅ and R₁₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₁₀₇ and R₁₀₈ are selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, wherein at least one of R₁₀₇ and R₁₀₈ is a substituted or unsubstituted alkyl group, a substituted or unsubstituted aryl group, -C(O)R, -C(O)OR, -C(O)NHR, -C(S)R, -C(S)OR or -C(O)SR;
   R₁₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₁₁₀ is selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, provided that R₁₁₀ is not -C(O)C₆H₅;
   R₁₁₁, R₁₁₂, R₁₁₃ and R₁₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
57. A pharmaceutical composition comprising a pharmaceutically acceptable carrier or diluent and a compound represented by Structural Formula (IX) or (X): 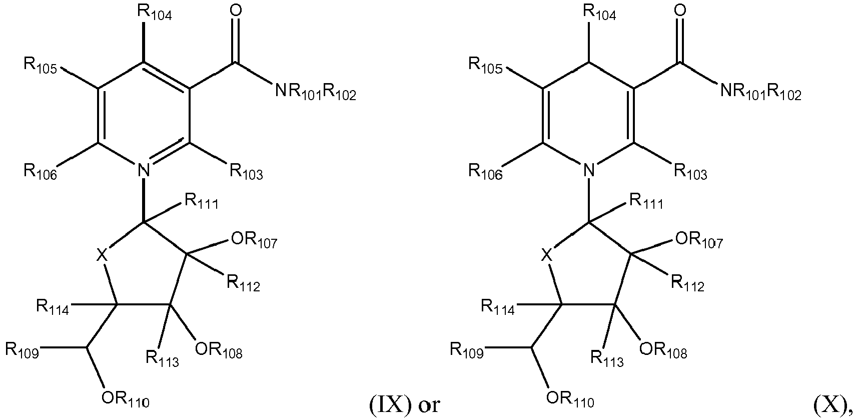 or a pharmaceutically acceptable salt thereof, wherein:
   R₁₀₁ and R₁₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁₀₁ and R₁₀₂ taken together with the atom to which they are attached form a substituted or unsubstituted non-aromatic heterocyclic group;
   R₁₀₃, R₁₀₄, R₁₀₅ and R₁₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₁₀₇ and R₁₀₈ are selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, wherein at least one of R₁₀₇ and R₁₀₈ is a substituted or unsubstituted alkyl group, a substituted or unsubstituted aryl group, -C(O)R, -C(O)OR, -C(O)NHR, -C(S)R, -C(S)OR or -C(O)SR;
   R₁₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₁₁₀ is selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, provided that R₁₁₀ is not -C(O)C₆H₅;
   R₁₁₁, R₁₁₂, R₁₁₃ and R₁₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
58. The pharmaceutical composition of sentence 57, wherein at least one of R₁₀₇ and R₁₀₈ is -C(O)R.
59. The pharmaceutical composition of sentence 58, wherein -C(O)R is - C(O)CH₃.
60. The pharmaceutical composition of sentence 58, wherein R₁₀₇, R₁₀₈ and R₁₁₀ are independently -H or -C(O)R.
61. The pharmaceutical composition of sentence 59, wherein R₁₀₇, R₁₀₈ and R₁₁₀ are independently -H or -C(O)CH₃.
62. The pharmaceutical composition of sentence 61, wherein R₁₀₁ and R₁₀₂ are each -H.
63. The pharmaceutical composition of sentence 62, wherein R₁₀₉ is -H.
64. The pharmaceutical composition of sentence 63, wherein R₁₀₃-R₁₀₆ and R₁₁₁-R₁₁₄ are each -H.
65. The pharmaceutical composition of sentence 57, wherein R₁₀₄ is -H or a halogen.
66. The pharmaceutical composition of sentence 65, wherein -H is -D and the halogen is -F.
67. A compound represented by Structural Formula (XI) or (XII): 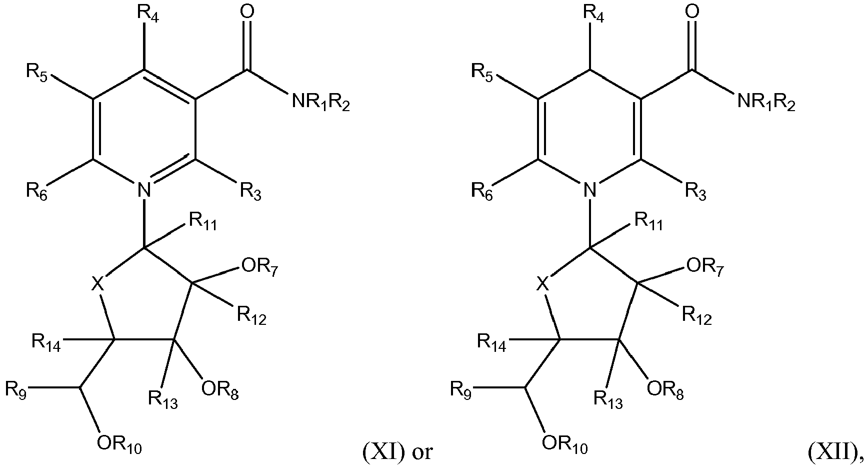 or a pharmaceutically acceptable salt thereof, wherein:
   R₁ and R₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁ and R₂ taken together with the atom to which they are attached form a substituted or unsubstituted non-aromatic heterocyclic group, provided that when one of R₁ and R₂ is -H, the other is not a 2,2,6,6-tetramethyl-1-oxypiperidin-4-yl group and is not an alkyl group substituted by -C(O)OCH₂CH₃;
   R₃ and R₄ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₅ is selected from the group consisting of -H, a substituted alkyl group, a substituted or unsubstituted aryl group, a substituted or unsubstituted non-aromatic heterocyclic group, halogen, -OR, -CN, -CO₂R, -OCOR, - OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₆ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRC(O)OR', -NO₂ and -NRC(O)R';
   R₇, R₈ and R₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₉ selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₁₁, R₁₂, R₁₃ and R₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2,
   provided that R₁-R₆ are not each -H.
68. The compound of sentence 67, wherein R₄ is -H or a halogen.
69. The compound of sentence 68, wherein -H is -D and the halogen is -F.
70. A compound represented by Structural Formula (XI) or (XII): 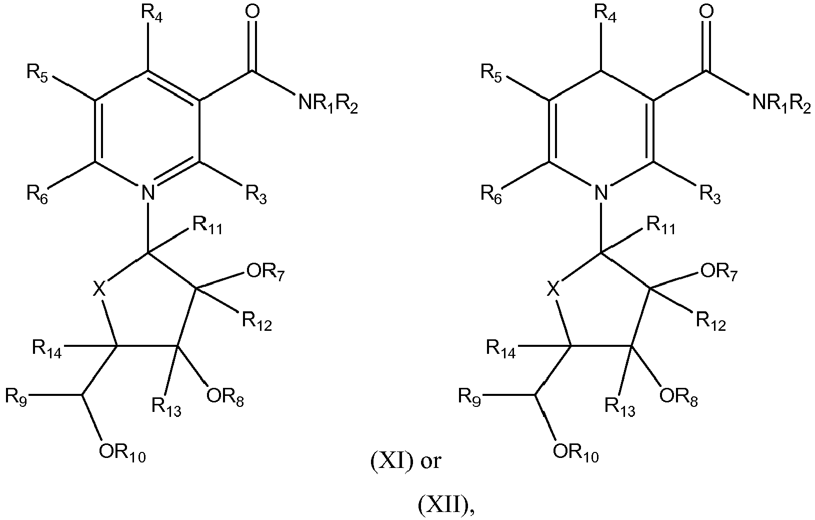 or a pharmaceutically acceptable salt thereof, wherein:
   R₁ and R₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁ and R₂ taken together with the atom to which they are attached form a substituted or unsubstituted non-aromatic heterocyclic group;
   R₃, R₄, R₅ and R₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₇, R₈ and R₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, wherein at least one of R₇ and R₈ is a substituted or unsubstituted alkyl group, a substituted or unsubstituted aryl group, -C(O)R, -C(O)OR, -C(O)NHR, - C(S)R, -C(S)OR or -C(O)SR, provided that none of R₇ and R₈ are -C(O)C₆H₅ and that R₇ and R₈ are not both -C(O)CH₃ or -C(O)C₆H₄F;
   R₉ selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₁₁, R₁₂, R₁₃ and R₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
71. The compound of sentence 70, wherein R₁-R₆, R₉ and R₁₁-R₁₄ are each -H.
72. The compound of sentence 70, wherein R₄ is -H or a halogen.
73. The compound of sentence 72, wherein -H is -D and the halogen is -F.
74. A compound represented by Structural Formula (XI) or (XII): 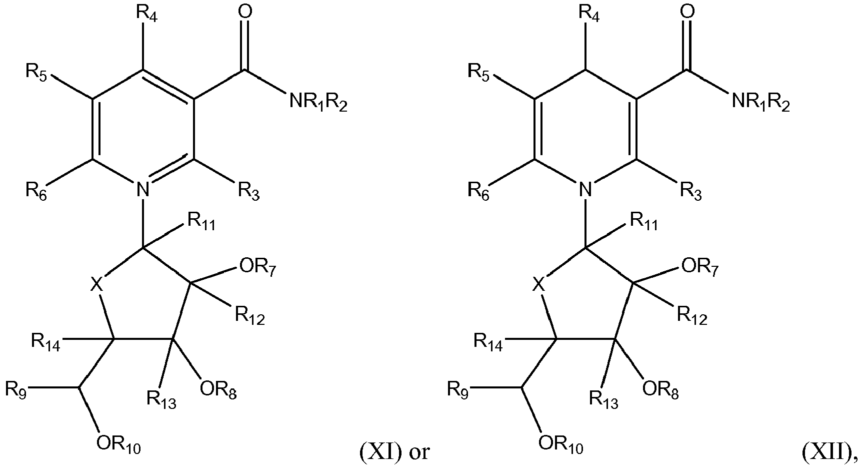 or a pharmaceutically acceptable salt thereof, wherein:
   R₁ and R₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁ and R₂ taken together with the atom to which they are attached form a substituted or unsubstituted non-aromatic heterocyclic group;
   R₃, R₄, R₅ and R₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₇ and R₈ are selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₉ selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₁₀ is selected from the group consisting of a substituted or unsubstituted alkyl group, a substituted or unsubstituted aryl group, -C(O)R, - C(O)OR, -C(O)NHR, -C(S)R, -C(S)OR and -C(O)SR, provided that R₁₀ is not -C(C₆H₅)₃, -C(O)C₆H₅, -C(O)CH₃, -C(O)C₆H₄F or - C(O)CH(OC(O)CH₃)CH(CH₃)CH₂CH₃;
   R₁₁, R₁₂, R₁₃ and R₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
75. The compound of sentence 74, wherein R₁-R₆, R₉ and R₁₁-R₁₄ are each -H.
76. The compound of sentence 75, wherein R₄ is -H or a halogen.
77. The compound of sentence 76, wherein -H is -D and the halogen is -F.
78. A composition comprising compound represented by Structural Formula (XI) or (XII): 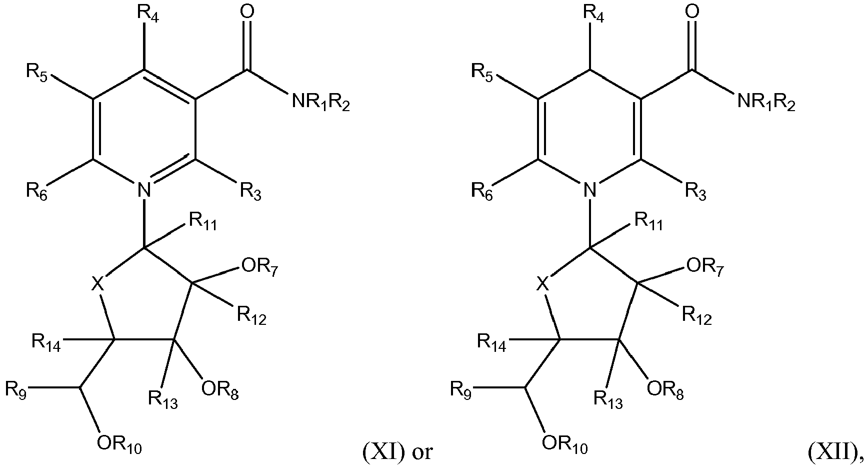 or a pharmaceutically acceptable salt thereof, wherein:
   R₁ and R₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁ and R₂ taken together with the atom to which they are attached form a substituted or unsubstituted non-aromatic heterocyclic group, provided that when one of R₁ and R₂ is -H, the other is not a 2,2,6,6-tetramethyl-1-oxypiperidin-4-yl group and is not an alkyl group substituted by -C(O)OCH₂CH₃;
   R₃ and R₄ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₅ is selected from the group consisting of -H, a substituted alkyl group, a substituted or unsubstituted aryl group, a substituted or unsubstituted non-aromatic heterocyclic group, halogen, -OR, -CN, -CO₂R, -OCOR, - OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₆ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRC(O)OR', -NO₂ and -NRC(O)R';
   R₇, R₈ and R₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₉ selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₁₁, R₁₂, R₁₃ and R₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2,
   provided that R₁-R₆ are not each -H.
79. The composition of sentence 78, wherein the composition is a solvate.
80. The composition of sentence 79, wherein the composition is a hydrate.
81. The composition of sentence 78, wherein the composition is crystalline.
82. A compound represented by Structural Formula (XI) or (XII): 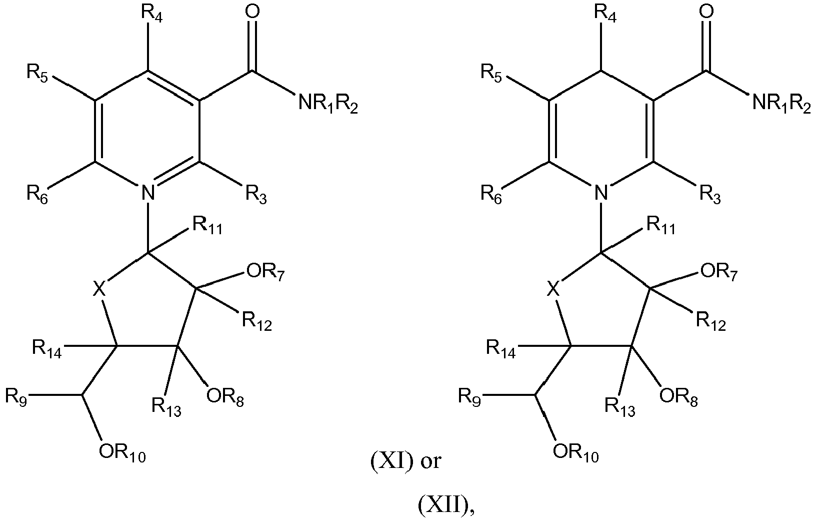 or a pharmaceutically acceptable salt thereof, wherein:
   R₁ and R₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁ and R₂ taken together with the atom to which they are attached form a substituted or unsubstituted non-aromatic heterocyclic group;
   R₃, R₄, R₅ and R₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₇, R₈ and R₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, wherein at least one of R₇ and R₈ is a substituted or unsubstituted alkyl group, a substituted or unsubstituted aryl group, -C(O)R, -C(O)OR, -C(O)NHR, - C(S)R, -C(S)OR or -C(O)SR, provided that none of R₇ and R₈ are -C(O)C₆H₅ and that R₇ and R₈ are not both -C(O)CH₃ or -C(O)C₆H₄F;
   R₉ selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₁₁, R₁₂, R₁₃ and R₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
83. The composition of sentence 82, wherein the composition is a solvate.
84. The composition of sentence 83, wherein the composition is a hydrate.
85. The composition of sentence 82, wherein the composition is crystalline.
86. A compound represented by Structural Formula (XI) or (XII): 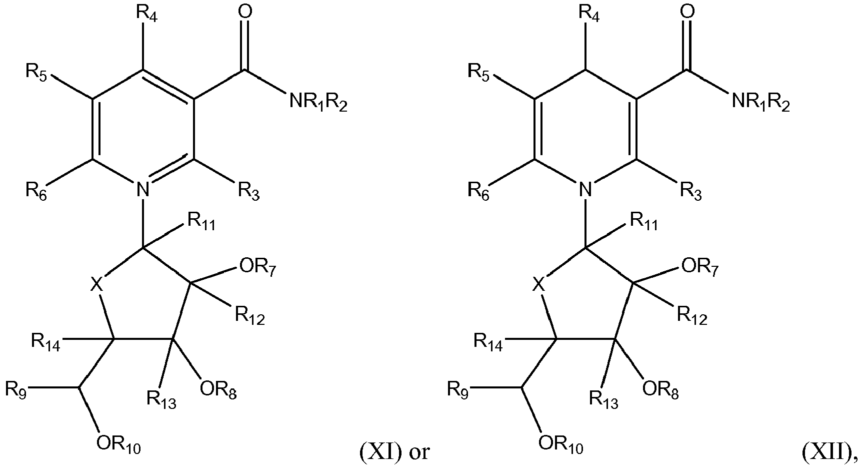 or a pharmaceutically acceptable salt thereof, wherein:
   R₁ and R₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁ and R₂ taken together with the atom to which they are attached form a substituted or unsubstituted non-aromatic heterocyclic group;
   R₃, R₄, R₅ and R₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₇ and R₈ are selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₉ selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₁₀ is selected from the group consisting of a substituted or unsubstituted alkyl group, a substituted or unsubstituted aryl group, -C(O)R, - C(O)OR, -C(O)NHR, -C(S)R, -C(S)OR and -C(O)SR, provided that R₁₀ is not -C(C₆H₅)₃, -C(O)C₆H₅, -C(O)CH₃, -C(O)C₆H₄F or - C(O)CH(OC(O)CH₃)CH(CH₃)CH₂CH₃;
   R₁₁, R₁₂, R₁₃ and R₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
87. The composition of sentence 86, wherein the composition is a solvate.
88. The composition of sentence 87, wherein the composition is a hydrate.
89. The composition of sentence 86, wherein the composition is crystalline.
90. A method for treating or preventing one or more of cataracts, retinopathy, retinitis pigmentosa, ocular neuritis or a vascular disease of the capillary beds of the eye, comprising administering to a subject a therapeutically effective amount of at least one compound of Structural Formula (I) or (II): 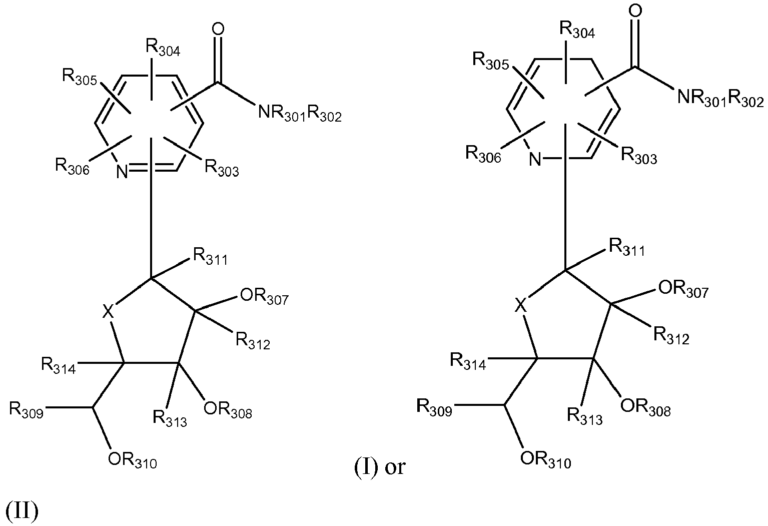 or a pharmaceutically acceptable salt thereof, wherein:
   R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
   R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
91. A method for treating or preventing one or more of cataracts, retinopathy, retinitis pigmentosa, ocular neuritis or a vascular disease of the capillary beds of the eye, comprising administering to a subject a therapeutically effective amount of at least one compound of Structural Formula (III) or (IV): 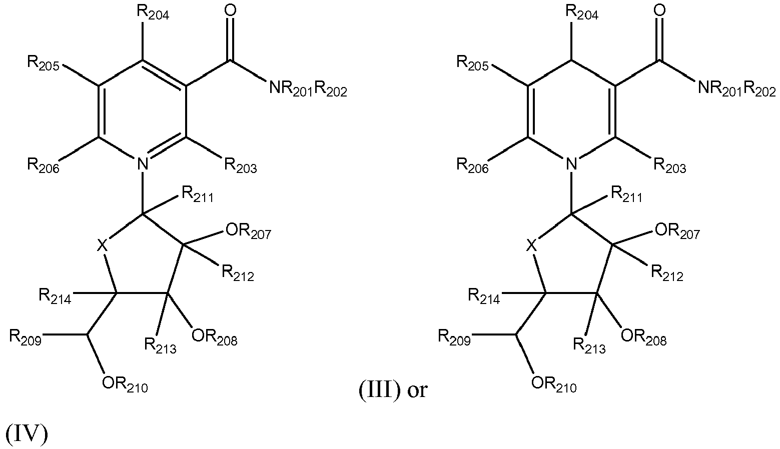 or a pharmaceutically acceptable salt thereof, wherein:
   R₂₀₁ and R₂₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₂₀₁ and R₂₀₂ taken together with the atom to which they are attached form a substituted or unsubstituted non-aromatic heterocyclic group;
   R₂₀₃, R₂₀₄, R₂₀₅ and R₂₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -COR, -SR, -OSO₃H, -S(O)ₙR, -S(O)ₙOR, -S(O)ₙNRR', -NRR', -NRC(O)OR', -NO₂ and -NRC(O)R';
   R₂₀₇, R₂₀₈ and R₂₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
   R₂₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -COR, -SR, -OSO₃H, -S(O)ₙR, -S(O)ₙOR, -S(O)ₙNRR', -NRR', -NRC(O)OR' and -NRC(O)R';
   R₂₁₁, R₂₁₂, R₂₁₃ and R₂₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -COR, -OSO₃H, -S(O)ₙR, -S(O)ₙOR, -S(O)ₙNRR', - NRR', -NRC(O)OR', -NO₂ and -NRC(O)R';
   R and R' are independently -H, a substituted or unsubstituted alkyl group, a substituted or unsubstituted aryl group or a substituted or unsubstituted non-aromatic heterocyclic group;
   X is O or S; and
   n is 1 or 2.
92. The method of sentence 91, wherein the method is treating or preventing cataracts.
93. The method of sentence 91, wherein the method is treating or preventing retinopathy.
94. The method of sentence 91, wherein the method is treating or preventing retinitis pigmentosa.
95. The method of sentence 91, wherein the method is treating or preventing ocular neuritis.
96. The method of sentence 91, wherein the method is treating or preventing a vascular disease of the capillary beds of the eye.

## Claims

1. A composition comprising at least one compound of Structural Formula (I) or (II): 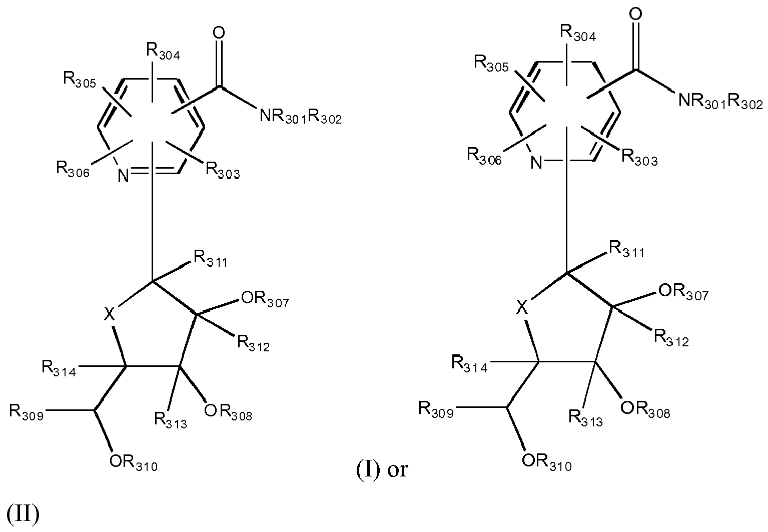 or a pharmaceutically acceptable salt thereof, for treating or preventing a disease or disorder associated with cell death, cell dysfunction or aging in a subject; for treating or preventing insulin resistance, a metabolic syndrome, hypercholesterolemia, artherogenic dyslipidemia, diabetes, or complications thereof, or for increasing insulin sensitivity in a subject; for therapeutically reducing the weight of a subject, or preventing weight gain in a subject; for treating or preventing a blood coagulation disorder in a subject; for treating or preventing one or more of cataracts, retinopathy, retinitis pigmentosa or a vascular disease of the capillary beds of the eye, wherein:
R₃₀₁ and R₃₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₃₀₁ and R₃₀₂ taken together with the atom to which they are attached form a substituted or unsubstituted non-aromatic heterocyclic group;
R₃₀₃, R₃₀₄, R₃₀₅ and R₃₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -SR, -OSO₃H, -S(O)ₙR, -S(O)ₙOR, -S(O)ₙNRR', -NRR', -NRC(O)OR', -NO₂ and -NRC(O)R';
R₃₀₇, R₃₀₈ and R₃₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₃₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -SR, -OSO₃H, -S(O)ₙR, -S(O)ₙOR, -S(O)ₙNRR', -NRR', -NRC(O)OR' and -NRC(O)R';
R₃₁₁, R₃₁₂, R₃₁₃ and R₃₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2,
wherein when a group is substituted, the substituents are independently selected from one or more of -OH, halogen, -OR^{a}, -O-COR^{a}, -C(O)R^{a}, -CN, -NO₂, -COOH, -COOR^{a}, -OCO₂R^{a}, -C(O)NR^{a}R^{b}, -OC(O)NR^{a}R^{b}, -SO₃H, -NH₂, -NHR^{a}, -N(R^{a}R^{b}), -COOR^{a}, -CHO, -CONH₂, -CONHR^{a}, -CON(R^{a}R^{b}), -NHCOR^{a}, -NRCOR^{a}, -NHCONH₂, -NHCONR^{a}H, -NHCON(R^{a}R^{b}), -NR^{c}CONH₂, -NR^{c}CONR^{a}H, -NR^{c}CON(R^{a}R^{b}), -C(=NH)-NH₂, -C(=NH)-NHR^{a}, -C(=NH)-N(R^{a}R^{b}), -C(=NR^{c})-NH₂, -C(=NR^{c})-NHR^{a}, -C(=NR^{c})-N(R^{a}R^{b}), -NH-C(=NH)-NHR^{a}, -NH-C(=NH)-N(R^{a}R^{b}), -NH-C(=NR^{c})-NH₂, -NH-C(=NR^{c})-NHR^{a}, -NH-C(=NR^{c})-N(R^{a}R^{b}), -NR^{d}-C(=NH)-NH₂, -NR^{d}-C(=NH)-NHR^{a}, -NR^{d}-C(=NH)-N(R^{a}R^{b}), -NR^{d}-C(=NR^{c})-NH₂, -NR^{d}-C(=NR^{c})-NHR^{a}, -NR^{d}-C(=NR^{c})-N(R^{a}R^{b}), -NHNH₂, -NHNHR^{a}, -SO₂NH₂, -SO₂NHRₐ, -SO₂NR^{a}R^{b}, -CH=CHR^{a}, -CH=CR^{a}R^{b}, -CR^{c}=CR^{a}R^{b}, -CR^{c}=CHR^{a}, -CR^{c}=CR^{a}R^{b}, -CCR^{a}, -SH, -SOₖR^{a}, -S(O)ₖOR^{a} and -NH-C(=NH)-NH₂, wherein
k is 0, 1 or 2;
R^{a}-R^{d} are each independently an aliphatic, benzyl, or aromatic group; and
-NR^{a}R^{b}, taken together, can also form an unsubstituted non-aromatic heterocyclic group;
wherein a non-aromatic heterocyclic group, benzylic group or aryl group can also have an aliphatic or substituted aliphatic group as a substituent; a substituted aliphatic group can also have a non-aromatic heterocyclic ring, a substituted non-aromatic heterocyclic ring, benzyl, substituted benzyl, aryl or substituted aryl group as a substituent; and a substituted aliphatic, non-aromatic heterocyclic group, substituted aryl, or substituted benzyl group can have more than one substituent.

2. A method for prolonging the lifespan of a subject comprising contacting the cell or subject, respectively, with at least one compound of Structural Formula (I) or (II) as defined in claim 1.

3. A method for preventing the differentiation of a preadipocyte comprising contacting the preadipocyte with at least one compound of Structural Formula (I) or (II) as defined in claim 1.

4. A pharmaceutical composition comprising a pharmaceutically acceptable carrier or diluent and a compound represented by Structural Formula (V) or (VI): 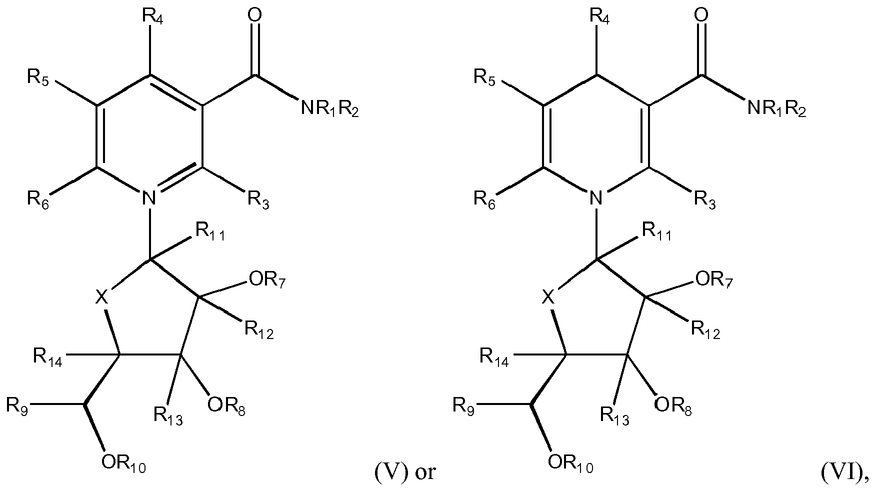 or a pharmaceutically acceptable salt thereof, wherein:
R₁ and R₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁ and R₂ taken together with the atom to which they are attached form a substituted or unsubstituted non-aromatic heterocyclic group, provided that when one of R₁ and R₂ is -H, the other is not an alkyl group substituted by -C(O)OCH₂CH₃;
R₃, R₄ and R₅ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -SR, -OSO₃H, -S(O)ₙR, -S(O)ₙOR, -S(O)ₙNRR', -NRR', -NRC(O)OR', -NO₂ and -NRC(O)R';
R₆ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -SR, -OSO₃H, -S(O)ₙR, -S(O)ₙOR, -S(O)ₙNRR', -NRC(O)OR', -NO₂ and -NRC(O)R';
R₇, R₈ and R₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₉ selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -SR, -OSO₃H, -S(O)ₙR, -S(O)ₙOR, -S(O)ₙNRR', -NRR', -NRC(O)OR' and -NRC(O)R';
R₁₁, R₁₂, R₁₃ and R₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2,
wherein when a group is substituted, the substituents are independently selected from one or more of -OH, halogen, -OR^{a}, -O-COR^{a}, -C(O)R^{a}, -CN, -NO₂, -COOH, -COOR^{a}, -OCO₂R^{a}, -C(O)NR^{a}R^{b}, -OC(O)NR^{a}R^{b}, -SO₃H, -NH₂, -NHR^{a}, -N(R^{a}R^{b}), -COOR^{a}, -CHO, -CONH₂, -CONHR^{a}, -CON(R^{a}R^{b}), -NHCOR^{a}, -NRCOR^{a}, -NHCONH₂, -NHCONR^{a}H, -NHCON(R^{a}R^{b}), -NR^{c}CONH₂, -NR^{c}CONR^{a}H, -NR^{c}CON(R^{a}R^{b}), -C(=NH)-NH₂, -C(=NH)-NHR^{a}, -C(=NH)-N(R^{a}R^{b}), -C(=NR^{c})-NH₂, -C(=NR^{c})-NHR^{a}, -C(=NR^{c})-N(R^{a}R^{b}), -NH-C(=NH)-NHR^{a}, -NH-C(=NH)-N(R^{a}R^{b}), -NH-C(=NR^{c})-NH₂, -NH-C(=NR^{c})-NHR^{a}, -NH-C(=NR^{c})-N(R^{a}R^{b}), -NR^{d}-C(=NH)-NH₂, -NR^{d}-C(=NH)-NHR^{a}, -NR^{d}-C(=NH)-N(R^{a}R^{b}), -NR^{d}-C(=NR^{c})-NH₂, -NR^{d}-C(=NR^{c})-NHR^{a}, -NR^{d}-C(=NR^{c})-N(R^{a}R^{b}), -NHNH₂, -NHNHR^{a}, -SO₂NH₂, -SO₂NHRₐ, -SO₂NR^{a}R^{b}, -CH=CHR^{a}, -CH=CR^{a}R^{b}, -CR^{c}=CR^{a}R^{b}, -CR^{c}=CHR^{a}, -CR^{c}=CR^{a}R^{b}, -CCR^{a}, -SH, -SOₖR^{a}, -S(O)ₖOR^{a} and -NH-C(=NH)-NH₂, wherein
k is 0, 1 or 2;
R^{a}-R^{d} are each independently an aliphatic, benzyl, or aromatic group; and
-NR^{a}R^{b}, taken together, can also form an unsubstituted non-aromatic heterocyclic group;
wherein a non-aromatic heterocyclic group, benzylic group or aryl group can also have an aliphatic or substituted aliphatic group as a substituent; a substituted aliphatic group can also have a non-aromatic heterocyclic ring, a substituted non-aromatic heterocyclic ring, benzyl, substituted benzyl, aryl or substituted aryl group as a substituent; and a substituted aliphatic, non-aromatic heterocyclic group, substituted aryl, or substituted benzyl group can have more than one substituent;
provided that R₁-R₁₄ are not each -H and that R₁-R₉ and R₁₁-R₁₄ are not each -H when R₁₀ is -C(O)C₆H₅.

5. A pharmaceutical composition comprising a pharmaceutically acceptable carrier or diluent and a compound represented by Structural Formula (VII) or (VIII): 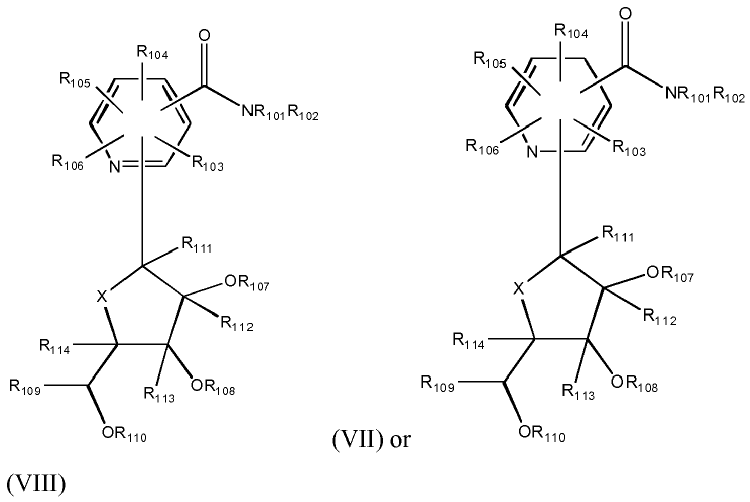 or a pharmaceutically acceptable salt thereof, wherein:
R₁₀₁ and R₁₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁₀₁ and R₁₀₂ taken together with the atom to which they are attached form a substituted or unsubstituted non-aromatic heterocyclic group;
R₁₀₃, R₁₀₄, R₁₀₅ and R₁₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -SR, -OSO₃H, -S(O)ₙR, -S(O)ₙOR, -S(O)ₙNRR', -NRR', -NRC(O)OR', -NO₂ and -NRC(O)R';
R₁₀₇ and R₁₀₈ are selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, wherein at least one of R₁₀₇ and R₁₀₈ is a substituted or unsubstituted alkyl group, a substituted or unsubstituted aryl group, -C(O)R, -C(O)OR, -C(O)NHR, -C(S)R, -C(S)OR or -C(O)SR;
R₁₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -SR, -OSO₃H, -S(O)ₙR, -S(O)ₙOR, -S(O)ₙNRR', -NRR', -NRC(O)OR' and -NRC(O)R';
R₁₁₀ is selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, provided that R₁₁₀ is not -C(O)C₆H₅;
R₁₁₁, R₁₁₂, R₁₁₃ and R₁₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2,
wherein when a group is substituted, the substituents are independently selected from one or more of -OH, halogen, -OR^{a}, -O-COR^{a}, -C(O)R^{a}, -CN, -NO₂, -COOH, -COOR^{a}, -OCO₂R^{a}, -C(O)NR^{a}R^{b}, -OC(O)NR^{a}R^{b}, -SO₃H, -NH₂, -NHR^{a}, -N(R^{a}R^{b}), -COOR^{a}, -CHO, -CONH₂, -CONHR^{a}, -CON(R^{a}R^{b}), -NHCOR^{a}, -NRCOR^{a}, -NHCONH₂, -NHCONR^{a}H, -NHCON(R^{a}R^{b}), -NR^{c}CONH₂, -NR^{c}CONR^{a}H, -NR^{c}CON(R^{a}R^{b}), -C(=NH)-NH₂, -C(=NH)-NHR^{a}, -C(=NH)-N(R^{a}R^{b}), -C(=NR^{c})-NH₂, -C(=NR^{c})-NHR^{a}, -C(=NR^{c})-N(R^{a}R^{b}), -NH-C(=NH)-NHR^{a}, -NH-C(=NH)-N(R^{a}R^{b}), -NH-C(=NR^{c})-NH₂, -NH-C(=NR^{c})-NHR^{a}, -NH-C(=NR^{c})-N(R^{a}R^{b}), -NR^{d}-C(=NH)-NH₂, -NR^{d}-C(=NH)-NHR^{a}, -NR^{d}-C(=NH)-N(R^{a}R^{b}), -NR^{d}-C(=NR^{c})-NH₂, -NR^{d}-C(=NR^{c})-NHR^{a}, -NR^{d}-C(=NR^{c})-N(R^{a}R^{b}), -NHNH₂, -NHNHR^{a}, -SO₂NH₂, -SO₂NHRₐ, -SO₂NR^{a}R^{b}, -CH=CHR^{a}, -CH=CR^{a}R^{b}, -CR^{c}=CR^{a}R^{b}, -CR^{c}=CHR^{a}, -CR^{c}=CR^{a}R^{b}, -CCR^{a}, -SH, -SOₖR^{a}, -S(O)ₖOR^{a} and -NH-C(=NH)-NH₂, wherein
k is 0, 1 or 2;
R^{a}-R^{d} are each independently an aliphatic, benzyl, or aromatic group; and
-NR^{a}R^{b}, taken together, can also form an unsubstituted non-aromatic heterocyclic group;
wherein a non-aromatic heterocyclic group, benzylic group or aryl group can also have an aliphatic or substituted aliphatic group as a substituent; a substituted aliphatic group can also have a non-aromatic heterocyclic ring, a substituted non-aromatic heterocyclic ring, benzyl, substituted benzyl, aryl or substituted aryl group as a substituent; and a substituted aliphatic, non-aromatic heterocyclic group, substituted aryl, or substituted benzyl group can have more than one substituent.

6. A pharmaceutical composition comprising a pharmaceutically acceptable carrier or diluent and a compound represented by Structural Formula (IX) or (X): 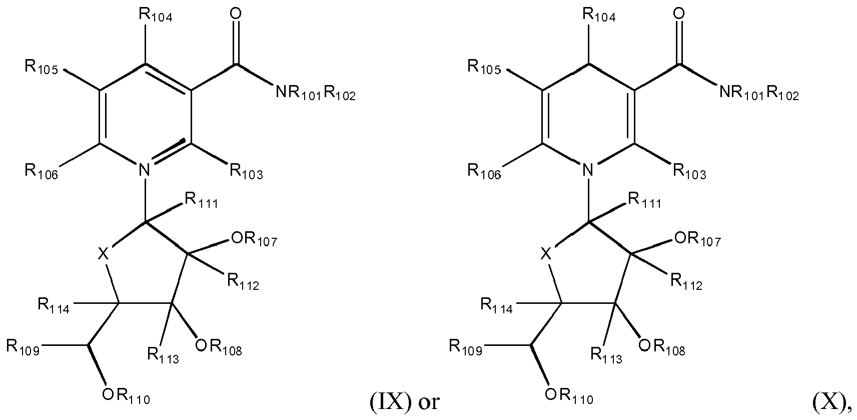 or a pharmaceutically acceptable salt thereof, wherein:
R₁₀₁ and R₁₀₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁₀₁ and R₁₀₂ taken together with the atom to which they are attached form a substituted or unsubstituted non-aromatic heterocyclic group;
R₁₀₃, R₁₀₄, R₁₀₅ and R₁₀₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, - C(O)NRR', -OC(O)NRR', -C(O)R, -SR, -OSO₃H, -S(O)ₙR, -S(O)ₙOR, -S(O)ₙNRR', -NRR', -NRC(O)OR', -NO₂ and -NRC(O)R';
R₁₀₇ and R₁₀₈ are selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, wherein at least one of R₁₀₇ and R₁₀₈ is a substituted or unsubstituted alkyl group, a substituted or unsubstituted aryl group, -C(O)R, -C(O)OR, -C(O)NHR, -C(S)R, -C(S)OR or -C(O)SR;
R₁₀₉ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -SR, -OSO₃H, -S(O)ₙR, -S(O)ₙOR, -S(O)ₙNRR', -NRR', -NRC(O)OR' and -NRC(O)R';
R₁₁₀ is selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, provided that R₁₁₀ is not -C(O)C₆H₅;
R₁₁₁, R₁₁₂, R₁₁₃ and R₁₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2,
wherein when a group is substituted, the substituents are independently selected from one or more of -OH, halogen, -OR^{a}, -O-COR^{a}, -C(O)R^{a}, -CN, -NO₂, -COOH, -COOR^{a}, -OCO₂R^{a}, -C(O)NR^{a}R^{b}, -OC(O)NR^{a}R^{b}, -SO₃H, -NH₂, -NHR^{a}, -N(R^{a}R^{b}), -COOR^{a}, -CHO, -CONH₂, -CONHR^{a}, -CON(R^{a}R^{b}), -NHCOR^{a}, -NRCOR^{a}, -NHCONH₂, -NHCONR^{a}H, -NHCON(R^{a}R^{b}), -NR^{c}CONH₂, -NR^{c}CONR^{a}H, -NR^{c}CON(R^{a}R^{b}), -C(=NH)-NH₂, -C(=NH)-NHR^{a}, -C(=NH)-N(R^{a}R^{b}), -C(=NR^{c})-NH₂, -C(=NR^{c})-NHR^{a}, -C(=NR^{c})-N(R^{a}R^{b}), -NH-C(=NH)-NHR^{a}, -NH-C(=NH)-N(R^{a}R^{b}), -NH-C(=NR^{c})-NH₂, -NH-C(=NR^{c})-NHR^{a}, -NH-C(=NR^{c})-N(R^{a}R^{b}), -NR^{d}-C(=NH)-NH₂, -NR^{d}-C(=NH)-NHR^{a}, -NR^{d}-C(=NH)-N(R^{a}R^{b}), -NR^{d}-C(=NR^{c})-NH₂, -NR^{d}-C(=NR^{c})-NHR^{a}, -NR^{d}-C(=NR^{c})-N(R^{a}R^{b}), -NHNH₂, -NHNHR^{a}, -SO₂NH₂, -SO₂NHRₐ, -SO₂NR^{a}R^{b}, -CH=CHR^{a}, -CH=CR^{a}R^{b}, -CR^{c}=CR^{a}R^{b}, -CR^{c}=CHR^{a}, -CR^{c}=CR^{a}R^{b}, -CCR^{a}, -SH, -SOₖR^{a}, -S(O)ₖOR^{a} and -NH-C(=NH)-NH₂, wherein
k is 0, 1 or 2;
R^{a}-R^{d} are each independently an aliphatic, benzyl, or aromatic group; and
-NR^{a}R^{b}, taken together, can also form an unsubstituted non-aromatic heterocyclic group;
wherein a non-aromatic heterocyclic group, benzylic group or aryl group can also have an aliphatic or substituted aliphatic group as a substituent; a substituted aliphatic group can also have a non-aromatic heterocyclic ring, a substituted non-aromatic heterocyclic ring, benzyl, substituted benzyl, aryl or substituted aryl group as a substituent; and a substituted aliphatic, non-aromatic heterocyclic group, substituted aryl, or substituted benzyl group can have more than one substituent.

7. A compound represented by Structural Formula (XI) or (XII): 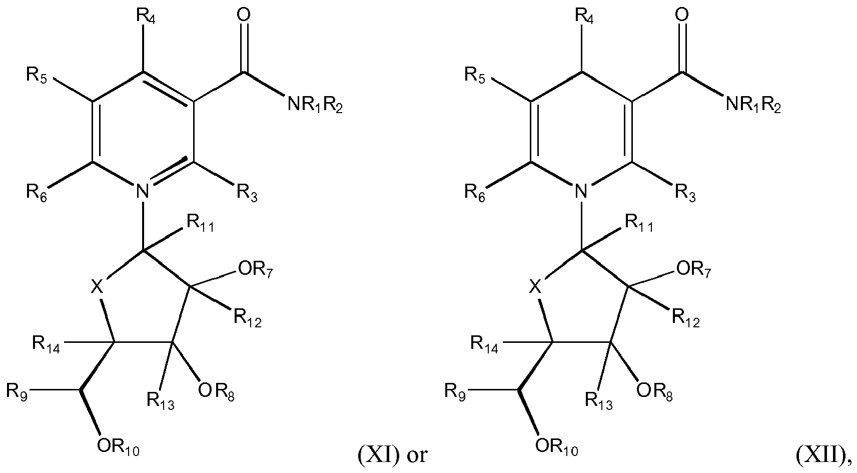 or a pharmaceutically acceptable thereof, wherein:
R₁ and R₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁ and R₂ taken together with the atom to which they are attached form a substituted or unsubstituted non-aromatic heterocyclic group, provided that when one of R₁ and R₂ is -H, the other is not a 2,2,6,6-tetramethyl-1-oxypiperidin-4-yl group and is not an alkyl group substituted by -C(O)OCH₂CH₃;
R₃ and R₄ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -SR, -OSO₃H, -S(O)ₙR, -S(O)ₙOR, -S(O)ₙNRR', -NRR', -NRC(O)OR', -NO₂ and -NRC(O)R';
R₅ is selected from the group consisting of -H, a substituted alkyl group, a substituted or unsubstituted aryl group, a substituted or unsubstituted non-aromatic heterocyclic group, halogen, -OR, -CN, -CO₂R, -OCOR, - OCO₂R, -C(O)NRR', -OC(O)NRR', -C(O)R, -SR, -OSO₃H, -S(O)ₙR, -S(O)ₙOR, -S(O)ₙNRR', -NRR', -NRC(O)OR', -NO₂ and -NRC(O)R';
R₆ is selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -SR, -OSO₃H, -S(O)ₙR, -S(O)ₙOR, -S(O)ₙNRR', -NRC(O)OR', -NO₂ and -NRC(O)R';
R₇, R₈ and R₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₉ selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -SR, -OSO₃H, -S(O)ₙR, -S(O)ₙOR, -S(O)ₙNRR', -NRR', -NRC(O)OR' and -NRC(O)R';
R₁₁, R₁₂, R₁₃ and R₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2,
wherein when a group is substituted, the substituents are independently selected from one or more of -OH, halogen, -OR^{a}, -O-COR^{a}, -C(O)R^{a}, -CN, -NO₂, -COOH, -COOR^{a}, -OCO₂R^{a}, -C(O)NR^{a}R^{b}, -OC(O)NR^{a}R^{b}, -SO₃H, -NH₂, -NHR^{a}, -N(R^{a}R^{b}), -COOR^{a}, -CHO, -CONH₂, -CONHR^{a}, -CON(R^{a}R^{b}), -NHCOR^{a}, -NRCOR^{a}, -NHCONH₂, -NHCONR^{a}H, -NHCON(R^{a}R^{b}), -NR^{c}CONH₂, -NR^{c}CONR^{a}H, -NR^{c}CON(R^{a}R^{b}), -C(=NH)-NH₂, -C(=NH)-NHR^{a}, -C(=NH)-N(R^{a}R^{b}), -C(=NR^{c})-NH₂, -C(=NR^{c})-NHR^{a}, -C(=NR^{c})-N(R^{a}R^{b}), -NH-C(=NH)-NHR^{a}, -NH-C(=NH)-N(R^{a}R^{b}), -NH-C(=NR^{c})-NH₂, -NH-C(=NR^{c})-NHR^{a}, -NH-C(=NR^{c})-N(R^{a}R^{b}), -NR^{d}-C(=NH)-NH₂, -NR^{d}-C(=NH)-NHR^{a}, -NR^{d}-C(=NH)-N(R^{a}R^{b}), -NR^{d}-C(=NR^{c})-NH₂, -NR^{d}-C(=NR^{c})-NHR^{a}, -NR^{d}-C(=NR^{c})-N(R^{a}R^{b}), -NHNH₂, -NHNHR^{a}, -SO₂NH₂, -SO₂NHRₐ, -SO₂NR^{a}R^{b}, -CH=CHR^{a}, -CH=CR^{a}R^{b}, -CR^{c}=CR^{a}R^{b}, -CR^{c}=CHR^{a}, -CR^{c}=CR^{a}R^{b}, -CCR^{a}, -SH, -SOₖR^{a}, -S(O)ₖOR^{a} and -NH-C(=NH)-NH₂, wherein
k is 0, 1 or 2;
R^{a}-R^{d} are each independently an aliphatic, benzyl, or saromatic group; and
-NR^{a}R^{b}, taken together, can also form an unsubstitutednon-aromatic heterocyclic group;
wherein a non-aromatic heterocyclic group, benzylic group or aryl group can also have an aliphatic or substituted aliphatic group as a substituent; a substituted aliphatic group can also have a non-aromatic heterocyclic ring, a substituted non-aromatic heterocyclic ring, benzyl, substituted benzyl, aryl or substituted aryl group as a substituent; and a substituted aliphatic, non-aromatic heterocyclic group, substituted aryl, or substituted benzyl group can have more than one substituent;
provided that R_{I}-R₆ are not each -H.

8. A compound represented by Structural Formula (XI) or (XII): 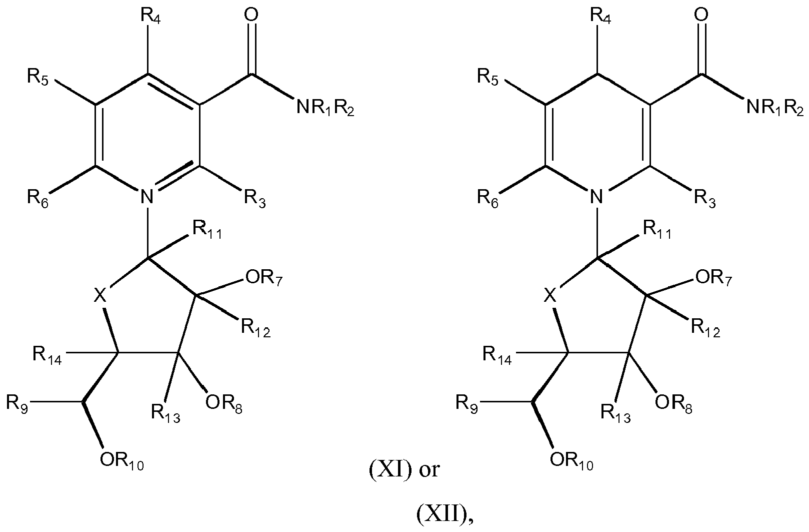 or a pharmaceutically acceptable salt thereof, wherein:
R₁ and R₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁ and R₂ taken together with the atom to which they are attached form a substituted or unsubstituted non-aromatic heterocyclic group;
R₃, R₄, R₅ and R₆ are independently selected from the group consisting of-H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -SR, -OSO₃H, -S(O)ₙR, -S(O)ₙOR, -S(O)ₙNRR', -NRR', -NRC(O)OR', -NO₂ and -NRC(O)R';
R₇, R₈ and R₁₀ are independently selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR, wherein at least one of R₇ and R₈ is a substituted or unsubstituted alkyl group, a substituted or unsubstituted aryl group, -C(O)R, -C(O)OR, -C(O)NHR, - C(S)R, -C(S)OR or -C(O)SR, provided that none of R₇ and R₈ are -C(O)C₆H₅ and that R₇ and R₈ are not both -C(O)CH₃ or -C(O)C₆H₄F;
R₉ selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -SR, -OSO₃H, -S(O)ₙR, -S(O)ₙOR, -S(O)ₙNRR', -NRR', -NRC(O)OR' and -NRC(O)R';
R₁₁, R₁₂, R₁₃ and R₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2,
wherein when a group is substituted, the substituents are independently selected from one or more of -OH, halogen, -OR^{a}, -O-COR^{a}, -C(O)R^{a}, -CN, -NO₂, -COOH, -COOR^{a}, -OCO₂R_{.}, -C(O)NR^{a}R^{b}, -OC(O)NR^{a}R^{b}, -SO₃H, -NH₂, -NHR^{a}, -N(R^{a}R^{b}), -COOR^{a}, -CHO, -CONH₂, -CONHR^{a}, -CON(R^{a}R^{b}), -NHCOR^{a}, -NRCOR^{a}, -NHCONH₂, -NHCONR^{a}H, -NHCON(R^{a}R^{b}), -NR^{c}CONH₂, -NR^{c}CONR^{a}H, -NR^{c}CON(R^{a}R^{b}), -C(=NH)-NH₂, -C(=NH)-NHR^{a}, -C(=NH)-N(R^{a}R^{b}), -C(=NR^{c})-NH₂, -C(=NR^{c})-NHR^{a}, -C(=NR^{c})-N(R^{a}R^{b}), -NH-C(=NH)-NHR^{a}, -NH-C(=NH)-N(R^{a}R^{b}), -NH-C(=NR^{c})-NH₂, -NH-C(=NR^{c})-NHR^{a}, -NH-C(=NR^{c})-N(R^{a}R^{b}), -NR'-C(=NH)-NH₂, -NR^{d}-C(=NH)-NHR^{a}, -NR^{d}-C(=NH)-N(R^{a}R^{b}), -NR^{d}-C(=NR^{c})-NH₂, -NR^{d}-C(=NR^{c})-NHR^{a}, -NR^{d}-C(=NR^{c})-N(R^{a}R^{b}), -NHNH₂, -NHNHR^{a}, -SO₂NH₂, -SO₂NHRₐ, -SO_{2N}R^{a}R^{b}, -CH=CHR^{a}, -CH=CR^{a}R^{b}, -CR^{c}=CR^{a}R^{b}, -CR^{c}=CHR^{a} -CR^{c}=CR^{a}R^{b}, -CCR^{a}, -SH, -SOₖR^{a}, -S(O)ₖOR^{a} and -NH-C(=NH)-NH₂, wherein
k is 0, 1 or 2;
R^{a}-R^{d} are each independently an aliphatic, benzyl, or aromatic group; and
-NR^{a}R^{b}, taken together, can also form an unsubstitutednon-aromatic heterocyclic group;
wherein a non-aromatic heterocyclic group, benzylic group or aryl group can also have an aliphatic or substituted aliphatic group as a substituent; a substituted aliphatic group can also have a non-aromatic heterocyclic ring, a substituted non-aromatic heterocyclic ring, benzyl, substituted benzyl, aryl or substituted aryl group as a substituent; and a substituted aliphatic, non-aromatic heterocyclic group, substituted aryl, or substituted benzyl group can have more than one substituent.

9. A compound represented by Structural Formula (XI) or (XII): 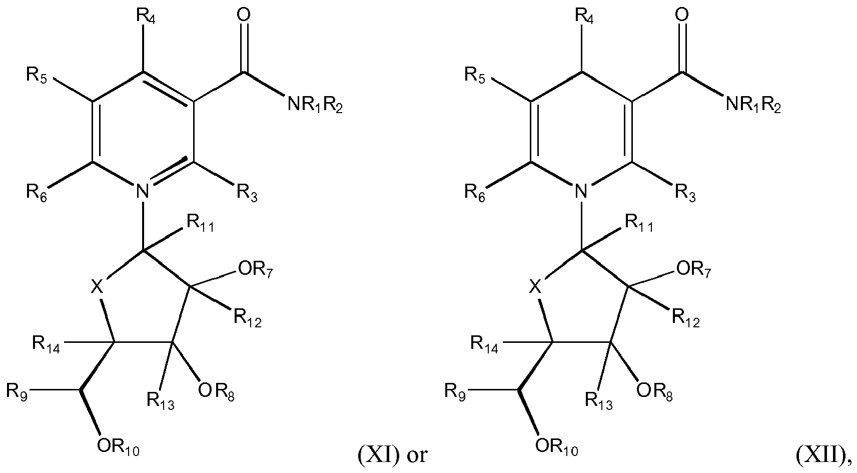 or a pharmaceutically acceptable salt thereof, wherein:
R₁ and R₂ are independently -H, a substituted or unsubstituted alkyl group, a substituted or unsubstituted alkenyl group, a substituted or unsubstituted alkynyl group, a substituted or unsubstituted non-aromatic heterocyclic group or a substituted or unsubstituted aryl group, or R₁ and R₂ taken together with the atom to which they are attached form a substituted or unsubstituted non-aromatic heterocyclic group;
R₃, R₄, R₅ and R₆ are independently selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -SR, -OSO₃H, -S(O)ₙR, -S(O)ₙOR, -S(O)ₙNRR', -NRR', -NRC(O)OR', -NO₂ and -NRC(O)R';
R₇ and R₈ are selected from the group consisting of -H, a substituted or unsubstituted alkyl group, a substituted or unsubstituted aryl group, -C(O)R, -C(O)OR, -C(O)NHR, -C(S)R, -C(S)OR and -C(O)SR;
R₉ selected from the group consisting of -H, a substituted or unsubstituted alkyl group, a substituted or unsubstituted aryl group, a substituted or unsubstituted non-aromatic heterocyclic group, halogen, -OR, -CN, -CO₂R, -OCOR, -OCO₂R, -C(O)NRR', -OC(O)NRR', -C(O)R, -SR, -OSO₃H, -S(O)ₙR, -S(O)ₙOR, -S(O)ₙNRR', -NRR', -NRC(O)OR' and -NRC(O)R';
R₁₀ is selected from the group consisting of a substituted or unsubstituted alkyl group, a substituted or unsubstituted aryl group, -C(O)R, - C(O)OR, -C(O)NHR, -C(S)R, -C(S)OR and -C(O)SR, provided that R₁₀ is not -C(C₆H₅)₃, -C(O)C₆H_{5,} -C(O)CH₃, -C(O)C₆H₄F or - C(O)CH(OC(O)CH₃)CH(CH₃)CH₂CH₃;
R₁₁, R₁₂, R₁₃ and R₁₄ are independently selected from the group consisting of -H, a substituted or unsubstituted alkyl group, a substituted or unsubstituted aryl group, a substituted or unsubstituted non-aromatic heterocyclic group, halogen, -CN, -CO₂R, -OCOR, -OCO₂R, -C(O)NRR', -OC(O)NRR', -C(O)R, -OSO₃H, -S(O)ₙR, -S(O)ₙOR, -S(O)ₙNRR', -NRR', -NRC(O)OR', -NO₂ and -NRC(O)R';
R and R' are independently -H, a substituted or unsubstituted alkyl group, a substituted or unsubstituted aryl group or a substituted or unsubstituted non-aromatic heterocyclic group;
X is O or S; and
n is 1 or 2,
wherein when a group is substituted, the substituents are independently selected from one or more of -OH, halogen, -OR^{a}, -O-COR^{a}, -C(O)R^{a}, -CN, -NO₂, -COOH, -COOR^{a}, -OCO₂R^{a}, -C(O)NR^{a}R^{b}, -OC(O)NR^{a}R^{b}, -SO₃H, -NH₂, -NHR^{a}, -N(R^{a}R^{b}), -COOR^{a}, -CHO, -CONH₂, -CONHR^{a}, -CON(R^{a}R^{b}), -NHCOR^{a}, -NRCOR^{a}, -NHCONH₂, -NHCONR^{a}H, -NHCON(R^{a}R^{b}), -NR^{c}CONH₂, -NR^{c}CONR^{a}H, -NR^{c}CON(R^{a}R^{b}), -C(=NH)-NH₂, -C(=NH)-NHR^{a}, -C(=NH)-N(R^{a}R^{b}), -C(=NR^{c})-NH₂, -C(=NR^{c})-NHR^{a}, -C(=NR^{c})-N(R^{a}R^{b}), -NH-C(=NH)-NHR^{a}, -NH-C(=NH)-N(R^{a}R^{b}), -NH-C(=NR^{c})-NH₂, -NH-C(=NR^{c})-NHR^{a}, -NH-C(=NR^{c})-N(R^{a}R^{b}), -NR^{d-}C(=NH)-NH₂, -NR^{d}-C(=N_{H})-NHR^{a}, -NR^{d}-C(=NH)-N(R^{a}R^{b}), -NR^{d}-C(=NR^{c})-NH₂, -NR^{d}-C(=NR^{c})-NHR^{a}, -NR^{d}-C(=NR^{c})-N(R^{a}R^{b}), -NHNH₂, -NHNHR^{a}, -SO₂NH₂, -SO₂NHRₐ, -SO₂NR^{a}R^{b}, -CH=CHR^{a}, -CH=CR^{a}R^{b}, -CR^{c}=CR^{a}R^{b}, -CR^{c}=CHR^{a}, -CR^{c}=CR^{a}R^{b}, -CCR^{a}, -SH, -SOₖR^{a}, -S(O)ₖOR^{a} and -NH-C(=NH)-NH₂, wherein
k is 0, 1 or 2;
R^{a}-R^{d} are each independently an aliphatic, benzyl, or aromatic group; and
-NR^{a}R^{b}, taken together, can also form an unsubstituted non-aromatic heterocyclic group;
wherein a non-aromatic heterocyclic group, benzylic group or aryl group can also have an aliphatic or substituted aliphatic group as a substituent; a substituted aliphatic group can also have a non-aromatic heterocyclic ring, a substituted non-aromatic heterocyclic ring, benzyl, substituted benzyl, aryl or substituted aryl group as a substituent; and a substituted aliphatic, non-aromatic heterocyclic group, substituted aryl, or substituted benzyl group can have more than one substituent.

10. A pharmaceutical composition comprising a compound represented by Structural Formula (XI) or (XII) as defined in claim 7.

11. A pharmaceutical composition comprising a compound represented by Structural Formula (XI) or (XII) as defined in claim 8.

12. A pharmaceutical composition comprising a compound represented by Structural Formula (XI) or (XII) as defined in claim 9.
